(19) Europäisches Patentamt
European Patent Office
Office européen des brevets

(11) EP 1 785 423 A1

(12) **EUROPÄISCHE PATENTANMELDUNG**

(43) Veröffentlichungstag:
**16.05.2007 Patentblatt 2007/20**

(51) Int Cl.:
***C07D 471/04*** *(2006.01)* ***A61K 31/4985*** *(2006.01)*
***A61P 35/00*** *(2006.01)*

(21) Anmeldenummer: **05024693.3**

(22) Anmeldetag: **11.11.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **Zentaris GmbH**
**60314 Frankfurt/Main (DE)**

(72) Erfinder:
- **Claus, Eckhard**
  **60388 Frankfurt a. M. (DE)**
- **Seipelt, Irene**
  **63069 Offenbach (DE)**
- **Günther, Eckhard**
  **63477 Maintal (DE)**
- **Polymeropoulos, Emmanuel**
  **60325 Frankfurt a. M. (DE)**
- **Czech, Michael**
  **60435 Frankfurt a. M. (DE)**
- **Schuster, Tilman**
  **63762 Grossostheim (DE)**

(54) **Neue Pyridopyrazine und deren Verwendung als Modulatoren von Kinasen**

(57) Die Erfindung betrifft neue Pyrido[2,3-b]pyrazin-Derivate der allgemeinen Formel I, deren Herstellung und Verwendung als Arzneimittel, insbesondere zur Behandlung von malignen und anderen, auf pathologischen Zellproliferationen beruhenden Erkrankungen.

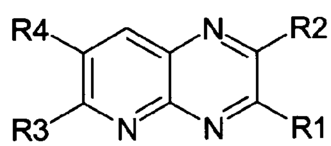

**I**

EP 1 785 423 A1

**Beschreibung**

Technisches Gebiet

Die Erfindung betrifft Kinase-Modulatoren vom Typ der Pyrido[2,3-b]pyrazine, deren Herstellung und Verwendung als Arzneimittel zur Modulation von fehlgeleiteten zellulären Signaltransduktionsprozessen, insbesondere zur Beeinflussung der Funktion von Tyrosin-, Serin-/Threonin- und Lipidkinasen und zur Behandlung von malignen bzw. benignen Tumorerkrankungen und anderen, auf pathologischen Zellproliferationen beruhenden Erkrankungen, wie z. B. Restenose, Psoriasis, Arteriosklerose und Leberzirrhose.

Stand der Technik

Die Aktivierung von Proteinkinasen ist ein zentrales Ereignis bei zellulären Signaltransduktions-Prozessen. Eine aberrante Kinaseaktivierung wird bei diversen Krankheitszuständen beobachtet. Daher ist die gezielte Inhibition von Kinasen ein fundamentales therapeutisches Ziel.

Die Phosphorylierung von Proteinen wird im Allgemeinen durch extrazelluläre Signale initiiert und stellt einen universellen Mechanismus für die Kontrolle von verschiedenen zellulären Ereignissen, wie z. B. metabolischen Prozessen, Zellwachstum, Zellmigration, Zelldifferenzierung, Membrantransport und Apoptose dar. Für die Proteinphosphorylierung ist die Proteinfamilie der Kinasen verantwortlich. Diese Enzyme katalysieren den Phosphat-Transfer zu spezifischen Substratproteinen. Basierend auf der Substratspezifität werden die Kinasen in drei Hauptklassen, die Tyrosinkinasen, die Serin/Threonin-Kinasen und die Lipidkinasen unterteilt. Sowohl die Rezeptor-Tyrosinkinasen als auch die cytoplasmatischen Tyrosin-, Serin/Threonin- und Lipidkinasen sind wichtige Proteine der Signaltransduktion der Zelle. Eine Überexpression bzw. Entartung dieser Proteine spielt eine wichtige Rolle bei auf pathologischen Zellproliferationen beruhenden Erkrankungen. Dazu zählen unter anderem Stoffwechselerkrankungen, Erkrankung des Bindegewebes und der Blutgefäße, sowie maligne und benigne Tumorerkrankungen. Bei der Tumorentstehung und Entwicklung treten sie häufig als Onkogene d.h. als aberrante, konstitutiv aktive Kinaseproteine auf. Die Folgen dieser übermäßigen Kinaseaktivierung sind z. B. das unkontrollierte Zellwachstum und der reduzierte Zelltod. Auch die Stimulation von tumorinduzierten Wachstumsfaktoren kann Ursache für die Überstimulation von Kinasen sein. Die Entwicklung von Kinasemodulatoren ist daher von besonderem Interesse für alle pathogenen Prozesse, die durch Kinasen beeinflusst werden.

Die Signaltransduktionskaskaden ras-Raf-Mek-Erk und PI3K-Akt spielen eine zentrale Rolle bei Zellwachstum, Zellproliferation, Apoptose, Adhäsion, Migration und Glukose Stoffwechsel. So ist die fundamentale Beteiligung an der Pathogenese von Erkrankungen wie Krebs, Neurodegeneration und endzündlichen Erkrankungen sowohl für den ras-Raf-Mek-Erk- als auch für den PI3K-Akt-Signalweg belegt. Daher stellen die Einzelkomponenten dieser Signalkaskaden wichtige therapeutische Angriffspunkte für die Intervention der verschiedenen Krankheitsprozesse dar (Weinstein-Oppenheimer C.R. et al 2000, Chang F. et al 2003, Katso R. et al 2001 und Lu Y. et al 2003).

Im Folgenden sind die molekularen und biochemischen Eigenschaften beider Signalwege zunächst gesondert beschrieben.

Eine Vielzahl von Wachstumsfaktoren, Zytokinen und Onkogenen transduziert ihre wachstumsfördernden Signale über die Aktivierung von G-Protein gekuppeltem ras, welches zur Aktivierung der Serin-Threonin-Kinase Raf und zur Aktivierung der Mitogen aktivierten Protein Kinase Kinase 1 und 2 (MAPKK1/2 oder Mek1/2) führt und in der Phosphorylierung und Aktivierung der MAPK 1 und 2 - auch bekannt als Extrazellulär Signal Regulierte Kinase (Erk1 und 2) - resuliert. Verglichen mit anderen Signalwegen vereint der ras-Raf-Mek-Erk-Signalweg eine große Anzahl an Proto-Onkogenen, eingeschlossen Liganden, Tyrosinkinase-Rezeptoren, G-Proteinen, Kinasen und nukleären Transkriptionsfaktoren. Tyrosin-Kinasen, wie z.B. der EGFR (Mendelsohn J. et al., 2000) vermitteln im Tumorgeschehen, bedingt durch Überexpression und Mutation, häufig konstitutiv aktive Signale an den nachgeschalteten ras-Raf-Mek-Erk-Signalweg. Ras Mutationen sind in 30% aller humanen Tumore mutiert (Khleif S.N. et al., 1999, Marshall C., 1999) wobei die höchste Inzidenz mit 90% bei Pankreaskarzinomen liegt (Friess H. et al., 1996, Sirivatanauksorn V. et al., 1998). Für c-Raf wurde bei verschiedenen Tumoren eine deregulierte Expression und/oder Aktivierung beschrieben (Hoshino R. et al., 1999, McPhillips F. et al., 2001). B-Raf-Punktmutanten wurden in 66% aller humanen malignen Melanome, 14% ovarialen und 12% Kolonkarzinomen detektiert (Davies H. et al., 2002). Daher ist es nicht überraschend, dass Erk1/2 an vielen zellulären Prozessen wie Zellwachstum, Zellproliferation und Zelldifferenzierung primär beteiligt ist (Lewis T.S. et al., 1998, Chang F. et al., 2003).

Darüberhinaus haben die Mitglieder der Raf-Kinasen auch Mek-Erk unabhängige, anti-apoptotische Funktionen, deren molekulare Schritte noch nicht vollständig beschrieben sind. Als mögliche Interaktionspartner für die Mek-Erk-unabhängige Raf-Aktivität wurden Ask1, Bcl-2, Akt und Bag1 beschrieben (Chen J et al., 2001, Troppmaier J. et al., 2003, Rapp U.R. et al., 2004, Gotz R. et al., 2005). Man geht heute davon aus, daß sowohl Mek-Erk-abhängige als auch Mek-Erk-unabhängige Signaltransduktions-Mechanismen die Aktivierung der oberhalb gelegenen Stimuli ras und Raf steuern.

Die Isoenzyme der Phosphatidylinositol 3-Kinasen (PI3Ks) fungieren vorwiegend als Lipidkinasen und katalysieren die D3-Phosphorylierung der Second-Messenger Lipide Ptdlns (Phosphatidylinositol) zu Ptdlns(3)P, Ptdins(3,4) $P_2$, Ptdlns(3,4,5)$P_3$ Phosphatidylinositolphosphaten. Die PI3Ks der Klasse I setzen sich strukturell aus der katalytischen (p110alpha, beta, gamma, delta) und der regulatorischen (p85alpha, beta oder p101gamma) Untereinheit zusammen. Weiterhin gehören die Klasse II-(PI3K-C2alpha, PI3K-C2beta) und Klasse III-(Vps34p)Enzyme zur Familie der PI3-Kinasen (Wymann M.P. et al., 1998, VanHaesebroeck B. et al., 2001). Der durch die PI3Ks ausgelöste PIP-Anstieg aktiviert einerseits den proliferativen ras-Raf-Mek-Erk Signalweg über die Kupplung von ras (Rodriguez-Viciana P. et al., 1994) und stimuliert andererseits den anti-apoptotischen Signalweg durch Rekrutierung von Akt an die Zellmembran und folglicher Überaktivierung dieser Kinase (Alessi D.R. et al., 1996, Chang H.W. et al., 1997, Moore S.M. et al., 1998). Somit erfüllt die Aktivierung der PI3Ks mindestens 2 entscheidende Mechanismen der Tumorentstehung, nämlich die Aktivierung von Zellwachstum und Zelldifferenzierung und die Inhibition der Apoptose. Darüberhinaus verfügen die PI3K auch über Protein-phosphorylierende Eigenschaften (Dhand et al., 1994, Bondeva T. et al., 1998, Bondev A. et al., 1999, VanHaesebroeck B. et al., 1999) die z.B. eine die PI3Ks intrinsisch regulierende Serin-Autophosphorylierung auslösen kann. Außerdem ist bekannt, dass PI3Ks auch Kinase-unabhängige, regulierende Effektor-Eigenschaften haben z.B. bei der Kontrolle der Herzkontraktion (Crackower M.A. et al., 2002, Patrucco et al., 2004). Ferner ist belegt, dass PI3Kdelta und PI3Kgamma auf hematopoietischen Zellen spezifisch exprimiert werden und damit potenzielle Angriffpunkte für Isoenzym-spezifische PI3Kdelta- und Pl3Kgamma-Inhibitoren bei der Behandlung von inflammatorischen Erkrankungen wie Rheuma, Asthma und Allergien und bei der Behandlung von B- und T-Zelllymphomen (Okkenhaug K. et al., 2003, Ali K. et al., 2004, Sujobert P. et al., 2005) darstellen. Die PI3Kalpha, welche kürzlich als Proto-Onkogen identifiziert wurde (Shayesteh L. et al., 1999, Ma Y.Y. et al., 2000, Samuels Y. et al., 2004, Campbell I.G. et al., 2004, Levine D.A., 2005) gilt als wichtiges Target bei der Therapie von Tumorerkrankungen. Die Bedeutung der PI3K-Spezies als Target für die Wirkstoffentwicklung ist daher äußerst vielfältig (Chang F. & Lee J.T. et al, 2003).

Von ebenso großem Interesse sind die PI3K verwandten Kinasen (PIKK), welche die Serin/Threonin-Kinasen mTOR, ATM, ATR, h-SMG-1 und DNA-PK (Chiang G.G. et al 2004) einschließen. Ihre katalytischen Domänen haben eine hohe Sequenz-Homologie zur katalytische Domäne der PI3Ks.

Überdies trägt der Verlust des Tumorsuppressor-Proteins PTEN (Li J. et al., 1997, Steck P.A. et al., 1997) - dessen Funktion die Reversion der durch die PI3K initiierte Phosphorylierung ist - zu einer Überaktivierung von Akt und deren abwärts gelegenen Kaskade-Komponenten bei und unterstreicht damit die kausale Bedeutung von PI3K als Zielmolekül für die Tumortherapie.

Diverse Inhibitoren von Einzelkomponenten der ras-Raf-Mek-Erk und PI3K-Akt-Signalwege sind bereits publiziert und patentiert worden.

Der gegenwärtige Entwicklungsstand auf dem Gebiet der Kinase-Inhibitoren, besonders des ras-Raf-Mek-Erk- und des PI3K-Akt-Weges wird in den Übersichten von J.S. Sebolt-Leopold et al., 2004, und R. Wetzker et al., 2004 dargestellt. In benannten Publikation finden sich umfassende Auflistungen der offengelegten Patentschriften, die die Synthese und Anwendung niedermolekularer ras-Raf-Mek-Erk- und PI3K-Inhibitoren beschreiben.

Der bereits in klinischer Erprobung befindliche Kinaseinhibitor Bay 43-9006 (WO 99/32111, WO 03/068223) zeigt ein relativ unspezifisches Inhibitionsmuster von Serin/Threonin- und von Tyrosin-Kinasen wie Raf, VEGFR2/3, Flt-3, PDGFR, c-Kit und weiteren Kinasen. Diesem Inhibitor wird bei durch Angiogenese-induzierten fortgeschrittenen Tumorerkrankungen (z.B. beim Nierenzellkarzinom) aber auch bei Melanomen mit hoher B-Raf-Mutationsrate eine große Bedeutung zugemessen. Die klinische Wirkung von Bay 43-9006 wird zurzeit außerdem an Patienten mit refraktären soliden Tumoren in Kombination z.B. mit Docetaxel ermittelt. Bislang wurden milde Nebenwirkungen und vielversprechende anti-tumorale Effekte beschrieben. Eine Inhibition der Kinasen im PI3K-Akt-Signalweg ist für Bay 43-9006 nicht beschrieben oder offenbart.

Der Mek1/2-Inhibitor PD0325901 (WO 02/06213) befindet sich derzeit in klinischer Erprobung der Phase I. Die Vorläufersubstanz Cl-1040 (WO 00/35435, WO 00/37141) fiel durch ihre hohe Mek-Spezifität und Target-Affinität auf. Jedoch erwies sich diese Verbindung in Phase I/II-Studien als metabolisch instabil. Klinische Daten der aktuellen Nachfolgersubstanz PD0325901 stehen noch aus. Jedoch ist für diesen Mek-Inhibitor weder eine Wechselwirkung mit Erk1 oder Erk2 noch eine den PI3K-Akt-Signalweg inhibierende Funktion oder deren gleichzeitige Modulation publiziert oder offenbart.

Die bislang publizierten PI3K-Inhibitoren befinden sich in präklinischer Erprobung. ICOS offenbarte einen PI3K-Inhibitor IC87114 mit hoher PI3Kdelta Isoenzymspezifität (WO 01/81346). Für PI103 (WO 04/017950) beschreiben Yamanouchi/Piramed eine Selektivität versus der PI3Kalpha Isoform. In der frühen Entwicklung von PI3K-Inhibitoren existiert darüberhinaus ein stark beachtetes Forschungs-Umfeld (s. Übersicht R. Wetzker et al., 2004).

Inhibitoren des SAPK-Signalweges, entweder von Jnk oder von p38 sind in der Literatur beschrieben (Gum R.J., 1998, Bennett B.L. et al 2001, Davies S.P. et al 2000). Jedoch ist für diese SAPK-Inhibitoren keine die PI3Ks inhibierende Funktion und auch keine spezifische Inhibition der Erk1 oder Erk2 oder auch gleichzeitige Inhibition von SAPKs, Erk1, Erk2, oder PI3Ks offenbart.

In 6- oder 7-Position substituierte Pyrido[2,3-b]pyrazin-Derivate finden als pharmakologisch aktive Verbindungen und als Synthesebausteine in der pharmazeutischen Chemie vielfältige Verwendung.

Beispielsweise werden in den Patentschriften WO 04/104002 und WO 04/104003 Pyrido[2,3-b]pyrazine beschrieben, die in 6- oder 7-Position mit Harnstoff-, Thioharnstoff-, Amidin- oder Guanidingruppen substituiert sein können. Diese Verbindungen besitzen Eigenschaften als Inhibitoren bzw. Modulatoren von Kinasen, insbesondere von Tyrosin- und Serin/Threoninkinasen und es ist eine Verwendung als Arzneimittel angegeben. Demgegenüber ist eine Verwendung dieser Verbindungen als Modulatoren von Lipidkinasen, alleine oder in Kombination mit Tyrosin- und Serin/Threoninkinasen, nicht beschrieben.

Weiterhin werden in der Patentschrift WO 99/17759 Pyrido[2,3-b]pyrazine beschrieben, die in 6-Position unter anderem Alkyl-, Aryl- und Heteroarylsubstituierte Carbamate tragen. Diese Verbindungen sollen dazu verwendet werden, die Funktion von Serin-Threonin-Proteinkinasen zu modulieren.

In der Patentschrift WO 05/007099 (Kawakami et al.) werden unter anderem auch Harnstoff-substituierte Pyrido [2,3-b]pyrazine als Inhibitoren der Serin/Threoninkinase PKB beschrieben. Allerdings ist in dieser Patentschrift der Rest R, der den Substitutionsumfang am Harnstoff beschreiben soll, nicht weiter definiert und somit ist der Umfang der Substitution am Harnstoff nicht eindeutig offenbart. Für diese Verbindungen wird eine Verwendung bei der Behandlung von Krebserkrankungen angegeben. Allerdings werden keine konkreten Beispiele von Harnstoff-substituierten Pyridopyrazinen mit diesen biologischen Eigenschaften aufgeführt. Zusätzlich unterscheiden sich die hier beschriebenen Pyridopyrazine strukturell deutlich von den in dieser Erfindung beschriebenen erfindungsgemäßen Pyrido[2,3-b]pyrazinen.

Weitere Beispiele für in 6- und 7-Position Harnstoff-substitutierte Pyrido[2,3-b]pyrazine werden in der Patentschrift WO 05/056547 (Bemis et al.) angegeben. Die Verbindungen in dieser Patentschrift haben allerdings in 2- oder 3-Position zusätzliche Carbonyl-, Sulfoxy-, Sulfon- oder Imin-Substitution, wodurch sich die Verbindungen strukturell deutlich von den in dieser Erfindung beschriebenen erfindungsgemäßen Pyrido[2,3-b]pyrazine unterscheiden. Die in WO 05/056547 angegebenen Pyridopyrazine werden als Inhibitoren von Protein-Kinasen, insbesondere von GSK-3, Syk und JAK-3, beschrieben. Für diese Verbindungen wird unter anderem eine Verwendung bei der Behandlung von proliferativen Erkrankungen angegeben. Eine Verwendung dieser Verbindungen als Modulatoren von Lipidkinasen, alleine oder in Kombination mit Serin/Threoninkinasen, ist nicht beschrieben.

In dem Patent WO 04/005472 von White et al. werden unter anderem in 6-Position Carbamat-substituierte Pyrido[2,3-b]pyrazine beschrieben, die als antibakterielle Substanzen das Wachstum von Bakterien hemmen. Eine Antitumorwirkung ist nicht beschrieben.

Bestimmte Diphenylchinoxaline und -pyrido[2,3-b]pyrazine mit speziellen Alkylpyrrolidin-, Alkylpiperidin- oder Alkylsulfonamid-Resten an einem Phenylring, die zusätzlich auch Harnstoff- oder Carbamat-Substitutionen in 6- oder 7-Position tragen können, werden in den Patentschriften WO 03/084473 (Barnett et al.), WO 03/086394 (Bilodeau et al.) und WO 03/086403 (Lindsley et al.) als Inhibitoren der Aktivität der Serin/Threonin-Kinase Akt beschrieben. Für diese Verbindungen wird eine Verwendung bei der Behandlung von Krebserkrankungen angegeben. Für die dort beschriebenen Pyrido[2,3-b]pyrazin-Beispiel-Verbindungen ist kein definierter Hinweis auf eine biologische Wirkung angegeben. Ausserdem besteht ein deutlicher struktureller Unterschied zu den in dieser Erfindung beschriebenen erfindungsgemäßen Pyrido[2,3-b]pyrazinen.

Weiterhin werden in dem Patent WO 03/024448 von Delorme et al. Amid- und Acrylamid-substituierte Pyrido [2,3-b]pyrazine beschrieben, die als zusätzliche Substituenten auch Carbamate enthalten und als Histon Deacetylase-Inhibitoren zur Behandlung von Zellproliferationserkrankungen verwendet werden können.

In einer weiteren Publikation (C. Temple, Jr.; J. Med. Chem. 1990, 3044-3050) wird an einem Beispiel die Synthese eines 6-Ethylcarbamat-substituierten Pyrido[2,3-b]pyrazin-Derivates beschrieben. Eine Antitumorwirkung ist weder offenbart noch nahegelegt.

Die Synthese von weiteren Derivaten des 6-Ethylcarbamat-substituierten Pyrido[2,3-b]pyrazins wird in einer Veröffentlichung von R. D. Elliott beschrieben (J. Org. Chem. 1968, 2393-2397). Eine biologische Wirkung dieser Verbindungen ist weder beschrieben noch nahegelegt.

In der Publikation von C.Temple, Jr. J. Med. Chem. 1968, 1216-1218 wird die Synthese und Untersuchung von 6-Ethylcarbamat-substituierten Pyrido[2,3-b]pyrazinen als potentielle Antimalaria-Wirkstoffe beschrieben. Eine Antitumorwirkung ist weder offenbart noch nahegelegt.

Darstellung der Erfindung

Die Erfindung ist daher darauf ausgerichtet, neue Verbindungen zu schaffen, die als Modulatoren von Rezeptor-Tyrosinkinasen, von cytoplasmatischen Tyrosin-, Serin/Threoninkinasen und Lipidkinasen geeignet sind. Da nicht alle in fehlregulierten Signaltransduktionskaskaden hintereinander geschalteten Kinasen - wie z. B. bei Raf-Mek-Erk oder PI3K-Akt - als onkogene Kinasen bzw. als konstitutiv aktive Enzyme vorliegen müssen, werden in dieser Erfindung auch die nicht-aktiven Kinasen als therapeutische Zielproteine betrachtet, d.h. die neuen Verbindungen können sowohl an aktiven als auch an nicht-aktiven Kinasen binden und damit die Signaltransduktion beeinflussen.

Die Erfindung ist weiterhin darauf ausgerichtet, neue Verbindungen zu schaffen, die als Modulatoren von Rezeptor-Tyrosinkinasen, cytoplasmatischen Tyrosin-, Serin/Threoninkinasen und Lipidkinasen die Eigenschaft besitzen, sowohl eine einzelne als auch zwei oder mehrere Kinasen, insbesondere Erk1/2 und PI3K, aus einem oder verschiedenen Signaltransduktionskaskaden, insbesondere ras-Raf-Mek-Erk und PI3K-Akt, zu beeinflussen. Ein dualer Mechanismus, d. h. die gleichzeitige Inhibition von zwei oder mehreren Signaltansduktionskaskaden gegenüber dem therapeutischen Angriff von nur einem Signaltransduktionsweg sollte durch die additive Wirkung zu einer Steigerung des Effektes bei der Behandlung von allen pathogenen Prozessen führen, die durch Kinasen beeinflusst werden.

Es wurde jetzt überraschend gefunden, dass neue Verbindungen aus der Reihe der Pyrido[2,3-b]pyrazine, welche in 6- oder 7-Position z. B. mit Harnstoff- oder Thioharnstoffgruppierungen substituiert sind, zur Herstellung von Arzneimitteln zur Modulation von fehlgeleiteten zellulären Signaltransduktionsprozessen, insbesondere zur Beeinflussung der Funktion von Rezeptor-Tyrosinkinasen, cytoplasmatischen Tyrosin-, Serin/Threonin- und Lipidkinasen und zur Behandlung von malignen bzw. benignen Tumorerkrankungen, wie z. B. der Brust, Prostata, Lunge, Haut, Eierstöcke und anderen, auf pathologischen Zellproliferationen beruhenden Erkrankungen geeignet sind.

Ein erster Aspekt der vorliegenden Anmeldung beschreibt neue Verbindungen aus der Reihe der Pyrido[2,3-b]pyrazine gemäß der allgemeinen Formel I,

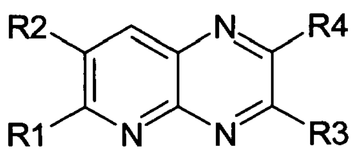

**I**

worin die Substituenten R1-R4 folgende Bedeutung haben:

R1 und R2 können unabhängig voneinander Wasserstoff oder NR5R6 bedeuten, unter der Voraussetzung, dass, wenn R1 = NR5R6 ist, R2 = H ist, und wenn R2 = NR5R6 ist, R1 = H ist,

wobei R5 Wasserstoff, Alkyl, R38, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum mit F, Cl, Br, I, CN, $CF_3$, $NH_2$, NH-Alkyl, NH-Aryl, $N(Alkyl)_2$, $NO_2$, SH, S-Alkyl, OH, $OCF_3$, $O(\text{-Alkyl-O})_p$-Alkyl, O-Aryl, $OSO_3H$, $OP(O)(OH)_2$, $OP(O)(OAlkyl)_2$, $OP(O)(OAryl)_2$ ,CHO, C(O)OH, C(O)OR12, $C(O)NH_2$, C(O)NHR12, C(O)NR12R13, $SO_3H$, $SO_2$Alkyl, $SO_2$Aryl, $P(O)(OH)_2$, $P(O)(OAlkyl)_2$, $P(O)(OAryl)_2$, Alkyl, Cycloalkyl, Heterocyclyl, Aryl, Heteroaryl ein- oder mehrfach, gleich oder verschieden substituiert sein können, p den Wert 0, 1, 2, 3, 4 oder 5 annehmen kann und die Reste R12 und R13 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R12 und R13 zusammen einen Heterocyclyl-Ring bilden können

und R6:

-C(Y)NR7R8 bedeuten kann, wobei Y unabhängig voneinander O oder S sein kann und R7 und R8 unabhängig voneinander Wasserstoff,

unsubstituiertes oder substituiertes Alkyl, wobei der Alkylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, $O(\text{-Alkyl-O})_p$-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Hete-

roaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, SO-Alkyl, SO-Aryl, $SO_2$-Alkyl, $SO_2$-Aryl, $SO_2NH_2$, $SO_2$NH-Alkyl, $SO_2$NH-Aryl, $SO_2$NH-Heteroaryl, $SO_2$NH-Alkyl-Aryl, $SO_3H$, $SO_2$O-Alkyl, $SO_2$O-Aryl, $SO_2$O-Alkyl-Aryl, Cycloalkyl, Heterocyclyl, Aryl oder Heteroaryl, ein- oder mehrfach, gleich oder verschieden substituiert sein kann, wobei p den Wert 0, 1, 2, 3, 4 oder 5 annehmen kann,

unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N$(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, OH, O(-Alkyl-O)$_p$-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, OP(O)$(OH)_2$, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, Alkyl, oder Aryl ein- oder mehrfach, gleich oder verschieden substituiert sein kann, wobei p den Wert 0, 1, 2, 3, 4 oder 5 annehmen kann,

unsubstituiertes oder substituiertes Heterocyclyl, wobei der Heterocyclylrest mit OH, O-Alkyl, O-Aryl, $NH_2$, NH-Alkyl, NH-Aryl, Alkyl, Alkyl-Aryl oder Aryl ein- oder mehrfach, gleich oder verschieden substituiert sein kann,

unsubstituiertes oder substituiertes Aryl, wobei der Arylrest mit F, Cl, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, N$(Alkyl)_2$, NHC(O)-Alkyl, NHC(O)-R38, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, O(-Alkyl-O)$_p$-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, OP(O)$(OH)_2$, C(O)-Alkyl, C(O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, SO-Alkyl, SO-Aryl, $SO_2$-Alkyl, $SO_2$-Aryl, $SO_2NH_2$, $SO_2$NH-Alkyl, $SO_2$NH-Aryl, $SO_2$NH-Heteroaryl, $SO_2$NH-Alkyl-Aryl, SO3H, $SO_2$O-Alkyl, $SO_2$O-Aryl, $SO_2$O-Alkyl-Aryl, Alkyl, Cycloalkyl, Heterocyclyl, Aryl oder Heteroaryl, ein- oder mehrfach, gleich oder verschieden substituiert sein kann, wobei p den Wert 0, 1, 2, 3, 4 oder 5 und n den Wert 1, 2 oder 3 annehmen kann,

unsubstituiertes oder substituiertes Heteroaryl, wobei der Heteroarylrest F, Cl, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, N$(Alkyl)_2$, NHC(O)-Alkyl, NHC(O)-R38, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O(-Alkyl-O)$_p$-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, OP(O)$(OH)_2$, C(O)-Alkyl, C(O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N(Hete-

roaryl)$_2$, $SO_2NH_2$, $SO_2$NH-Alkyl, $SO_2$NH-Aryl, $SO_2$NH-Heteroaryl, $SO_2$NH-Alkyl-Aryl, $SO_3H$, $SO_2$O-Alkyl, $SO_2$O-Aryl, $SO_2$O-Alkyl-Aryl, Alkyl, Cycloalkyl, Heterocyclyl, Aryl oder Heteroaryl, ein- oder mehrfach, gleich oder verschieden substituiert sein kann, wobei p den Wert 0, 1, 2, 3, 4 oder 5 annehmen kann,

-C(O)-R39, wobei R39 Alkyl, Aryl oder Heteroaryl sein kann, und die Alkyl-, Aryl- und Heteroarylsubstituenten ihrerseits wiederum substituiert sein können,

sein können, oder R7 und R8 zusammen einen Heterocyclyl-Ring bilden können,

R3 und R4 können unabhängig voneinander:

Wasserstoff, wobei R3 und R4 nicht gleichzeitig Wasserstoff sind,

substituiertes Alkyl, wobei der Alkylrest mit F, Cl, Br, I, CN, $CF_3$, $NH_2$, NH-Alkyl, NH-Aryl, N(Alkyl)$_2$, SH, S-Alkyl, OH, $OCF_3$, O(-Alkyl-O)$_p$-Alkyl, O-Aryl, $OSO_3H$, OP(O)(OH)$_2$, OP(O)(OAlkyl)$_2$, OP(O)(OAryl)$_2$, C(O)OH, C(O)OR14, C(O)$NH_2$, C(O)NHR14, C(O)NR14R15, $SO_3H$, $SO_2$Alkyl, $SO_2$Aryl, P(O)(OH)$_2$, P(O)(OAlkyl)$_2$, P(O)(OAryl)$_2$, Cycloalkyl, Heterocyclyl, Aryl oder Heteroaryl ein- oder mehrfach, gleich oder verschieden substituiert ist, wobei p den Wert 0, 1, 2, 3, 4 oder 5 annehmen kann und die Reste R14 und R15 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R14 und R15 zusammen einen Heterocyclyl-Ring bilden können,

substituiertes Aryl, wobei der Arylrest ein- oder mehrfach, gleich oder verschieden substituiert ist mit Substituenten ausgewählt aus der Gruppe NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, N(Alkyl)$_2$, N(Aryl)$_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, S-Alkyl, S-Aryl, S-Heteroaryl, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC (O)-Heterocyclyl, OC(O)-Aryl, OC(O)-Heteroaryl, OC(O)-Alkyl-Aryl, OC(O)-Alkyl-Heteroaryl, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, C(O)-Alkyl, C (O)-Aryl, C(O)-Heteroaryl, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)NH-Alkyl, C(O)NH-Cycloalkyl, C(O) NH-Heterocyclyl, C(O)NH-Aryl, C(O)NH-Heteroaryl, C(O)NH-Alkyl-Cycloalkyl, C(O)NH-Alkyl-Heterocyclyl, C(O) NH-Alkyl-Aryl, C(O)NH-Alkyl-Heteroaryl, C(O)N(Alkyl)$_2$, C(O)N(Cycloalkyl)$_2$, C(O)N(Aryl)$_2$, C(O)N(Heteroaryl)$_2$, SO-Alkyl, SO-Aryl, $SO_2$-Alkyl, $SO_2$-Aryl, $SO_2$NH-Alkyl, $SO_2$NH-Aryl, $SO_2$NH-Heteroaryl, $SO_2$NH-Alkyl-Aryl, $SO_2$O-Alkyl, $SO_2$O-Aryl, $SO_2$O-Alkyl-Aryl, Alkyl, Cycloalkyl, Heterocyclyl, Aryl, Heteroaryl und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum mit O(-Alkyl-O)$_p$-Alkyl, OP(O)(OAlkyl)$_2$, OP(O)(OAryl)$_2$, C(O)OR16, C(O)$NH_2$, C(O)NHR16, C (O)NR16R17, $SO_2$Alkyl, $SO_2$Aryl, P(O)(OH)$_2$, P(O)(OAlkyl)$_2$, P(O)(OAryl)$_2$, Cycloalkyl, Heterocyclyl, Aryl, Heteroaryl oder Alkylaryl substituiert sind, wobei p den Wert 1, 2, 3, 4 oder 5 annehmen kann und die Reste R16 und R17 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R16 und R17 zusammen einen Heterocyclyl-Ring bilden können,

unter der Voraussetzung, dass, wenn R3 oder R4 Alkyl-Heterocyclyl-substituiertes Aryl bedeutet, entsprechend R4 bzw. R3 $\neq$ Aryl ist,

und wobei der Arylrest ein- oder mehrfach, gleich oder verschieden substituiert ist mit Substituenten ausgewählt aus der Gruppe NR20-Alkyl, NH-R38, NHC(O)-R38, NR19C(O)-Alkyl, NR19C(O)-Cycloalkyl, NR19C(O)-Heterocyclyl, NR19C(O)-Aryl, NR19C(O)-Heteroaryl, NR18C(O)-Alkyl-Cycloalkyl, NR18C(O)-Alkyl-Heterocyclyl, NR19C (O)-Alkyl-Aryl, NR19C(O)-Alkyl-Heteroaryl, NR18C(O)O-R19, NR18C(O)NR18R18, O-R38, OC(O)-R38, OC(O)-Alkyl-Cycloalkyl, OC(O)-Alkyl-Heterocyclyl, OC(O)O-R19, OC(O)NR18R18, OP(O)(OAlkyl)$_2$, OP(O)(OAryl)$_2$, C(O)O-R38, C(O)NH-R38, C(O)NR20-Alkyl, C(O)NR19-Alkyl-R21, C(O)NR18O-R18, C(O)NR18NR18R18 und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum mit F, Cl, Br, I, CN, $CF_3$, $NH_2$, NH-Alkyl, NH-Aryl, N(Alkyl)$_2$, $NO_2$, SH, S-Alkyl, OH, $OCF_3$, O(-Alkyl-O)$_p$-Alkyl, O-Aryl, $OSO_3H$, OP(O)(OH)$_2$, OP(O)(OAlkyl)$_2$, OP(O)(OAryl)$_2$, CHO, C(O)OH, C(O) OR22, C(O)$NH_2$, C(O)NHR22, C(O)NR22R23, $SO_3H$, $SO_2$Alkyl, $SO_2$Aryl, P(O)(OH)$_2$, P(O)(OAlkyl)$_2$, P(O)(OAryl)$_2$, Alkyl, Cycloalkyl, Heterocyclyl, Aryl, Heteroaryl oder Alkylaryl substituiert sein können, wobei p den Wert 0, 1, 2, 3, 4 oder 5 annehmen kann und die Reste R22 und R23 unabhängig voneinander Alkyl, Cycloalkyl, Heterocyclyl, Aryl,

Heteroaryl, Alkyl-Cycloalkyl, Alkyl-Heterocyclyl, Alkyl-Aryl oder Alkyl-Heteroaryl sein können oder R22 und R23 zusammen einen Heterocyclyl-Ring bilden können,

substituiertes Heteroaryl, wobei der Heteroarylrest ein- oder mehrfach, gleich oder verschieden substituiert ist mit Substituenten ausgewählt aus der Gruppe NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, $N(Aryl)_2$, NHC(O)-Alkyl, NHC (O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, S-Alkyl, S-Aryl, S-Heteroaryl, O-Alkyl, O-Cycloalkyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, C(O)-Alkyl, C(O)-Aryl, C(O)-Heteroaryl, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)NH-Alkyl, C(O)NH-Cycloalkyl, C(O)NH-Heterocyclyl, C(O) NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2$NH-Alkyl, $SO_2$NH-Aryl, $SO_2$NH-Heteroaryl, $SO_2$NH-Alkyl-Aryl, $SO_2$O-Alkyl, $SO_2$O-Aryl, $SO_2$O-Alkyl-Aryl, Alkyl, Cycloalkyl, Heterocyclyl, Aryl, Heteroaryl und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum mit $O(\text{-Alkyl-O})_p$-Alkyl, $OP(O)(OAlkyl)_2$, OP(O) $(OAryl)_2$, C(O)OR16, $C(O)NH_2$, C(O)NHR16, C(O)NR16R17, $SO_2$Alkyl, $SO_2$Aryl, $P(O)(OH)_2$, $P(O)(OAlkyl)_2$, P(O) $(OAryl)_2$, Cycloalkyl, Heterocyclyl, Aryl, Heteroaryl oder Alkylaryl substituiert sind, wobei p den Wert 1, 2, 3, 4 oder 5 annehmen kann und die Reste R16 und R17 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R16 und R17 zusammen einen Heterocyclyl-Ring bilden können,

und wobei der Heteroarylrest ein- oder mehrfach, gleich oder verschieden substituiert ist mit Substituenten ausgewählt aus der Gruppe NR20-Alkyl, NH-R38, NHC(O)-R38, NR19C(O)-Alkyl, NR19C(O)-Cycloalkyl, NR19C(O)-Heterocyclyl, NR19C(O)-Aryl. NR19C(O)-Heteroaryl, NR18C(O)-Alkyl-Cycloalkyl, NR18C(O)-Alkyl-Heterocyclyl, NR19C(O)-Alkyl-Aryl, NR19C(O)-Alkyl-Heteroaryl, NR18C(O)O-R19, NR18C(O)NR18R18, $NHSO_2$-Alkyl-Heterocyclyl, O-R38, O-Heterocyclyl, OC(O)-R38, OC(O)-Alkyl-Cycloalkyl, OC(O)-Alkyl-Heterocyclyl, OC(O)O-R19, OC (O)NR18R18, $OP(O)(OAlkyl)_2$, $OP(O)(OAryl)_2$, C(O)O-R38, C(O)NH-R38, C(O)NR20-Alkyl, C(O)NR19-Alkyl-R21, C(O)NR18O-R18, C(O)NR18NR18R18 und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum mit F, Cl, Br, I, CN, $CF_3$, $NH_2$, NH-Alkyl, NH-Aryl, $N(Alkyl)_2$, $NO_2$, SH, S-Alkyl, OH, $OCF_3$, $O(\text{-Alkyl-O})_p$-Alkyl, O-Aryl, $OSO_3H$, $OP(O)(OH)_2$, $OP(O)(OAlkyl)_2$, $OP(O)(OAryl)_2$, CHO, C(O)OH, C(O)OR22, $C(O)NH_2$, C(O)NHR22, C(0)NR22R23, $SO_3H$, $SO_2$Alkyl, $SO_2$Aryl, $P(O)(OH)_2$, $P(O)(OAlkyl)_2$, $P(O)(OAryl)_2$, Alkyl, Cycloalkyl, Heterocyclyl, Aryl, Heteroaryl oder Alkylaryl substituiert sein können, wobei p den Wert 0, 1, 2, 3, 4 oder 5 annehmen kann und die Reste R22 und R23 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R22 und R23 zusammen einen Heterocyclyl-Ring bilden können,

NR24R25, wobei R24 -C(O)-R26, $-SO_2R26$, -C(O)OR26 oder -C(O)-NR27R28 sein kann und wobei R25 Wasserstoff, Alkyl, Cycloalkyl, Aryl oder Heteroaryl sein kann und wobei R26 Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl sein kann, und R27 und R28 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl sein können, oder R27 und R28 zusammen einen Heterocyclyl-Ring bilden können und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können,

und R18 Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R19 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R20 Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R21 Cycloalkyl, Heterocyclyl, Aryl, Heteroaryl sein kann,

und R38 Alkyl sein kann, wobei der Alkylrest gesättigt oder ungesättigt, geradkettig oder verzweigt sein kann, mit 9 bis 30 C-Atomen, d.h. $C_9$-$_{30}$-Alkanyle, $C_9$-$_{30}$-Alkenyle und $C_9$-$_{30}$-Alkinyle. Dabei weisen Alkenyle mindestens eine C-C-Doppelbindung und Alkinyle mindestens eine C-C-Dreifachbindung auf, wobei die Alkenyle sowohl in (E)-als auch in (Z)-Konformation vorliegen können. Es ist bevorzugt, dass der Alkylrest ausgewählt ist aus der Gruppe, die nonyl, decyl, dodecyl, hexadecyl, octadecyl, eicosyl, henicosyl, docosyl, tetracosyl, nonacosyl, octadecenyl, docosenyl, tetracosenyl und octadecinyl enthält,

bedeuten.

Gemäß einem weiteren Aspekt werden in der vorliegenden Anmeldung neue Verbindungen aus der Reihe der Pyrido[2,3-b]pyrazine gemäß der allgemeinen Formel I beschrieben,

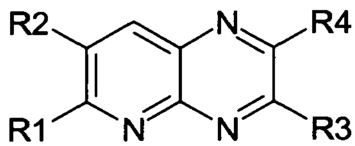

**I**

worin die Substituenten R1-R4 folgende Bedeutung haben:

R1 und R2 können unabhängig voneinander Wasserstoff oder NR5R6 bedeuten, unter der Voraussetzung, dass, wenn R1 = NR5R6 ist, R2 = H ist, und wenn R2 = NR5R6 ist, R1 = H ist,

wobei R5 Wasserstoff, Alkyl, R38, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum mit F, Cl, Br, I, CN, $CF_3$, $NH_2$, NH-Alkyl, NH-Aryl, $N(Alkyl)_2$, $NO_2$, SH, S-Alkyl, OH, $OCF_3$, O(-Alkyl-O)$_p$-Alkyl, O-Aryl, $OSO_3H$, OP(O)$(OH)_2$, OP(O)(OAlkyl)$_2$, OP(O)(OAryl)$_2$, CHO, C(O)OH, C(O)$NH_2$, C(O)OR12, C(O)NHR12, C(O)NR12R13, $SO_3H$, $SO_2$Alkyl, $SO_2$Aryl, P(O)$(OH)_2$, P(O)(OAlkyl)$_2$, P(O)(OAryl)$_2$, Alkyl, Cycloalkyl, Heterocyclyl, Aryl, Heteroaryl ein- oder mehrfach, gleich oder verschieden substituiert sein können, p den Wert 0, 1, 2, 3, 4 oder 5 annehmen kann und die Reste R12 und R13 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R12 und R13 zusammen einen Heterocyclyl-Ring bilden können

und R6:

-C(O)NR9-Y-R10 bedeuten kann, wobei Y unabhängig voneinander O oder NR11 sein kann

und R9 Wasserstoff, Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl sein kann und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits substituiert sein können,

und R10 und R11 unabhängig voneinander

Wasserstoff,

unsubstituiertes oder substituiertes Alkyl,

unsubstituiertes oder substituiertes Cycloalkyl,

unsubstituiertes oder substituiertes Heterocyclyl, wobei der Heterocyclylrest mit OH, O-Alkyl, O-Aryl, $NH_2$, NH-Alkyl, NH-Aryl, Alkyl, Alkyl-Aryl oder Aryl ein- oder mehrfach, gleich oder verschieden substituiert sein kann,

unsubstituiertes oder substituiertes Aryl, wobei der Arylrest mit F, Cl, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-R38, NHC(O)-Cycloalkyl, NHC (O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, $O(\text{-Alkyl-O})_p$-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C (O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, SO3H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, wobei p den Wert 0, 1, 2, 3, 4 oder 5 und n den Wert 1, 2 oder 3 annehmen kann,

unsubstituiertes oder substituiertes Heteroaryl, wobei der Heteroarylrest F, Cl, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-R38, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, $O(\text{-Alkyl-O})_p$-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2NH_2$, $SO_2NH$-Alkyl, $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, wobei p den Wert 0, 1, 2, 3, 4 oder 5 annehmen kann,

sein können, oder R10 und R11 zusammen einen Heterocyclyl-Ring bilden können,

R3 und R4 können unabhängig voneinander:

Wasserstoff

Hydroxyl

Halogen, wie Fluor, Chlor, Brom, Iod

unsubstituiertes oder substituiertes Alkyl, wobei der Alkylrest mit F, Cl, Br, I, CN, $CF_3$, $NH_2$, NH-Alkyl, NH-Aryl, N $(Alkyl)_2$, SH, S-Alkyl, OH, $OCF_3$, $O(\text{-Alkyl-O})_p$-Alkyl, 0-Aryl, $OSO_3H$, $OP(O)(OH)_2$, $OP(O)(OAlkyl)_2$, $OP(O)(OAryl)_2$, C(O)OH, C(O)OR14, C(O)$NH_2$, C(O)NHR14, C(O)NR14R15, $SO_3H$, $SO_2Alkyl$, $SO_2Aryl$, $P(O)(OH)_2$, $P(O)(OAlkyl)_2$, $P(O)(OAryl)_2$, Cycloalkyl, Heterocyclyl, Aryl oder Heteroaryl ein- oder mehrfach, gleich oder verschieden substituiert sein kann, wobei p den Wert 0, 1, 2, 3, 4 oder 5 annehmen kann und die Reste R14 und R15 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R14 und R15 zusammen einen Heterocyclyl-Ring bilden können,

unsubstituiertes oder substituiertes Aryl, wobei der Arylrest mit F, Cl, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, $N(Aryl)_2$, NR20-Alkyl, NHC(O)-Alkyl, NHC(O)-R38, NR19(O)-Alkyl, NHC(O)-Cycloalkyl, NR19C(O)-Cycloalkyl, NHC(O)-Heterocyclyl, NR19C(O)-Heterocyclyl, NHC(O)-Aryl, NR19C(O)-Aryl, NHC(O)-Heteroaryl, NR19C(O)-Heteroaryl, NR18C(O)-Alkyl-Cycloalkyl, NR18C(O)-Alkyl-Heterocyclyl, NHC(O)-Alkyl-Aryl, NR19C(O)-Alkyl-Aryl, NHC(O)-Alkyl-Heteroaryl, NR19C(O)-Alkyl-Heteroaryl, NR18C(O)O-R19, NR18C(O)NR18R18, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Heterocyclyl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-$(CH_2)_n$-O, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Cycloalkyl, OC(O)-Alkyl-Heterocyclyl OC(O)-Alkyl-Aryl, OC(O)-Alkyl-Heteroaryl, OC(O)O-R19, OC(O)NR18R18, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, $OP(O)(OAlkyl)_2$, $OP(O)(OAryl)_2$, C(O)-Alkyl, C(O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, C(O)NR20-Alkyl, C(O)NR19-Alkyl-R21,- C(O)NR18O-R18, -C(O)NR18NR18R18, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Alkyl, Alkyl-Cycloalkyl, Alkyl-Heterocyclyl, Alkyl-Aryl, Alkyl-Heteroaryl, Cycloalkyl, Heterocyclyl, Aryl oder Heteroaryl, ein- oder mehrfach, gleich oder verschieden substituiert sein kann, n den Wert 1,2 oder 3 annehmen kann, und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum substituiert sein können,

unsubstituiertes oder substituiertes Heteroaryl, wobei der Heteroarylrest mit F, Ci, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, $N(Aryl)_2$, NR20-Alkyl, NHC(O)-Alkyl, NHC(O)-R38, NR19C(0)-Alkyl, NHC(O)-Cycloalkyl, NR19C(O)-Cycloalkyl, NHC(O)-Heterocyclyl, NR19C(O)-Heterocyclyl, NHC(O)-Aryl, NR19C(O)-Aryl, NHC(O)-Heteroaryl, NR19C(O)-Heteroaryl, NR18C(O)-Alkyl-Cycloalkyl, NR18C(O)-Alkyl-Heterocyclyl, NHC(O)-Alkyl-Aryl, NR19C(O)-Alkyl-Aryl, NHC(O)-Alkyl-Heteroaryl, NR19C(O)-Alkyl-Heteroaryl, NR18C(O)O-R19, NR18C(O)NR18R18, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Heterocyclyl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Cycloalkyl, OC(O)-Alkyl-Heterocyclyl OC(O)-Alkyl-Aryl, OC(O)-Alkyl-Heteroaryl, OC(O)O-R19, OC(O)NR18R18, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, $OP(O)(OAlkyl)_2$, $OP(O)(OAryl)_2$, C(O)-Alkyl, C(O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, C(O)NR20-Alkyl, C(O)NR19-Alkyl-R21, -C(O)NR18O-R18, -C(O)NR18NR18R18, $SO_2NH_2$, $SO_2NH$-Alkyl, $SO_2NH$-Aryl, $SO_2NH$-Heteroaryl, $SO_2NH$-Alkyl-Aryl, S03H, $SO_2O$-Alkyl, $SO_2O$-Aryl, $SO_2O$-Alkyl-Aryl, Alkyl, Alkyl-Cycloalkyl, Alkyl-Heterocyclyl, Alkyl-Aryl, Alkyl-Heteroaryl, Cycloalkyl, Heterocyclyl, Aryl oder Heteroaryl ein- oder mehrfach, gleich oder verschieden substituiert sein kann, und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum substituiert sein können,

OR29, wobei R29 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl-, Heteroaryl-, Alkyl-Cycloalkyl, Alkyl-Heterocyclyl, Alkyl-Aryl oder Alkyl-Heteroarylsubstituenten ihrerseits wiederum substituiert sein können,

SR30, wobei R30 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl-, Alkyl-Heterocyclyl-, Alkyl-Aryl- oder Alkyl-Heteroarylsubstituenten ihrerseits wiederum substituiert sein können,

NR31 R32, wobei R31 und R32 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl, -C(O)-R33, -$SO_2$R33, -C(O)OR33 und -C(O)-NR34R35 sein können, wobei R33 Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl sein kann, und R34 und R35 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl sein können oder R34 und R35 zusammen einen Heterocyclyl-Ring bilden können,

oder R31 und R32 zusammen einen Heterocyclyl-Ring bilden können,

und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können,

und R18 Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R19 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R20 Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R21 Cycloalkyl, Heterocyclyl, Aryl, Heteroaryl sein kann,

und R38 Alkyl sein kann, wobei der Alkylrest gesättigt oder ungesättigt, geradkettig oder verzweigt sein kann, mit 9 bis 30 C-Atomen, d.h. $C_{9-30}$-Alkanyle, $C_{9-30}$-Alkenyle und $C_{9-30}$-Alkinyle. Dabei weisen Alkenyle mindestens eine C-C-Doppelbindung und Alkinyle mindestens eine C-C-Dreifachbindung auf, wobei die Alkenyle sowohl in (E)-als auch in (Z)-Konformation vorliegen können. Es ist bevorzugt, dass der Alkylrest ausgewählt ist aus der Gruppe, die nonyl, decyl, dodecyl, hexadecyl, octadecyl, eicosyl, henicosyl, docosyl, tetracosyl, nonacosyl, octadecenyl, docosenyl, tetracosenyl und octadecinyl enthält,

bedeuten.

Besonders bevorzugt sind Pyrido[2,3-b]pyrazin-Derivate der allgemeinen Formel I, wobei R2 = H ist.

Weiterhin besonders bevorzugt sind Pyrido[2,3-b]pyrazin-Derivate der allgemeinen Formel I, wobei R2 und R4 = H sind.

Besonders bevorzugt sind dabei die folgenden Pyrido[2,3-b]pyrazin-Derivate der allgemeinen Formel I enthalten und die in Form ihrer freien Base oder auch als pharmazeutisch annehmbare Salze physiologisch verträglicher Säuren vorliegen können:

1-Ethyl-3-(3-phenylethynyl-pyrido[2,3-b]pyrazin-6-yl)-harnstoff (Beispiel 1)

1-Ethyl-3-(3-thiophen-3-ylethynyl-pyrido[2,3-b]pyrazin-6-yl)-harnstoff (Bsp. 2)

1-(3-Cyclopropylethynyl-pyrido[2,3-b]pyrazin-6-yl)-3-ethyl-harnstoff (Bsp. 3)

1-[3-(3-Dimethylamino-prop-1-ynyl)-pyrido[2, 3-b]pyrazin-6-yl]-3-ethyl-harnstoff (Bsp. 4)

1-[3-((E)-2-Cyclohexyl-vinyl)-pyrido[2,3-b]pyrazin-6-yl]-3-ethyl-harnstoff (Bsp. 5)

1-Ethyl-3-[3-((E)-3-methoxy-propenyl)-pyrido[2,3-b]pyrazin-6-yl]-harnstoff (Bsp. 6)

1-Methoxy-3-(3-phenyl-pyrido[2,3-b]pyrazin-6-yl)-harnstoff (Bsp. 7)

1-Allyloxy-3-(3-phenyl-pyrido[2,3-b]pyrazin-6-yl)-harnstoff (Bsp. 8)

Kohlensäure-4-[6-(3-ethyl-harnstoff)-pyrido[2,3-b]pyrazin-3-yl]-phenyl-methyl-ester (Bsp. 9)

Kohlensäure-4-[6-(3-ethyl-harnstoff)-pyrido[2,3-b]pyrazin-3-yl]-phenyl-(2-methoxyethyl)-ester (Bsp. 10)

Kohlensäure-4-[6-(3-ethyl-harnstoff)-pyrido[2,3-b]pyrazin-3-yl]-phenyl-phenyl-ester (Bsp. 11)

Diethyl-carbaminsäure-4-[6-(3-ethyl-harnstoff)-pyrido[2,3-b]pyrazin-3-yl]-phenylester (Bsp. 12)

(E)-3-Phenyl-acrylsäure-4-[6-(3-ethyl-harnstoff)-pyrido[2,3-b]pyrazin-3-yl]-phenylester (Bsp. 13)

Nonadecansäure 4-[6-(3-ethyl-harnstoff)-pyrido[2,3-b]pyrazin-3-yl]-phenyl ester (Bsp. 14)

1-(3-Benzyl-pyrido[2,3-b]pyrazin-6-yl]-3-ethyl-harnstoff (Bsp. 15)

Die oben dargestellten generischen Verbindungen der allgemeinen Formel I und bevorzugten Ausführungsformen sowie die explizit genannten Pyridopyrazin-Verbindungen 1 bis 15 werden im folgenden gemeinsam als "erfindungsgemäße Verbindungen" bezeichnet.

Die zur Erläuterung der erfindungsgemäßen Verbindungen der allgemeinen Formel I, der bevorzugten Ausführungsformen und Verbindungen **1 bis 15** angegebenen Ausdrücke und Begriffe haben grundsätzlich, sofern in der Beschreibung oder in den Ansprüchen nichts anderes angegeben ist, folgende Bedeutungen:

Der Ausdruck "Alkyl" umfasst im Sinne dieser Erfindung acyclische gesättigte oder ungesättigte Kohlenwasserstoffreste, die verzweigt oder geradkettig sein können, mit 1 bis 8 C-Atomen, d.h. $C_{1-8}$-Alkanyle, $C_{2-8}$-Alkenyle und $C_{2-8}$-Alkinyle. Dabei weisen Alkenyle mindestens eine C-C-Doppelbindung und Alkinyle mindestens eine C-C-Dreifachbindung auf. Es ist bevorzugt, dass der Alkylrest ausgewählt ist aus der Gruppe, die Methyl, Ethyl, *n*-Propyl, 2-Propyl, *n*-Butyl, *sec*.-Butyl, *tert*.-Butyl, *n*-Pentyl, *iso*-Pentyl, *neo*-Pentyl, *n*-Hexyl, 2-Hexyl, *n*-Octyl, Ethylenyl (Vinyl), Ethinyl, Propenyl ($-CH_2CH=CH_2$; $-CH=CH-CH_3$, $-C(=CH_2)-CH_3$), Propinyl ($-CH_2-C\equiv CH$, $-C\equiv C-CH_3$), Butenyl, Butinyl, Pentenyl, Pentinyl, Hexenyl, Hexinyl, Heptenyl, Heptinyl, Octenyl und Octinyl enthält.

Der Ausdruck "Cycloalkyl" bedeutet für die Zwecke dieser Erfindung cyclische, nicht-aromatische Kohlenwasserstoffe mit 3-12 Kohlenstoffen, die gesättigt oder ungesättigt sein können. Die Bindung an die Verbindungen der allgemeinen Struktur I kann über jedes beliebige und mögliche Ringglied des Cycloalkyl-Restes erfolgen. Der Cycloalkyl-Rest kann auch Teil eines bi- oder polycyclischen Systems sein. Es ist bevorzugt, dass der Cycloalkyl-Rest ausgewählt ist aus der Gruppe, die Cyclopropyl, Cyclobutyl, Cyclopentyl, Cyclohexyl, Cycloheptyl und Cyclooctyl enthält.

Der Ausdruck "Heterocyclyl" steht für einen 3-, 4-, 5-, 6-, 7- oder 8-gliedrigen cyclischen organischen Rest, der mindestens 1, ggf. 2, 3, 4 oder 5 Heteroatome enthält, wobei die Heteroatome gleich oder verschieden sind und der cyclische Rest gesättigt oder ungesättigt, aber nicht aromatisch ist. Die Bindung an die Verbindungen der allgemeinen Struktur I kann über jedes beliebige und mögliche Ringglied des Heterocyclyl-Restes erfolgen. Der Heterocyclus kann auch Teil eines bi- oder polycyclischen Systems sein, wobei die Heteroatome ausgewählt sind aus der Gruppe Stickstoff, Sauerstoff und Schwefel. Es ist bevorzugt, dass der Heterocyclyl-Rest ausgewählt ist aus der Gruppe, die Tetrahydrofuryl, Pyrrolidinyl, Imidazolidinyl, Thiazolidinyl, Tetrahydropyranyl, Piperidinyl, Piperazinyl und Morpholinyl enthält.

Der Ausdruck "Aryl" bedeutet im Sinne dieser Erfindung aromatische Kohlenwasserstoffe mit 6 bis 14 C-Atomen, u.a. Phenyle, Naphthyle und Anthracenyle. Die Reste können auch mit weiteren gesättigten und ungesättigten Ringsystemen, wie beispielsweise Tetrahydrofuran, Tetrahydrothiophen, Pyrrolidin, Imidazolidin, Thiazolidin, Tetrahydropyran, Dihydropyran, Piperidin, Furan, Thiophen, Imidazol, Thiazol, Oxazol, Isoxazol kondensiert sein. Die Bindung an die Verbindungen der allgemeinen Struktur I kann über jedes beliebige und mögliche Ringglied des Aryl-Restes erfolgen.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome enthält, wobei die Heteroatome gleich oder verschieden sind. Die Bindung an die Verbindungen der allgemeinen Struktur I kann über jedes beliebige und mögliche Ringglied des Heteroaryl-Restes erfolgen. Der Heterocyclus kann auch Teil eines bi- oder polycyclischen Systems sein, wobei die Heteroatome ausgewählt sind aus der Gruppe Stickstoff, Sauerstoff und Schwefel. Es ist bevorzugt, dass der Heteroaryl-Rest ausgewählt ist aus der Gruppe, die Pyrrolyl, Furyl, Thienyl, Thiazolyl, Oxazolyl, Oxadiazolyl, Isoxazolyl, Pyrazolyl, Imidazolyl, Triazol, Tetrazol, Pyridinyl, Pyrimidinyl, Pyridazinyl, Pyrazinyl, Triazin, Phthalazinyl, Indolyl, Indazolyl, Indolizinyl, Chinolinyl, Isochinolinyl, Chinoxalinyl, Chinazolinyl, Pteridinyl, Carbazolyl, Phenazinyl, Phenoxazinyl, Phenothiazinyl, Acridinyl enthält.

Die Ausdrücke "Alkyl-Cycloalkyl", "Alkyl-Heterocyclyl", "Alkyl-Aryl" oder "Alkyl-Heteroaryl" bedeuten für die Zwecke der vorliegenden Erfindung, daß Alkyl, Cycloalkyl, Heterocyclyl, Aryl und Heteroaryl die oben definierten Bedeutungen haben und der Cycloalkyl-, Heterocyclyl-, Aryl- und Heteroaryl-Rest über eine $C_{1-8}$-Alkyl-Gruppe an die Verbindungen der allgemeinen Struktur I gebunden ist.

Im Zusammenhang mit "Alkyl", "Cycloalkyl", "Heterocyclyl", "Aryl", "Heteroaryl", "Alkyl-Cycloalkyl", "Alkyl-Heterocyclyl", "Alkyl-Aryl" und "Alkyl-Heteroaryl" versteht man unter dem Begriff substituiert im Sinne dieser Erfindung, insofern oben in der Beschreibung oder den Ansprüchen nicht explicit definiert, die Substitution eines oder mehrerer Wasserstoffreste durch F, Cl, Br, I, CN, $CF_3$, $NH_2$, NH-Alkyl, NH-Aryl, $N(Alkyl)_2$, $NO_2$, SH, S-Alkyl, OH, $OCF_3$, $O(\text{-Alkyl-}O)_p$-Alkyl, O-Aryl, $OSO_3H$, $OP(O)(OH)_2$, $OP(O)(OAlkyl)_2$, $OP(O)(OAryl)_2$, CHO, C(O)OH, C(O)OR36, $C(O)NH_2$, C(O) NHR36, C(O)NR36R37, $SO_3H$, $SO_2$Alkyl, $SO_2$Aryl, $P(O)(OH)_2$, $P(O)(OAlkyl)_2$, $P(O)(OAryl)_2$, Alkyl, Cycloalkyl, Heterocyclyl, Aryl, Heteroaryl oder Alkylaryl, wobei p den Wert 0, 1, 2, 3, 4 oder 5 annehmen kann, und wobei die Reste R36 und R37 unabhängig voneinander Alkyl, Cycloalkyl, Heterocyclyl, Aryl, Heteroaryl, Alkyl-Cycloalkyl, Alkyl-Heterocyclyl,

Alkyl-Aryl oder Alkyl-Heteroaryl sein können und R36 und R37 zusammen einen Heterocyclyl-Ring bilden können. Die Substituenten können gleich oder verschieden sein und die Substitution kann in jeder beliebigen und möglichen Position des Alkyl-, Cycloalkyl-, Heterocyclyl-, Aryl- und Heteroarylrestes vorkommen.

Unter mehrfach substituierten Resten sind solche zu verstehen, die entweder an verschiedenen oder an gleichen Atomen mehrfach, z. B. zwei- oder dreifach substituiert sind, beispielsweise dreifach am gleichen C-Atom wie im Falle von $CF_3$, $-CH_2CF_3$ oder an verschiedenen Stellen wie im Falle von $-CH(OH)-CH=CH-CHCl_2$. Die Mehrfachsubstitution kann mit dem gleichen oder verschiedenen Substituenten erfolgen.

Sofern die erfindungsgemäßen Verbindungen der allgemeinen Formel I mindestens ein Asymmetriezentrum aufweisen, können sie in Form ihrer Racemate, in Form der reinen Enantiomeren und/oder Diastereomeren oder in Form von Mischungen dieser Enantiomeren und/oder Diastereomeren vorliegen. Die Mischungen können in jedem beliebigen Mischungsverhältnis der Stereoisomeren vorliegen.

So lassen sich beispielsweise die erfindungsgemäßen Verbindungen gemäß der allgemeinen Formel I, welche ein oder mehrere Chiralitätszentren aufweisen und die als Racemate auftreten, nach an sich bekannten Methoden in ihre optischen Isomeren, also Enantiomere oder Diastereomere auftrennen. Die Trennung kann durch Säulentrennung an chiralen Phasen oder durch Umkristallisation aus einem optisch aktiven Lösungsmittel oder unter Verwendung einer optisch aktiven Säure oder Base oder durch Derivatisierung mit einem optisch aktiven Reagenz, wie beispielsweise einem optisch aktiven Alkohol, und anschließender Abspaltung des Restes erfolgen.

Sofern möglich, können die erfindungsgemäßen Verbindungen in Form der Tautomeren vorliegen.

Die erfindungsgemäßen Verbindungen der allgemeinen Formel I können, falls sie eine ausreichend basische Gruppe, wie zum Beispiel ein primäres, sekundäres oder tertiäres Amin besitzen, mit anorganischen und organischen Säuren in ihre physiologisch verträglichen Salze überführt werden. Vorzugsweise werden die pharmazeutisch annehmbaren Salze der erfindungsgemäßen Verbindungen gemäß der allgemeinen Formel I mit Salzsäure, Bromwasserstoffsäure, Schwefelsäure, Phosphorsäure, Methansulfonsäure, p-Toluolsulfonsäure, Kohlensäure, Ameisensäure, Essigsäure, Trifluoressigsäure, Sulfoessigsäure, Oxalsäure, Malonsäure, Maleinsäure, Bernsteinsäure, Weinsäure, Traubensäure, Äpfelsäure, Embonsäure, Mandelsäure, Fumarsäure, Milchsäure, Citronensäure, Glutaminsäure oder Asparaginsäure gebildet. Bei den gebildeten Salzen handelt es sich u.a. um Hydrochloride, Hydrobromide, Sulfate, Hydrogensulfate, Phosphate, Methansulfonate, Tosylate, Carbonate, Hydrogencarbonate, Formiate, Acetate, Triflate, Sulfoacetate, Oxalate, Malonate, Maleate, Succinate, Tartrate, Malate, Embonate, Mandelate, Fumarate, Lactate, Citrate, Glutaminate und Aspartate. Die Stöchiometrie der gebildeten Salze der erfindungsgemäßen Verbindungen kann dabei ganzzahlige oder nicht ganzzahlige Vielfache von eins betragen.

Die erfindungsgemäßen Verbindungen der allgemeinen Formel I können, falls sie eine ausreichend saure Gruppe, wie zum Beispiel die Carboxygruppe enthalten, mit anorganischen und organischen Basen in ihre physiologisch verträglichen Salze überführt werden. Als anorganische Basen kommen beispielsweise Natriumhydroxid, Kaliumhydroxid, Calciumhydroxid, als organische Basen Ethanolamin, Diethanolamin, Triethanolamin, Cyclohexylamin, Dibenzylethylendiamin und Lysin in Betracht. Die Stöchiometrie der gebildeten Salze der erfindungsgemäßen Verbindungen kann dabei ganzzahlige oder nicht ganzzahlige Vielfache von eins betragen.

Ebenfalls bevorzugt sind Solvate und insbesondere Hydrate der erfindungsgemäßen Verbindungen, die z. B. durch Kristallisation aus einem Lösungsmittel oder aus wässriger Lösung erhalten werden können: Es können sich dabei ein, zwei, drei oder beliebig viele Solvat- oder Wasser-Moleküle mit den erfindungsgemäßen Verbindungen zu Solvaten und Hydraten verbinden.

Es ist bekannt, dass chemische Substanzen Festkörper ausbilden, die in verschiedenen Ordnungszuständen vorliegen, die man als polymorphe Formen oder Modifikationen bezeichnet. Die verschiedenen Modifikationen einer polymorphen Substanz können sich in ihren physikalischen Eigenschaften stark unterscheiden. Die erfindungsgemäßen Verbindungen der allgemeinen Formel I können in verschiedenen polymorphen Formen vorliegen, dabei können bestimmte Modifikationen metastabil sein.

Ebenfalls können die erfindungsgemäßen Verbindungen in Form beliebiger Prodrugs wie beispielsweise Ester, Karbonate oder Phosphate vorkommen, bei denen die tatsächlich biologisch aktive Form erst durch Verstoffwechselung freigesetzt wird.

Es ist bekannt, dass chemische Substanzen im Körper zu Metaboliten umgewandelt werden die gegebenenfalls ebenfalls die erwünschte biologische Wirkung - unter Umständen sogar in stärker ausgeprägter Form - hervorrufen können.

Entsprechende Prodrugs und Metaboliten der erfindungsgemäßen Verbindungen sind als zur Erfindung gehörig anzusehen.

Es wurde nun überraschend und vorteilhaft festgestellt, dass die erfindungsgemäßen Verbindungen auch gleichzeitig auf zwei oder mehrere Signaltransduktionswege bzw. Enzyme solcher Wege einwirken bzw. modulierend oder hemmend wirken können. Dabei hat sich herausgestellt, dass die erfindungsgemäßen Verbindungen mit hoher Selektivität einwirken bzw. modulierend oder hemmend wirken.

Eine solche gleichzeitige, bspw. duale, Modulation oder Inhibition von zwei oder mehreren Signaltransduktionswegen, z.B. ras-Raf-Mek-Erk-Signaltweg, PI3K-Akt-Signalweg und/oder SAPK-Signalweg, spezieller Erk1/Erk2 und/oder PI3K und/oder Jnk und/oder p38, ist von Vorteil gegenüber der nur einfachen Modulation oder Inhibition eines Signaltransduktionsweges, da synergistische therapeutische Effekte bewirkt werden können, wie bspw. eine verstärkerte Apoptose und schnellere und effizientere Tumorregression.

Die überraschenden vorteilhaften Wirkungen der erfindungsgemäßen Verbindungen ermöglichen die Verfolgung multipler Therapieansätze in den physiologischen und/oder pathophysiologischen Zuständen oder Krankheitsbildern, die sensitiv sind für die Behandlung oder Modulation von bzw. vermittelt werden durch zwei oder mehrere Signaltransduktionswege.

Ferner wurde überraschend und vorteilhaft festgestellt, dass die erfindungsgemäßen Verbindungen auch mit hoher Selektivität einwirken bzw. modulierend oder hemmend wirken können auf den ras-Raf-Mek-Erk-Signaltransduktionsweg bzw. Enzyme dessen und dass die oben dargestellten multiplen Wirkmechansimen und Therapieansätze auch mit diesem Signalweg bzw. Enzymen Anwendung finden können.

Ferner wurde überraschend und vorteilhaft festgestellt, dass die erfindungsgemäßen Verbindungen auch mit hoher Selektivität einwirken bzw. modulierend oder hemmend wirken können auf den PI3K-Akt-Signaltransduktionsweg bzw. Enzyme dessen und dass die oben dargestellten multiplen Wirkmechansimen und Therapieansätze auch mit diesem Signalweg bzw. Enzymen Anwendung finden können.

Weiterhin wurde überraschend und vorteilhaft festgestellt, dass die erfindungsgemäßen Verbindungen auch mit hoher Selektivität einwirken bzw. modulierend oder hemmend wirken können auf den SAPK-Signaltransduktionsweg bzw. Enzyme dessen und dass die oben dargestellten multiplen Wirkmechansimen und Therapieansätze auch mit diesem Signalwegn bzw. Enzymen Anwendung finden können.

Desweiteren wurde überraschend und vorteilhaft festgestellt, dass die erfindungsgemäßen Verbindungen auch mit hoher Selektivität einwirken bzw. modulierend oder hemmend wirken können auf Enzyme, wie ATM, ATR, mTOR, DNA-PK und/oder hSMG-1, und dass die oben dargestellten multiplen Wirkmechansimen und Therapieansätze auch mit diesen Enzymen Anwendung finden können.

Unter dem Begriff "Modulation" wird erfindungsgemäß folgendes verstanden: "Aktivierung, partielle Aktivierung, Inhibierung, partielle Inhibierung". Hierbei liegt es im Fachwissen des durchschnittlichen Fachmanns, eine solche Aktivierung, partielle Aktivierung, Inhibierung oder partielle Inhibierung mittels der üblichen Mess- und Bestimmungsverfahren zu messen und zu bestimmen. So kann eine partielle Aktivierung bspw. in Relation zu einer vollständigen Aktivierung gemessen und bestimmt werden; ebenso eine partielle Inhibierung in Relation zu einer vollständigen Inhibierung.

Unter den Begriffen "Inhibierung, Inhibition und/oder Hemmung" wird erfindungsgemäß folgendes verstanden: "partielle oder vollständige Inhibierung, Inhibition und/oder Hemmung". Hierbei liegt es im Fachwissen des durchschnittlichen Fachmanns, eine solche partielle oder vollständige Inhibierung, Inhibition und/oder Hemmung mittels der üblichen Mess- und Bestimmungsverfahren zu messen und zu bestimmen. So kann eine partielle Inhibierung, Inhibition und/oder Hemmung bspw. in Relation zu einer vollständigen Inhibierung, Inhibition und/oder Hemmung gemessen und bestimmt werden.

Die Begriffe "Modulation" und "Inhibierung, Inhibition und/oder Hemmung" beziehen sich im Zusammenhang mit "Enzymen" und/oder "Kinasen" im Rahmen dieser Erfindung sowohl auf die inaktive Form (enzymatisch inaktiv) und/oder aktive Form (enzymatisch aktiv) des jeweiligen Enzyms und/oder Kinase. Dies bedeutet im Rahmen dieser Erfindung, dass eine erfindungsgemäße Verbindung ihre modulierende Wirkung an der inaktiven Form, aktiven Form oder beiden Formen des Enzyms und/oder Kinase entfalten kann.

Die erfinderische Aufgabe wurde in einem weiteren Aspekt überraschender Weise dadurch gelöst, dass die erfindungsgemäßen Verbindungen bereitgestellt werden, die als Wirkstoffe in Arzneimitteln zur Modulation von fehlgeleiteten zellulären Signaltransduktionsprozessen, insbesondere zur Beeinflussung der Funktion von aktiven und inaktiven Rezeptor-Tyrosinkinasen, sowie cytoplasmatischen Tyrosin-, Serin/Threonin- und Lipidkinasen, wie c-Raf, B-Raf, Mek, MAPKs, PDGFRbeta, Flt-3, IGF1R, PI3K, PKB/Akt1, c-Kit, c-Abl, FGFR1 und KDR, verwendet warden können.

Die erfinderische Aufgabe wurde in einem weiteren Aspekt überraschender Weise dadurch gelöst, dass die erfindungsgemäßen Verbindungen bereitgestellt werden, die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation des oder der Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" bewirkt wird.

In einem weiteren Aspekt wurde die erfinderische Aufgabe überraschender Weise dadurch gelöst, dass die erfindungsgemäßen Verbindungen bereitgestellt werden, die zur Herstellung eines Arzneimittels zur Behandlung oder Prophylaxe von durch Enzyme ausgewählt aus der Gruppe bestehend aus: "ATM, ATR, mTOR, DNA-PK, hSMG-1" vermittelte physiologischen und/oder pathophysiologischen Zuständen in Säugetieren verwendet werden können.

In einem weiteren Aspekt wurde die erfinderische Aufgabe überraschender Weise dadurch gelöst, dass die erfindungsgemäßen Verbindungen bereitgestellt werden, die die zur Herstellung eines Arzneimittels zur Behandlung oder Prophylaxe von physiologischen und/oder pathophysiologischen Zuständen in Säugetieren verwendet werden

können, wobei die Behandlung oder Prophylaxe durch Modulation eines oder mehrerer Enzyme ausgewählt aus der Gruppe bestehend aus: "ATM, ATR, mTOR, DNA-PK, hSMG-1" bewirkt wird.

In einer bevorzugten Ausführungsform werden die erfindungsgemäßen Verbindungen bereitgestellt zur Verwendung zur Herstellung eines Arzneimittels zur Behandlung und/oder Prophylaxe von durch den ras-Raf-Mek-Erk-Signaltransduktionsweg und den PI3K-Akt-Signaltransduktionsweg vermittelten physiologischen und/oder pathophysiologischen Zuständen in Säugetieren und/oder zur Herstellung eines Arzneimittels zur Behandlung oder Prophylaxe von physiologischen und/oder pathophysiologischen Zuständen in Säugetieren, wobei die Behandlung oder Prophylaxe durch Modulation des ras-Raf-Mek-Erk-Signaltransduktionswegs und des PI3K-Akt-Signaltransduktionswegs bewirkt wird.

In einem weiteren Aspekt wurde die erfinderische Aufgabe überraschender Weise dadurch gelöst, dass die erfindungsgemäßen Verbindungen bereitgestellt werden, die zur Herstellung eines Arzneimittels zur Behandlung oder Prophylaxe von durch den ras-Raf-Mek-Erk-Signaltransduktionsweg vermittelte physiologischen und/oder pathophysiologischen Zuständen in Säugetieren verwendet werden können.

In einem weiteren Aspekt wurde die erfinderische Aufgabe überraschender Weise dadurch gelöst, dass die erfindungsgemäßen Verbindungen bereitgestellt werden, die zur Herstellung eines Arzneimittels zur Behandlung oder Prophylaxe von durch den PI3K-Akt-Signaltransduktionsweg vermittelte physiologischen und/oder pathophysiologischen Zuständen in Säugetieren verwendet werden können.

In einem weiteren Aspekt wurde die erfinderische Aufgabe überraschender Weise dadurch gelöst, dass die erfindungsgemäßen Verbindungen bereitgestellt werden, die die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation des PI3K-Akt-Signaltransduktionswegs bewirkt wird.

In einer bevorzugten Ausführungsform werden die erfindungsgemäßen Verbindungen bereitgestellt zur Verwendung zur Herstellung eines Arzneimittels zur Behandlung und/oder Prophylaxe von durch den SAPK-Signaltransduktionsweg und den PI3K-Akt-Signaltransduktionsweg vermittelten physiologischen und/oder pathophysiologischen Zuständen in Säugetieren und/oder zur Herstellung eines Arzneimittels zur Behandlung oder Prophylaxe von physiologischen und/oder pathophysiologischen Zuständen in Säugetieren, wobei die Behandlung oder Prophylaxe durch Modulation des SAPK-Signaltransduktionswegs und des PI3K-Akt-Signaltransduktionswegs bewirkt wird.

In einem weiteren Aspekt wurde die erfinderische Aufgabe überraschender Weise dadurch gelöst, dass die erfindungsgemäßen Verbindungen bereitgestellt werden, die zur Herstellung eines Arzneimittels zur Behandlung oder Prophylaxe von durch den SAPK-Signaltransduktionsweg vermittelte physiologischen und/oder pathophysiologischen Zuständen in Säugetieren verwendet werden können.

In einem weiteren Aspekt wurde die erfinderische Aufgabe überraschender Weise dadurch gelöst, dass die erfindungsgemäßen Verbindungen bereitgestellt werden, die die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation des SAPK-Signaltransduktionswegs bewirkt wird.

In einer bevorzugten Ausführungsform werden die erfindungsgemäßen Verbindungen bereitgestellt zu den oben dargestellten Verwendungen, wobei die Modulation des ras-Raf-Mek-Erk-Signaltransduktionsweges bewirkt wird durch Modulation eines oder mehrerer Enzyme ausgewählt aus der Gruppe bestehend aus: "Tyrosinkinase, Serin/Threoninkinase, Rezeptor-Tyrosinkinase, cytoplasmatische Tyrosinkinase, cytoplasmatische Serin/Threoninkinase" und bevorzugt ausgewählt aus der Gruppe bestehend aus: "Erk, Erk1, Erk2".

In einer weiteren bevorzugten Ausführungsform werden die erfindungsgemäßen Verbindungen bereitgestellt zu den oben dargestellten Verwendungen, wobei die Modulation des PI3K-Akt-Signaltransduktionsweges bewirkt wird durch Modulation eines oder mehrerer Enzyme ausgewählt aus der Gruppe bestehend aus: "Lipidkinasen" und bevorzugt ausgewählt aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

In einer weiteren bevorzugten Ausführungsform werden die erfindungsgemäßen Verbindungen bereitgestellt zu den oben dargestellten Verwendungen, wobei die Modulation des SAPK-Signaltransduktionsweges bewirkt wird durch Modulation eines oder mehrerer Enzyme ausgewählt aus der Gruppe bestehend aus: "Tyrosinkinase, Serin/Threoninkinase, Rezeptor-Tyrosinkinase, cytoplasmatische Tyrosinkinase, cytoplasmatische Serin/Threoninkinase"und bevorzugt ausgewählt aus der Gruppe bestehend aus: "Jnk, Jnk1, Jnk2, Jnk3, p38, p38alpha, p38beta, p38gamma, p38delta".

In einem weiteren Aspekt wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation von zwei oder mehrerer Enzyme bewirkt wird.

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten

Behandlung oder Prophylaxe mindestens ein Enzym ausgewählt ist aus der Gruppe bestehend aus: "Erk, Erk1, Erk2" und mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "Jnk, Jnk1, Jnk2, Jnk3, p38, p38alpha, p38beta, p38gamma, p38delta" und mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "Erk, Erk1, Erk2" und mindestens ein Enzym ausgewählt ist aus der Gruppe bestehend aus: "ATM, ATR, mTOR, DNA-PK, hSMG-1".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "Jnk, Jnk1, Jnk2, Jnk3, p38, p38alpha, p38beta, p38gamma, p38delta" und mindestens ein Enzym ausgewählt ist aus der Gruppe bestehend aus: "ATM, ATR, mTOR, DNA-PK, hSMG-1".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p" und mindestens ein Enzym ausgewählt ist aus der Gruppe bestehend aus: "ATM, ATR, mTOR, DNA-PK, hSMG-1".

In einer ferner bevorzugten Ausführungsform werden die erfindungsgemäßen Verbindungen bereitgestellt zu den oben dargestellten Verwendungen, wobei die Modulation eine Inhibition ist.

Die erfindungsgemäßen Verbindungen können im Rahmen dieser Erfindung allen bekannten Säugetieren, insbesondere dem Menschen, zur Behandlung und/oder Prophylaxe verabreicht werden.

In einer anderen bevorzugten Ausführungsform werden die erfindungsgemäßen Verbindungen bereitgestellt zu den oben dargestellten Verwendungen, wobei das Säugetier ausgewählt ist aus der Gruppe bestehend aus: "Mensch, Nutztier, Vieh, Haustier, Rind, Kuh, Schaf, Schwein, Ziege, Pferd, Pony, Esel, Maulesel, Maultier, Hase, Kaninchen, Katze, Hund, Meerschweinchen, Hamster, Ratte, Maus" und bevorzugt ein Mensch ist.

Die erfindungsgemäßen Verbindungen können im Rahmen dieser Erfindung zur Behandlung und/oder Prophylaxe aller bekannten physiologischen und/oder pathophysiologischen Zustände verwendet warden.

In einer bevorzugten Ausführungsform werden die erfindungsgemäßen Verbindungen bereitgestellt zu den oben dargestellten Verwendungen, wobei die physiologischen und/oder pathophysiologischen Zustände ausgewählt sind aus der Gruppe bestehend aus: "maligne Tumore, benigne Tumore, entzündliche Erkrankungen, Entzündungen, Schmerzen, rheumatische Erkrankungen, arthritische Erkrankungen, HIV Infektionen, neurologische oder neurodegenerative Erkrankungen, Rheuma, Arthritis, AIDS, ARC (AIDS related complex), Kaposi-Sarkom, Tumore ausgehend vom Hirn und/oder Nervensystem und/oder Hirnhäuten, Demenz, Alzheimer, hyperproliferative Erkrankungen, Psoriasis, Endometriose, Narbenbildung, benigne Prostatahyperpläsie (BPH), Erkrankungen des Immunsystems, Autoimmunerkrankungen, Immundefizienzerkrankungen, Kolontumor, Magentumor, Darmtumor, Lungentumor, Pankreastumor, Ovarialtumor, Prostatatumor, Leukämie, Melanom, Lebertumor, Nierentumor, Kopftumor, Halstumor, Gliom, Brust-Tumor, Gebärmutterkrebs, Endometriumkrebs, Gebärmutterhalskrebs, Hirntumor, Adenokanthom, Blasenkrebs, Magentumor, Kolorectalrumor, Speiseröhrenkrebs, gynokologischer Tumor, Ovartumor, Schilddrüsenkrebs, Lymphom, chronische Leukämie, akute Leukämie, Restenose, Diabetis, diabetische Nephropathie, fibrotische Erkrankungen, cystische Fibrose, maligne Nephrosklerose, thrombotische Microangiopathie Syndrom, Organtransplantat-Abstoßung, Glomerulpathien, Erkrankungen des Stoffwechsels, solide/feste Tumore, rheumatische Arthritis, diabetische Retinopathie, Asthma, Allergien, allergische Erkrankungen, chronische obstruktive pulmonare Erkrankungen, inflammatorische Bowel-Erkrankung, Fibrose, Atheriosklerose, Herzerkrankungen, cardiovaskuläre Erkrankungen, Erkrankungen des Herzmuskels, vaskuläre Erkrankungen, angiogenetische Erkrankungen, Nierenerkrankungen, Rhinitis, Grave Erkrankung, fokale Ischemie, Herzversagen, Ischemie, kardische Hypertrophie, Nierenversagen, kardische Myozyten Fehlfunktion, Bluthochdruck, Vasoconstriktion, Schlaganfall, anaphylaktischer Schock, Blutplättchen-Verklumpung, Skelettmuskelatrophie, Fettleibigkeit, Übergewicht, Glukose Homeostase, kongestive Herzinsuffizienz, Angina, Herzanfall, Herzinfarkt, Hyperglykämie, Hypoglykämie, Hypertension".

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels zur Behandlung oder Prophylaxe von physiologischen und/oder pathophysiologischen Zuständen in Säugetieren, wobei das Arzneimittel mindestens eine weitere pharmakologisch aktive Substanz umfasst.

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels zur Behandlung oder Prophylaxe von physiologischen und/oder pathophysiologischen Zuständen in Säugetieren, wobei das Arzneimittel vor und/oder während und/oder nach der Behandlung mit mindestens einer weiteren pharmakologisch aktiven Substanz verabreicht wird.

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels zur Behandlung oder Prophylaxe von physiologischen und/oder pathophysiologischen Zuständen in Säugetieren, wobei das Arzneimittel vor und/oder während und/oder nach der Behandlung Strahlentherapie und/oder Chirurgie verabreicht wird.

Die erfindungsgemäßen Verbindungen können dabei Rahmen dieser Erfindung mit allen bekannten pharmakologisch aktiven Substanzen in einer Kombinationstherapie wie dargestellt verabreicht werden.

In einer bevorzugten Ausführungsform werden die erfindungsgemäßen Verbindungen bereitgestellt zu den oben dargestellten Verwendungen, wobei die weitere pharmakologisch aktive Substanz ausgewählt ist aus der Gruppe bestehend aus: "DNA Topoisomerase I und/oder II Inhibitoren, DNA Interkalatoren, alkylierende Agentien, Microtubuli Destabilisatoren, Hormon- und/oder Wachstumsfaktor-Rezeptor-Agonisten und/oder -Antagonisten, Antikörper gegen Wachstumsfaktoren und deren Rezeptoren, Kinase Inhibitoren, Antimetabolite".

In einer bevorzugten Ausführungsform werden die erfindungsgemäßen Verbindungen bereitgestellt zu den oben dargestellten Verwendungen, wobei die weitere pharmakologisch aktive Substanz ausgewählt ist aus der Gruppe bestehend aus: "Asparaginase, Bleomycin, Carboplatin, Carmustin, Chlorambucil, Cisplatin, Colaspase, Cyclophosphamid, Cytarabin, Dacarbazin, Dactinomycin, Daunorubicin, Doxorubicin(Adriamycin), Epirubicin, Etoposide, 5-Fluorouracil, Hexamethylmelamin, Hydroxharnstoff, Ifosfamid, Irinotecan, Leucovorin, Lomustin, Mechlorethamin,6-Mercaptopurin, Mesna, Methotrexat, Mitomycin C, Mitoxantrone, Prednisolon, Prednison, Procarbazin, Raloxifen, Streptozocin, Tamoxifen, Thioguanin, Topotecan, Vinblastin, Vincristin, Vindesin, Aminoglutethimid, L-Asparaginase, Azathioprin, 5-Azacytidin cladribin, Busulfan, Diethylstilbestrol, 2', 2'-Difluorodeoxycytidin, Docetaxel, Erythrohydroxynonyladenin, Ethinylestradiol, 5-Fluorodeoxyuridin, 5- Fluorodeoxyuridin Monophosphat, Fludarabin Phosphate, Fluoxymesteron, Flutamid, Hydroxyprogesteron Caproat, Idarubicin, Interferon, Medroxyprogesteron Acetat, Megestrol Acetat, Melphalan, Mitotan, Paclitaxel, Oxaliplatin, Pentostatin, N-Phosphonoacetyl-L-aspartat (PALA), Plicamycin, Semustin, Teniposide, Testosteron Propionat, Thiotepa, Trimethylmelamin, Uridine, Vinorelbin, Epothilon, Gemcitabin, Taxotere, BCNU, CCNU, DTIC, 5-Fluorouarcil, Herceptin, Avastin, Erbitux, Sorafenib, Gleevec, Iressa, Tarceva, Rapamycin, Actinomycin D".

Die orale Verabreichung kann beispielsweise in fester Form als Tablette, Kapsel, Gelkapsel, Dragee, Granulat oder Pulver, jedoch auch in Form einer trinkbaren Lösung erfolgen. Zur oralen Verabreichung können die neuen, erfindungsgemäßen Verbindungen, wie vorstehend definiert, mit bekannten und gewöhnlich verwendeten, physiologisch verträglichen Hilfs- und Trägerstoffen kombiniert werden, wie z.B. Gummiarabikum, Talkum, Stärke, Zucker wie z.B. Mannit, Methylcellulose, Lactose, Gelatine, oberflächenaktive Mittel, Magnesiumstearat, Cyclodextrine, wässrige oder nichtwässrige Trägerstoffe, Verdünnungsmittel, Dispergiermittel, Emulgatoren, Schmiermittel, Konservierungsstoffe und Geschmacksstoffe (z.B. etherische Öle). Die erfindungsgemäßen Verbindungen können auch in einer mikropartikulären, z.B. nanopartikulären Zusammensetzung dispergiert sein.

Die nicht orale Verabreichung kann beispielsweise durch intravenöse, subkutane oder intramuskuläre Injektion steriler wässriger oder öliger Lösungen, Suspensionen oder Emulsionen, mittels Implantaten oder durch Salben, Cremes oder Suppositorien erfolgen. Gegebenenfalls kann auch eine Verabreichung als Retardform erfolgen. Implantate können inerte Materialen enthalten, z.B. biologisch abbaubare Polymere oder synthetische Silicone wie z.B. Silicongummi. Eine intravaginale Verabreichung kann z.B. mittels Vaginalringen erfolgen. Eine intrauterine Verabreichung kann z.B. mittels Diaphragmen oder anderer geeigneter intrauteriner Vorrichtungen erfolgen. Darüber hinaus ist auch eine transdermale Verabreichung, insbesondere mittels einer dazu geeigneten Formulierung und/oder geeigneter Mittel wie z.B. Pflaster, vorgesehen.

Die erfindungsgemäßen Arzneimittel können in einer geeigneten Darreichungsform auf die Haut, epicutan als Lösung, Suspension, Emulsion, Schaum, Salbe, Paste oder Pflaster; über die Mund- und Zungenschleimhaut, buccal, lingual oder sublingual als Tablette, Pastille, Dragees, Linctus oder Gurgelwasser; über die Magen- und Darmschleimhaut, enteral als Tablette, Dragees, Kapsel, Lösung, Suspension oder Emulsion; über die Rectumschleimhaut, rectal als Suppositorium, Rectalkapsel oder Salbe; über die Nasenschleimhaut, nasal als Tropfen, Salben oder Spray; über das Bronchial- und Alveolarepithel, pulmonal oder per inhalationem als Aerosol oder Inhalat; über die Conjunctiva, conjunctival als Augentropfen, Augensalbe, Augentabletten, Lamellae oder Augenwasser; über die Schleimhäute der Genitalorgane, intravaginal als Vaginalkugeln, Salben und Spülung, intrauterin als Uterus-Pessare; über die ableitenden Harnwege, intraurethral als Spülung, Salbe oder Arzneistäbchen; in eine Arterie, intraarteriell als Injektion; in eine Vene,

intravenös als Injektion oder Infusion; in die Haut, intracutan als Injektion oder Implantat; unter die Haut, subcutan als Injektion oder Implantat; in den Muskel, intramusculär als Injektion oder Implantat; in die Bauchhöhle, intraperitoneal als Injektion oder Infusion verabreicht werden.

Die erfindungsgemäßen Verbindungen der allgemeinen Formel I können in Hinblick auf praktische therapeutische Erfordernisse mittels geeigneter Maßnahmen in ihrer Arzneistoffwirkung verlängert werden. Dieses Ziel kann auf chemischem und/oder galenischem Wege erreicht werden. Beispiele für die Erzielung einer Wirkungsverlängerung sind der Einsatz von Implantaten und Liposomen, die Bildung von schwerlöslichen Salzen und Komplexen oder der Einsatz von Kristall-Suspensionen.

Wie bereits vorstehend erläutert, können die neuen, erfindungsgemäßen Verbindungen auch mit weiteren pharmazeutisch aktiven Wirkstoffen kombiniert werden. Im Rahmen einer Kombinationstherapie können die einzelnen wirksamen Bestandteile gleichzeitig oder getrennt verabreicht werden, und zwar entweder auf demselben Wege (z.B. oral) oder auf getrennten Wegen (z.B. oral und als Injektion). Sie können in gleichen oder unterschiedlichen Mengen in einer Einheitsdosis vorliegen bzw. verabreicht werden. Es kann auch ein bestimmtes Dosierungsregime angewendet werden, sofern dies zweckmäßig erscheint. Auf diese Weise können auch mehrere der neuen, erfindungsgemäßen Verbindungen miteinander kombiniert werden.

Die Dosierung kann je nach Art der Indikation, der Schwere der Erkrankung, der Art der Verabreichung, dem Alter, Geschlecht, Körpergewicht und der Sensitivität des zu behandelnden Subjekts in einem breiten Rahmen variieren. Es entspricht den Fähigkeiten eines Fachmanns, eine "pharmakologisch wirksame Menge" der kombinierten pharmazeutischen Zusammensetzung zu bestimmen. Die Verabreichung kann in einer einzigen Dosis oder mehreren getrennten Dosierungen erfolgen.

Eine geeignete Einheitsdosis ist z.B. 0,001 mg bis 100 mg des Wirkstoffs, d.h. mindestens einer erfindungsgemäßen Verbindung und gegebenenfalls eines weiteren pharmazeutisch aktiven Wirkstoffs, pro kg Körpergewicht eines Patienten.

In einem weiteren Aspekt der vorliegenden Erfindung sind demnach auch pharmazeutische Zusammensetzungen umfassend eine pharmakologisch aktive Menge mindestens einer erfindungsgemäßen Verbindung, bevorzugt **Verbindung 1, 2, 3, 4, 5, 6, 7, 8, 9, 10, 11, 12, 13, 14,** und/oder **Verbindung 15,** sowie gegebenenfalls pharmazeutisch verträgliche Träger- und/oder Hilfsstoffe, von der vorliegenden Erfindung erfasst.

Bevorzugte und besonders bevorzugte pharmazeutische Zusammensetzungen sind jene, die mindestens eine der vorstehend genannten bevorzugten erfindungsgemäßen Verbindungen umfassen, In pharmazeutischen Zusammensetzungen gemäß der vorliegenden Erfindung können neben mindestens einer erfindungsgemäßen Verbindung, wie vorstehend definiert, auch noch mindestens ein weiterer pharmazeutisch aktiver Wirkstoffe vorliegen, wie vorstehend bereits näher aufgeführt.

In den erfindungsgemäßen pharmazeutischen Zusammensetzungen liegt mindestens eine der neuen, erfindungsgemäßen Verbindungen, wie vorstehend definiert, in einer pharmakologischen aktiven Menge, vorzugsweise in einer Einheitsdosis, z.B. der vorstehend genannten Einheitsdosis, vor, und zwar vorzugsweise in einer Verabreichungsform, die eine orale Verabreichung ermöglicht.

Bezüglich der erfindungsgemäße Verbindungen umfassende pharmazeutische Zusammensetzungen sowie bezüglich der Verwendung der erfindungsgemäßen Verbindungen als Arzneimittel sei hinsichtlich Verwendungs- und Verabreichungsmöglichkeiten auf das bereits im Zusammenhang mit der Verwendung der neuen, erfindungsgemäßen Verbindungen selbst, Gesagte verwiesen.

In einem weiteren Aspekt der vorliegenden Erfindung wurde die erfinderische Aufgabe überraschender Weise gelöst durch die Bereitstellung eines Kits umfassend eine pharmakologisch aktive Menge mindestens einer bevorzugten erfindungsgemäßen Verbindung, wie oben dargestellt, und eine pharmakologisch aktive Menge mindestens eines weiteren pharmakologisch aktiven Wirkstoffs wie vorstehend definiert.

## Allgemeine Synthesevorschriften für die erfindungsgemäßen Verbindungen der allgemeinen Formel I

Die Verfahren zur Herstellung erfindungsgemäßer substituierter Pyrido[2,3-b]pyrazine werden nachstehend erläutert.

Die Verbindungen der allgemeinen Formel I sind gemäß der folgenden Schemata (Schema 1 - 8) bzw. entsprechenden, dem Fachmann bekannten Verfahren erhältlich:

Schema 1

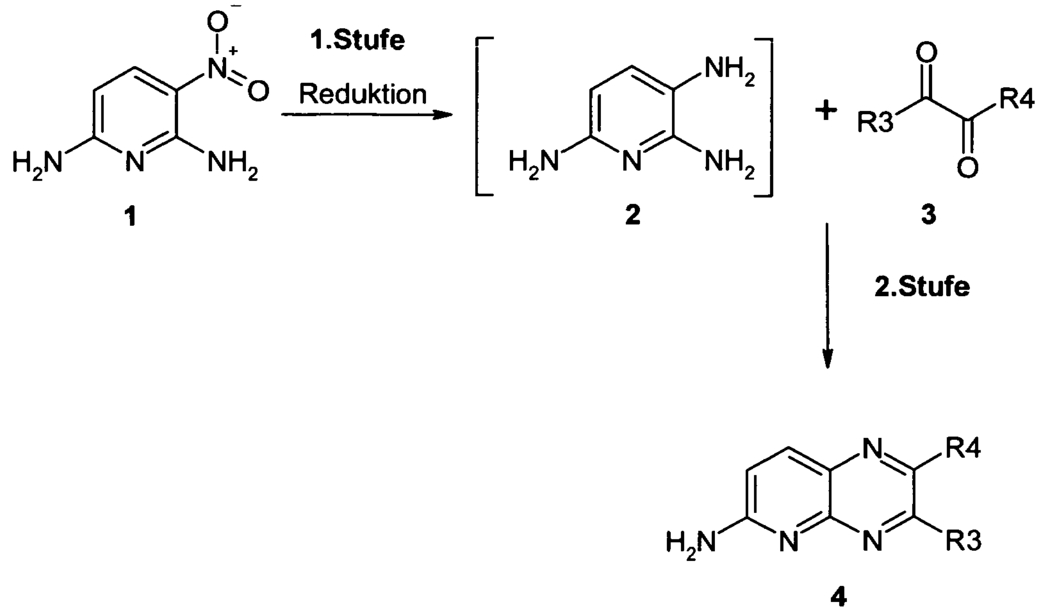


1 2 3 4

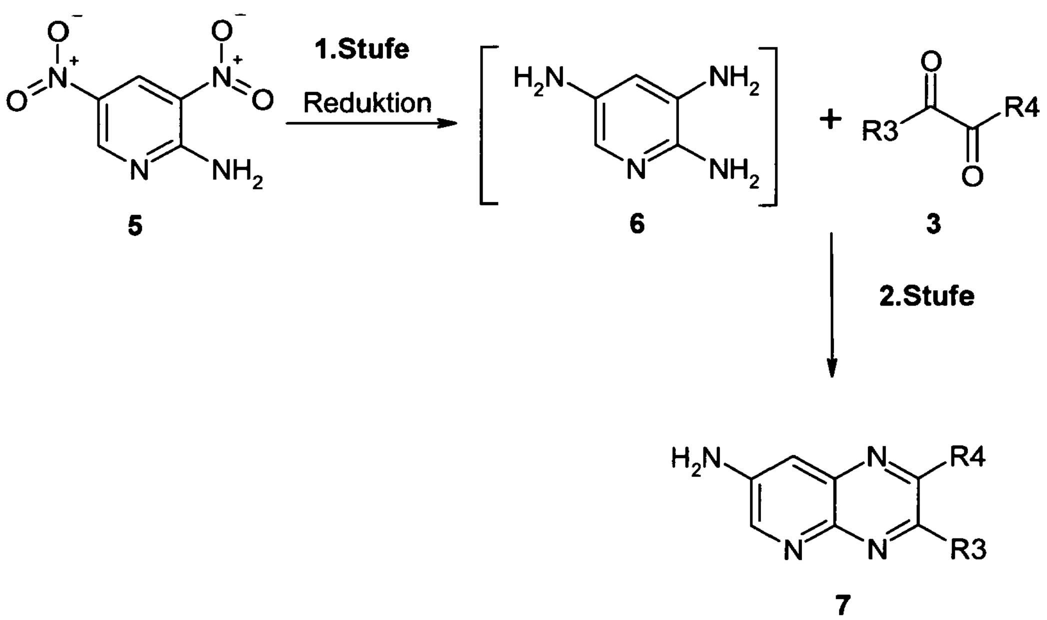


5 6 3 7

Vorstufen für ausgewählte Beispiele der erfindungsgemäßen Pyrido[2,3-b]pyrazine gemäß der allgemeinen Formel I, bei denen die Substituenten R2 und R4 mit Wasserstoff substituiert sein sollen, sind beispielsweise nach dem Verfahren in Schema 2 bzw. einem entsprechenden, dem Fachmann bekannten Verfahren erhältlich

Schema 2

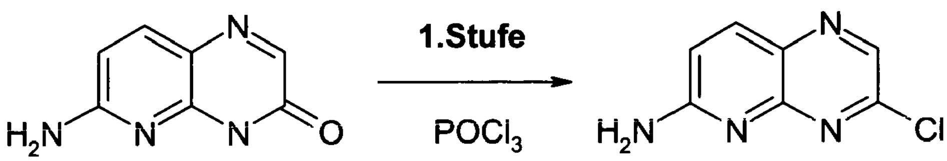


8 9

Vorstufen für ausgewählte Beispiele der erfindungsgemäßen Pyrido[2,3-b]pyrazine gemäß der allgemeinen Formel I, bei denen die Substituenten R3 und/oder R4 die Reste OR31, SR32, NR33R34 sein sollen, sind beispielsweise nach dem Verfahren in Schema 3 bzw. einem entsprechenden, dem Fachmann bekannten Verfahren erhältlich.

Schema 3

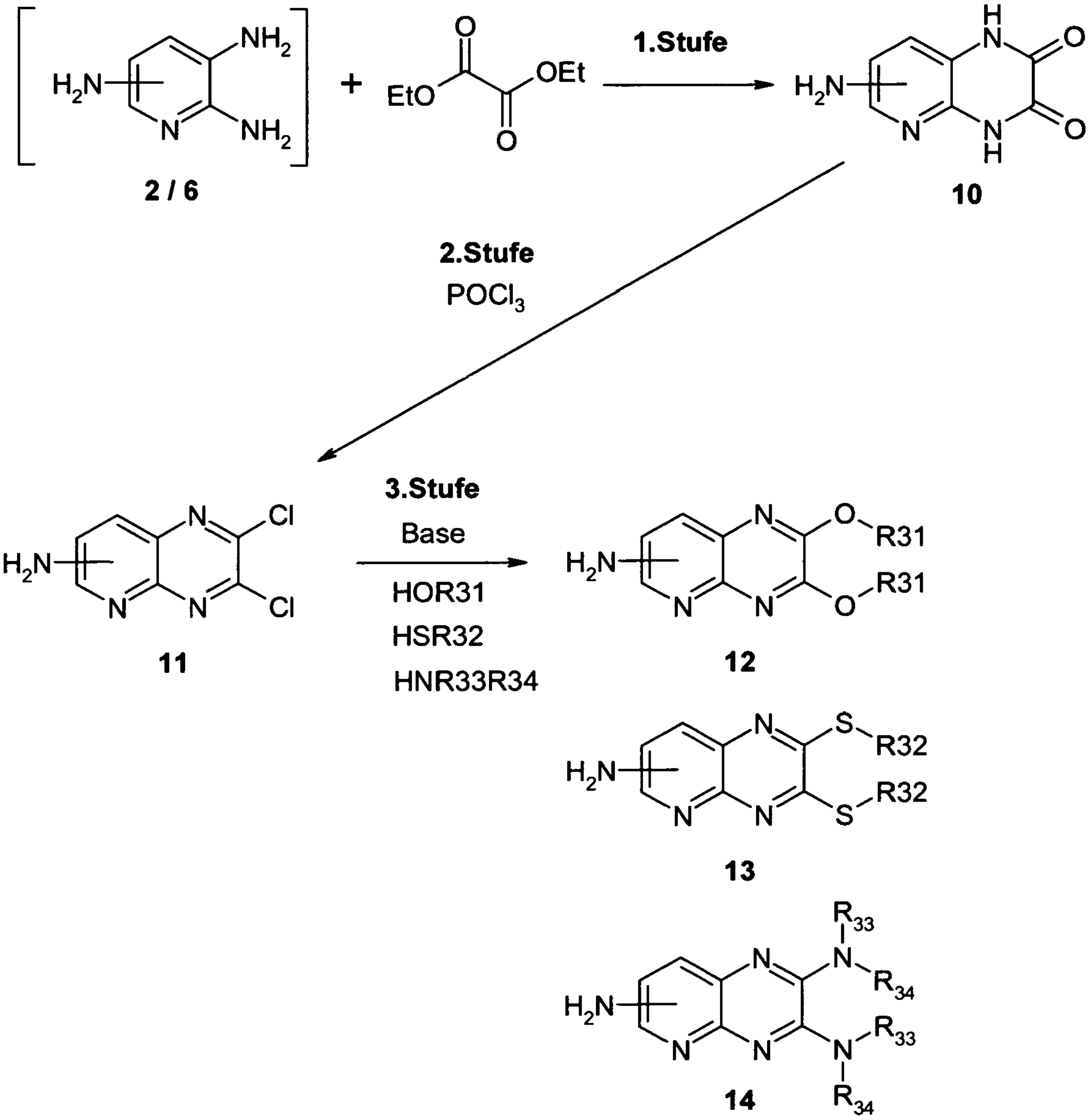


2 / 6

10

11

12

13

14

Vorstufen für ausgewählte Beispiele der erfindungsgemäßen Pyrido[2,3-b]pyrazine gemäß der allgemeinen Formel I, bei denen der Substituent R9 ≠ H sein soll, sind beispielsweise nach dem Verfahren in Schema 4 bzw. einem entsprechenden, dem Fachmann bekannten Verfahren erhältlich.

Schema 4

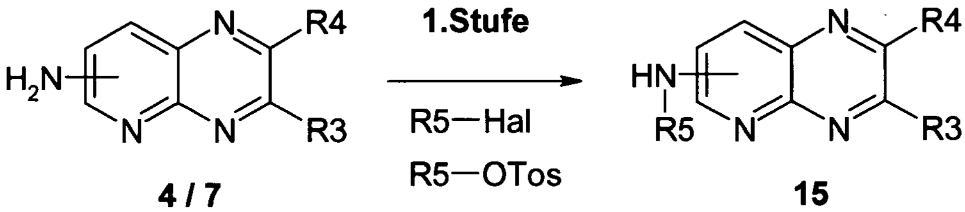


4 / 7

15

Die Umsetzung der Vorstufen **4, 7, 9** und **15** aus den Schemata 1-4 zu den erfindungsgemäßen substituierten Pyrido[2,3-b]pyrazinen gemäß der allgemeinen Formel I kann beispielsweise nach den Verfahren in Schema 5 bzw. einem entsprechenden, dem Fachmann bekannten Verfahren erfolgen.

Schema 5

**1.Stufe**

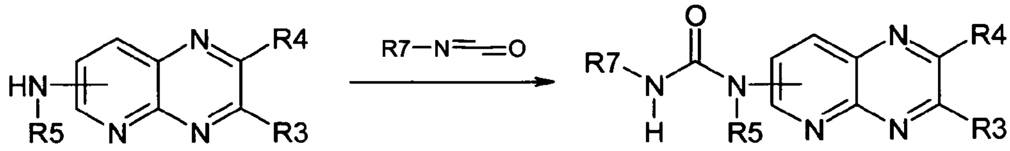

**4 / 7 / 9 / 15** **16**

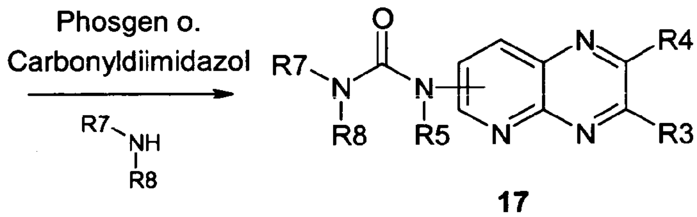


**17**

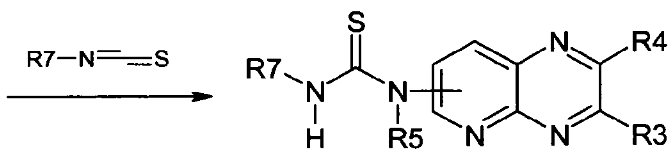

**18**

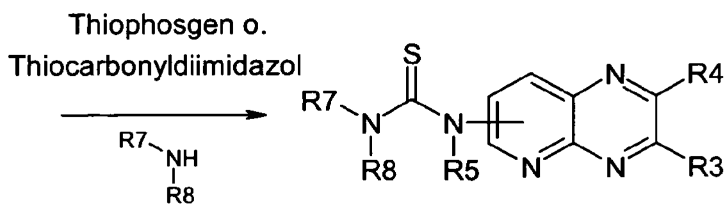


**19**

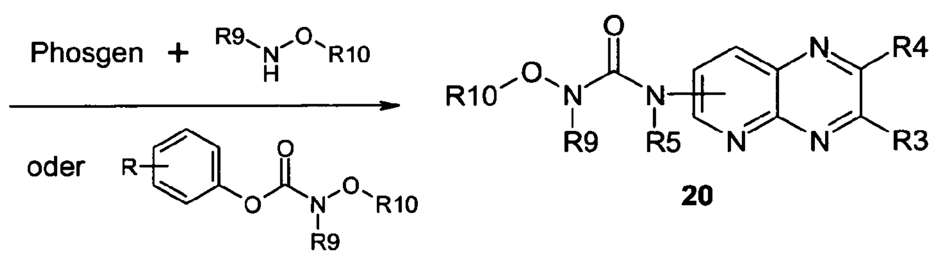


**20**

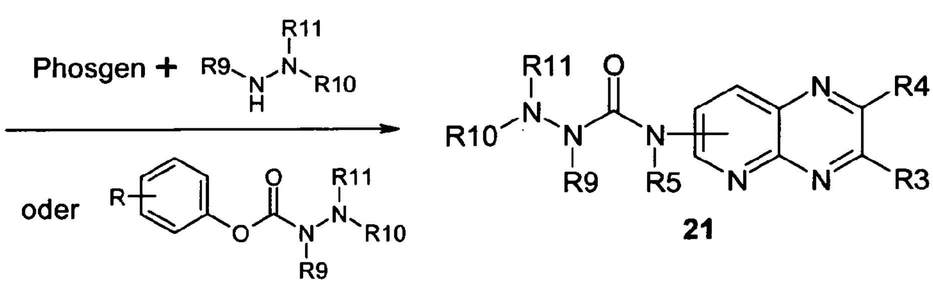


**21**

Ausgewählte Beispiele der erfindungsgemäßen Pyrido[2,3-b]pyrazine gemäß der allgemeinen Formel I, bei denen die Substituenten R3 und/oder R4 ausgewählte substituierte Aryl- Heteroaryl-, Alkyl-, Alkenyl- oder Alkinyl-Reste sein können, sind beispielsweise nach dem Verfahren in Schema 6 oder entsprechenden, dem Fachmann bekannten Verfahren erhältlich.

Schema 6

1.Stufe

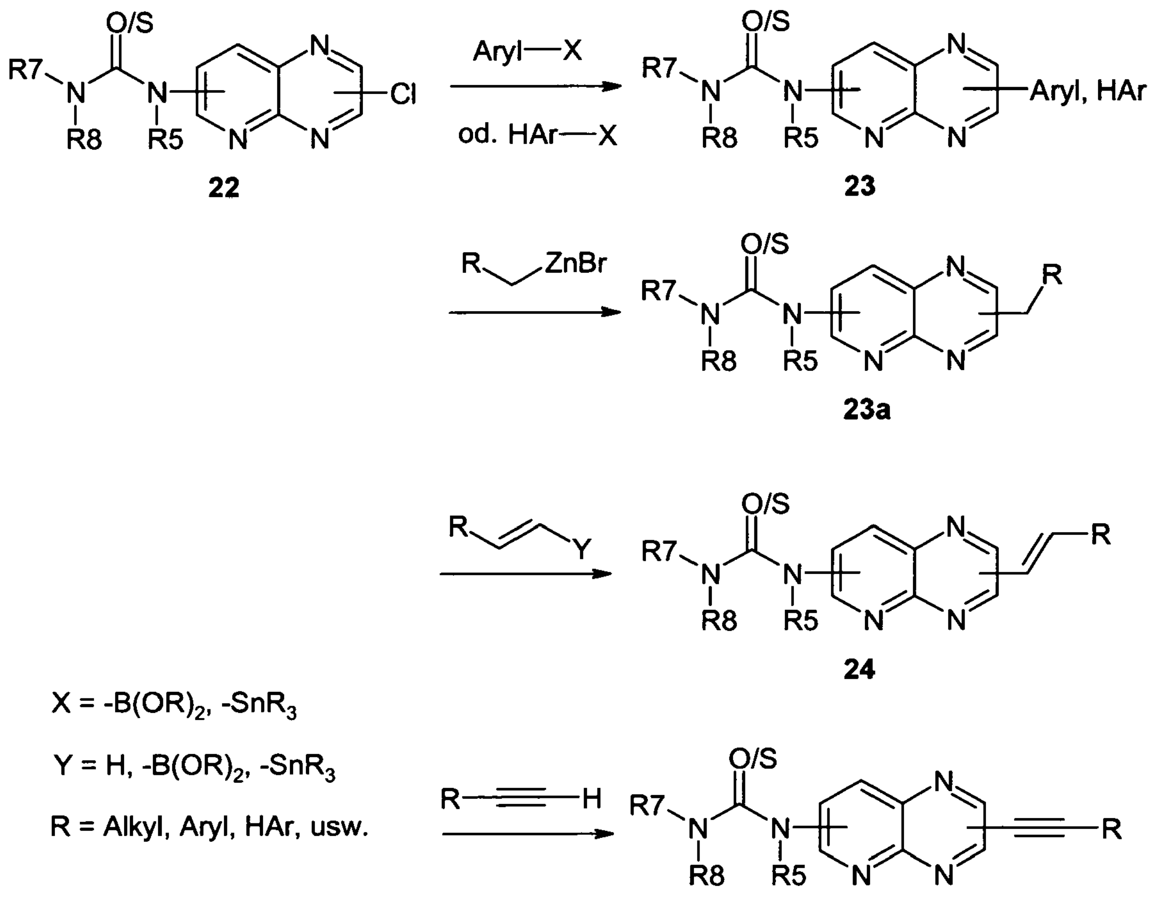


22

23

23a

24

X = $-B(OR)_2$, $-SnR_3$

Y = H, $-B(OR)_2$, $-SnR_3$

R = Alkyl, Aryl, HAr, usw.

25

Ausgewählte Beispiele der erfindungsgemäßen Pyrido[2,3-b]pyrazine gemäß der allgemeinen Formel I, bei denen die Substituenten R3 und/oder R4 -N-C(O)-, $-N-SO_2-$, -N-C(O)-O- und -N-C(O)-N- sein können, sind beispielsweise nach dem Verfahren in Schema 7 oder entsprechenden, dem Fachmann bekannten Verfahren erhältlich.

Schema 7

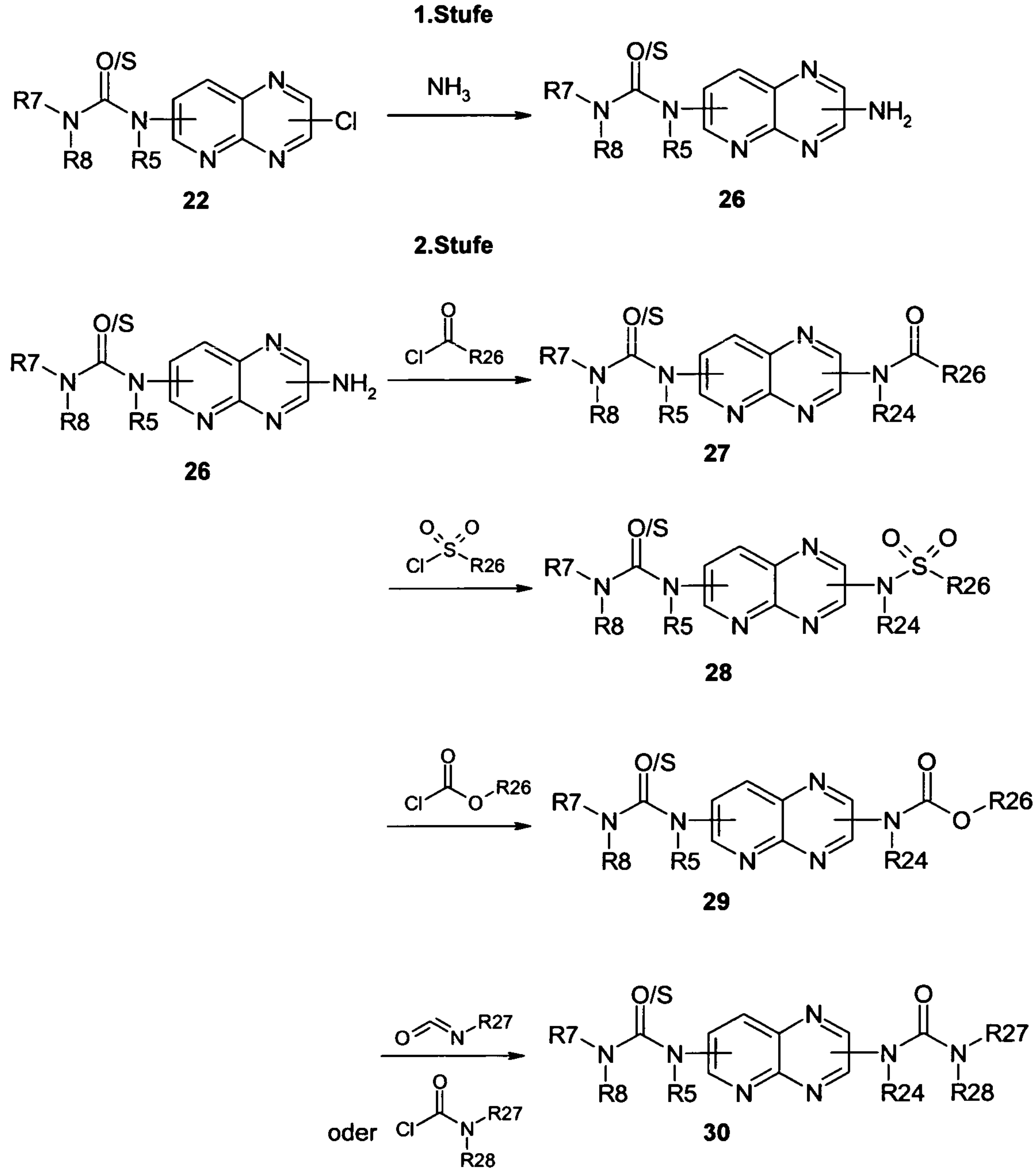


1.Stufe

22 → 26

2.Stufe

26 → 27, 28, 29, 30

Ausgewählte Beispiele der erfindungsgemäßen Pyrido[2,3-b]pyrazine gemäß der allgemeinen Formel I, bei denen die Substituenten R3 und/oder R4 ausgewählte Harnstoff-, Carbamat- oder Carbonat-substituierte Reste sein können, sind beispielsweise nach dem Verfahren in Schema 8 oder entsprechenden, dem Fachmann bekannten Verfahren erhältlich.

Schema 8

1.Stufe

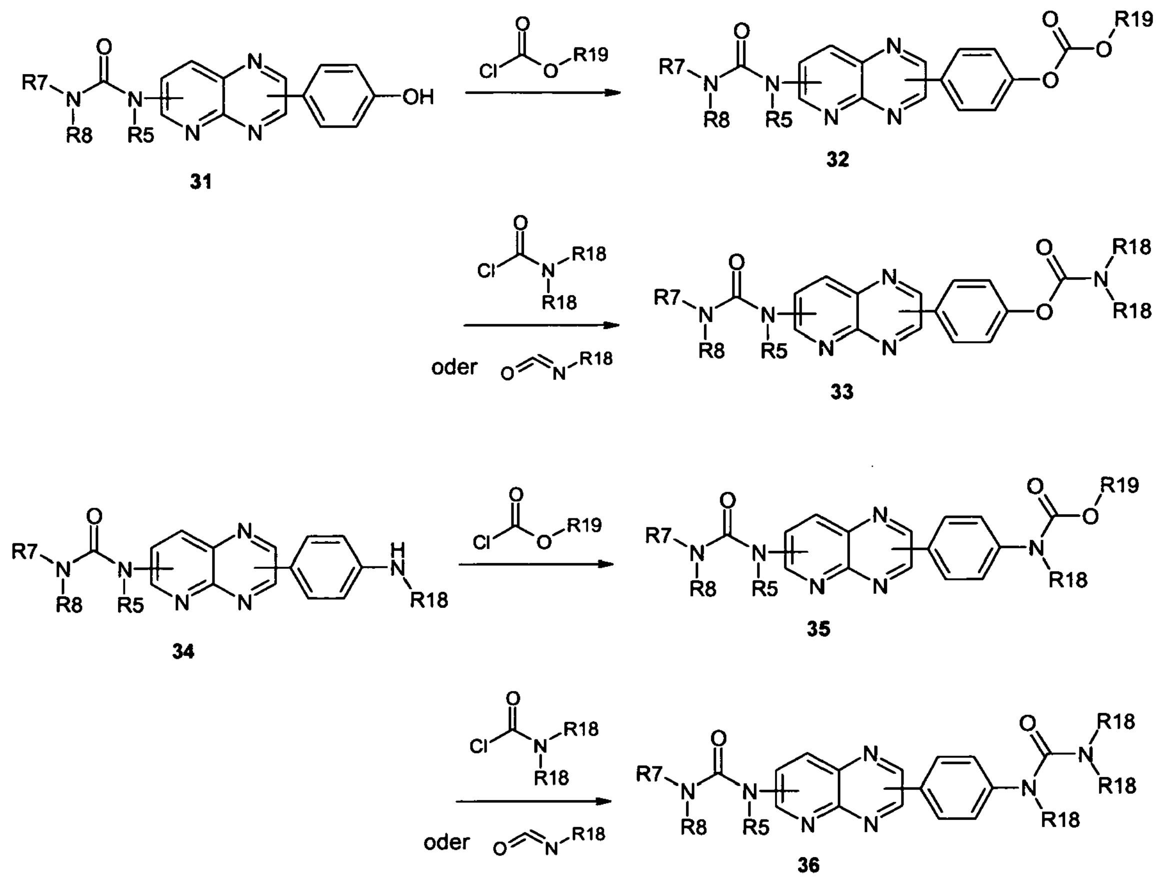


Die Ausgangsverbindungen sind entweder im Handel erhältlich oder können nach an sich oder dem Fachmann bekannten Verfahrensweisen hergestellt werden. Die Edukte 4, 7, 9-15, 22 und 26 stellen wertvolle Zwischenverbindungen für die Herstellung der erfindungsgemäßen Pyridopyrazine der allgemeinen Formel I dar.

Für die Herstellung der Ausgangs-, Zwischenverbindungen und der erfindungsgemäßen Pyridopyrazine gemäß der allgemeinen Formel I sei unter anderem auf die Patentschriften WO 2004/104002 und WO 2004/104003, sowie beispielsweise auf folgende Primärliteratur verwiesen, deren Inhalt hiermit Bestandteil der Offenbarung der vorliegenden Anmeldung werden soll:

1) Houben-Weyl, Methoden der Organischen Chemie, Band 4/1a, S. 343-350

2) Houben-Weyl, Methoden der Organischen Chemie, 4.Aufl., Band E 7b (Teil 2), S. 579; Degussa GB 1184848 (1970); S. Seko, et al. EP 735025 (1996)

3) D. Catarzi, et al.; J. Med. Chem. 1996, 1330-1336; J. K. Seydel, et al.; J. Med. Chem. 1994, 3016-3022

4) Houben-Weyl, Methods of Organic Chemistry, Volume E 9c, S.231-235

5) Houben-Weyl/Science of Synthesis, Volume 16, S. 1269

6) C. L. Leese, H. N. Rydon J. Chem. Soc. 1955, 303-309; T. S. Osdene, G. M. Timmis J. Chem. Soc. 1955, 2033-2035

7) W. He, et al. Bioorg. Med. Chem. Lett. 2003, 13, 3097-3100

8) M. S. A. El-Gaby, et al. Indian J. Chem. Sect. B 2001, 40, 195- 200; M. R. Myers, et al. Bioorg. Med. Chem. Lett.

2003, 13, 3091-3096 ; A. R. Renslo, et al. J. Amer. Chem. Soc. 1999, 121, 7459-7460; C. O. Okafor, et al. J. Heterocyclic Chem. 1983, 20, 199-203; C. R. Hopkins, et al. Tet. Lett. 2004, 45, 8631-8633

9) J. Yin, et al. Org. Lett. 2002, 4, 3481-3484; O. A. El-Sayed, et al. Arch. Pharm. 2002, 335, 403-410 ; C. Temple, et al. J. Med. Chem. 1992, 35, 988-993

10) A. M. Thompson, et al. J. Med. Chem. 2000, 43, 4200-4211; N. A. Dales, et al. Org. Lett. 2001, 2313-2316; G. Dannhardt, et al. Arch. Pharm. 2000, 267-274; G. S. Poindexter, et al. Bioorg. Med. Chem. 2004, 12, 507-521; J.-M. Receveur, et al. Bioorg. Med. Chem. Lett. 2004, 14, 5075-5080

11) G. Heinisch, et al. Arch. Pharm. 1997, 207-210; K. Matsuno, et al. J. Med. Chem. 2002, 45, 4513-4523; A. M. Papini, et al. J. Med. Chem. 2004, 47, 5224-5229

12) J. Mindl, et al. Collect. Czech. Chem. Commun. 1983, 48, 900-905; S. Sasaki, et al. J. Med. Chem. 2003, 46, 113-124; B.-B. Zeng, et al. Bioorg. Med. Chem. Lett. 2004, 14, 5565-5568

13) Q. Wang, et al. Synthetic Commun. 2004, 34, 255-264 ; W. Mederski, et al. Bioorg. Med. Chem. Lett. 2003, 13, 13715-3718 ; R. J. Brown, et al. Tetrahedron 2004, 60, 4361-4375

14) L. Mao, et al. Synthesis 2004, 15, 2535-2539; M. Darabantu, et al. Tetrahedron 2005, 61, 2897-2905; E. Ford, et al. Tet. Lett. 2000, 41, 3197-3198; T. Shiota, et al. J. Org. Chem. 1999, 64, 453-457; E. C. Taylor, et al. Synthetic Commun. 1987, 17, 1865-1868; G. A. Molander, et al. J. Org. Chem. 2002, 67, 8424-8429; G. Hughes, et al. Org. & Biomolecular Chem. 2004, 2, 3363-3367

15) R. P. Tangallapally, et al. J. Med. Chem. 2004, 47, 5276-5283; R. H. Bradburry, et al. J. Med. Chem. 1997, 40, 996-1004

16) X. He, et al. Bioorg. Med. Chem. 2004, 12, 4003-4008; A. Gopalsamy, et al. Bioorg. Med. Chem. Lett. 2005, 15, 1591-1594; J.-F. Cheng, et al. Bioorg. Med. Chem. Lett. 2004, 14, 2411-2416; E. R. Parmee, et al. Bioorg. Med. Chem. Lett. 2004, 14, 43-46

**Allgemeine Vorschriften zur Darstellung der Verbindungen der allgemeinen Formel I :**

**Schema 1: 1. Stufe**

2,6-Diamino-3-nitropyridin oder 2-Amino-3,5-dinitro-pyridin werden in einem geeigneten, inerten Lösungsmittel, wie beispielsweise Methanol, Ethanol, Dimethylformamid oder Dioxan, gelöst. Nach Zugabe eines Katalysators, beispielsweise Raney-Nickel, Palladium auf Kohle oder Platin(IV)dioxid, setzt man das Reaktionsgemisch unter eine Wasserstoff-Atmosphäre, wobei ein Druck zwischen 1 und 5 bar eingestellt wird. Man läßt das Reaktionsgemisch mehrere Stunden, beispielsweise 1-16 Stunden, in einem Temperaturbereich zwischen 20 °C und 60 °C reagieren. Nach beendeter Umsetzung filtriert man die unlöslichen Rückstände ab, wobei das Filtermedium beispielsweise aus Kieselgel, Celite oder handelsüblichen Glasfaserfiltern bestehen kann, und wäscht mit dem entsprechenden Lösungsmittel nach. Das Rohprodukt wird, in Lösung vorliegend, ohne weitere Aufreinigung für die nächste Umsetzung verwendet.

**2. Stufe**

Das 1,2-Dion-Derivat wird in einem geeigneten, inerten Lösungsmittel, beispielsweise Methanol, Ethanol, Dioxan, Toluol oder Dimethylformamid, vorgelegt. 2,3,6-Triaminopyridin oder 2,3,5-Triaminopyridin werden direkt nach der Reduktion als Lösung der Rohprodukte in einem der oben genannten Lösungsmittel zum vorgelegten 1,2-Dion gegeben, gegebenenfalls unter Zugabe einer Säure, wie z. B. Essigsäure oder einer Base, beispielsweise Kaliumhydroxid. Das Reaktionsgemisch läßt man in einem Temperaturbereich von 20 °C bis 80 °C einige Zeit, beispielsweise 20 Minuten bis 40 Stunden, reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Bei Verwendung von Dimethylformamid wird das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organischen Phasen im Vakuum eingeengt. Die Reinigung des zurückbleibenden Rohproduktes erfolgt durch Umkristallisieren aus einem

geeigneten Lösungsmittel, beispielsweise Dioxan, oder durch Säulen- bzw. Flash-Chromatographie an Kieselgel oder Aluminiumoxid. Als Laufmittel dient beispielsweise ein Gemisch aus Methanol und Dichlormethan.

**Schema 2: 1. Stufe**

Das Pyridopyrazin-on-Derivat 8 wird in einem geeigneten, inerten Lösungsmittel, beispielsweise Dimethylformamid, Dioxan oder Toluol, oder ohne Lösungsmittel vorgelegt. Ein Chlorierungsmittel, z. B. Phosphorylchlorid oder Thionylchlorid, wird bei Raumtemperatur zugegeben und das Reaktionsgemisch lässt man in einem Temperaturbereich von 20 ˚C bis 100 ˚C einige Zeit, beispielsweise 1 Stunde bis 24 Stunden, reagieren. Nach beendeter Umsetzung wird das Reaktionsgemisch auf Wasser gegossen und mit einer geeigneten wässrigen Base, beispielsweise Natronlauge, neutralisiert. Ein eventuell ausgefallener Niederschlag wird abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, oder die wässrige Phase wird mit einem geeigneten organischen Lösungsmittel, wie z. B. Dichlormethan oder Ethylacetat, extrahiert, und die organischen Phasen im Vakuum eingeengt. Die Reinigung des zurückbleibenden Rohproduktes erfolgt durch Umkristallisieren aus einem geeigneten Lösungsmittel, beispielsweise Dioxan oder Toluol, oder durch Säulen- bzw. Flash-Chromatographie an Kieselgel oder Aluminiumoxid. Als Laufmittel dient beispielsweise ein Gemisch aus Methanol und Dichlormethan.

**Schema 3: 1. Stufe**

2,3,6-Triaminopyridin oder 2,3,5-Triaminopyridin werden direkt nach der Reduktion als Lösung der Rohprodukte in einem der oben genannten Lösungsmittel vorgelegt. Nach Zugabe eines Oxalsäure-Derivates, wie z. B. Oxalsäurediethylester oder Oxalylchlorid, läßt man das Reaktionsgemisch, gegebenenfalls unter Zugabe einer Säure, wie z. B. Salzsäure, Schwefelsäure oder Eisessig, in einem Temperaturbereich von 20 ˚C bis 150 ˚C einige Zeit, beispielsweise 10 Minuten bis 24 Stunden, reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Base, wie z. B. Natronlauge, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Dioxan oder Toluol, oder durch Säulen- bzw. Flash-Chromatographie an Kieselgel oder Aluminiumoxid. Als Laufmittel dient beispielsweise ein Gemisch aus Methanol und Dichlormethan.

**2.Stufe**

Das Dion-Derivat 10 wird in einem geeigneten, inerten Lösungsmittel, beispielsweise Dimethylformamid, Dioxan oder Toluol, oder ohne Lösungsmittel vorgelegt. Ein Chlorierungsmittel, z. B. Phosphorylchlorid oder Thionylchlorid, wird bei Raumtemperatur zugegeben und das Reaktionsgemisch lässt man in einem Temperaturbereich von 20 ˚C bis 100 ˚C einige Zeit, beispielsweise 1 Stunde bis 24 Stunden, reagieren. Nach beendeter Umsetzung wird das Reaktionsgemisch auf Wasser gegossen und mit einer geeigneten wässrigen Base, beispielsweise Natronlauge, neutralisiert. Ein eventuell ausgefallener Niederschlag wird abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, oder die wässrige Phase wird mit einem geeigneten organischen Lösungsmittel, wie z. B. Dichlormethan oder Ethylacetat, extrahiert, und die organischen Phasen im Vakuum eingeengt. Die Reinigung des zurückbleibenden Rohproduktes erfolgt durch Umkristallisieren aus einem geeigneten Lösungsmittel, beispielsweise Dioxan oder Toluol, oder durch Säulen- bzw. Flash-Chromatographie an Kieselgel oder Aluminiumoxid. Als Laufmittel dient beispielsweise ein Gemisch aus Methanol und Dichlormethan.

**3. Stufe**

Die Zwischenstufe **11** kann mit einem entsprechenden Alkohol, Thiol oder Amin und gegebenenfalls mit einer geeigneten Base, vorzugsweise Natriumhydrid, Pyridin, Triethylamin, Kaliumcarbonat oder Natriummethanolat in Methanol, in einem geeigneten, inerten Lösungsmittel, wie beispielsweise Dimethylformamid, Dimethylsulfoxid, Methanol, Toluol, oder in einer Base als Lösungsmittel, wie z. B. Pyridin oder Triethylamin, oder ohne Lösungsmittel, umgesetzt werden. Das Reaktionsgemisch lässt man einige Zeit, beispielsweise 30 Minuten bis 2 Tage, in einem Temperaturbereich zwischen 20 ˚C und 140 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Dioxan, Ethylacetat oder Toluol, oder durch Säulen- bzw. Flash-Chromatographie an Kieselgel oder Aluminiumoxid. Als Laufmittel dient beispielsweise ein Gemisch aus Methanol und Dichlormethan.

**Schema 4: 1. Stufe**

Die Zwischenstufen **4** und **7** können mit einem entsprechenden, geeigneten Chlorid, Bromid oder Tosylat und gegebenenfalls mit einer geeigneten Base, vorzugsweise Natriumhydrid, Pyridin, Triethylamin, Kaliumcarbonat oder Natriummethanolat in Methanol, in einem geeigneten, inerten Lösungsmittel, wie beispielsweise Dimethylformamid, Dimethylsulfoxid, Methanol oder in einer Base als Lösungsmittel, wie z. B. Pyridin oder Triethylamin, oder ohne Lösungsmittel, umgesetzt werden. Das Reaktionsgemisch lässt man einige Zeit, beispielsweise 1 Stunde bis 24 Stunden, in einem Temperaturbereich zwischen 20 ˚C und 150 ˚C reagieren. Alternativ können die Zwischenstufen **4** und **7** mit einem entsprechenden Aryl-Bromid oder -Iodid und einem geeigneten Katalysator, wie z. B. Palladiumacetat oder $Pd_2(dba)_3$, und einem geeigneten Liganden, wie z. B. BINAP, und einer geeigneten Base, beispielsweise, Kaliumcarbonat oder Natrium-tert.butanolat, in einem geeignetem Lösungsmittel, wie beispielsweise Toluol oder Dioxan, umgesetzt werden. Das Reaktionsgemisch lässt man einige Zeit, beispielsweise 10 Stunden bis 30 Stunden, in einem Temperaturbereich zwischen 60 ˚C und 120 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. werden evtl. vorhandene Katalysatorreste abfiltriert mit dem entsprechenden Lösungsmittel nachgewaschen und das Lösungsmittel im Vakuum entfernt, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Toluol, oder durch Säulen- bzw. Flash-Chromatographie an Kieselgel oder Aluminiumoxid. Als Laufmittel dient beispielsweise ein Gemisch aus Methanol und Dichlormethan.

**Schema 5: 1. Stufe**

Im Anschluß an die Grundverfahren können in Folgereaktionen die nach dem Grundverfahren entstandenen Produkte in einer dem Fachmann bekannten Vorgehensweise zu erfindungsgemässen Folgeprodukten gemäß der Formel I umgesetzt werden.

So kann, wenn das Produkt ein Derivat der Verbindung **16** gemäß Schema 5 sein soll, das Reaktionsprodukt **4, 7, 9** oder **15** mit einem entsprechenden Isocyanat und gegebenenfalls einer geeigneten Base, vorzugsweise Natriumhydrid, Kaliumhexamethyldisilazid, Pyridin, Triethylamin oder Kaliumcarbonat, in einem geeigneten, inerten Lösungsmittel, wie beispielsweise Dimethylformamid, Dimethylsulfoxid, Acetonitril, Dichlormethan, 1,2-Dichlorethan oder Dioxan, oder in einer Base als Lösungsmittel, wie z. B. Pyridin oder Triethylamin, oder ohne Lösungsmittel, umgesetzt werden. Das Reaktionsgemisch läßt man mehrere Stunden, beispielsweise 1 - 24 Stunden, in einem Temperaturbereich zwischen 0 und 80 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

Oder es kann alternativ, wenn das Produkt ein Derivat der Verbindung **17** gemäß Schema 5 sein soll, das Reaktionsprodukt **4, 7, 9** oder **15** mit Phosgen oder Carbonyldiimidazol und einem entsprechenden Amin in einem geeigneten, inerten Lösungsmittel, wie beispielsweise Dimethylformamid, Tetrahydrofuran, Toluol, Dichlormethan oder

Acetonitril umgesetzt werden. Gegebenenfalls wird eine geeignete Base, vorzugsweise Pyridin, Natriumhydrogencarbonat, Triethylamin, N-Methyl-morpholin oder Natriumacetat verwendet. Das Reaktionsgemisch läßt man einige Zeit, beispielsweise 15 Minuten bis 24 Stunden, in einem Temperaturbereich zwischen 0 und 60 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **18** gemäß Schema 5 sein soll, das Reaktionsprodukt **4, 7, 9** oder **15** mit einem entsprechenden Isothiocyanat und gegebenenfalls einer geeigneten Base, vorzugsweise Natriumhydrid, Triethylamin, Kaliumcarbonat oder Pyridin, in einem geeigneten, inerten Lösungsmittel, wie beispielsweise Dimethylformamid, Tetrahydrofuran, Aceton oder Toluol, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 30 Minuten bis 90 Stunden, in einem Temperaturbereich zwischen 0 und 115 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

Oder es kann alternativ, wenn das Produkt ein Derivat der Verbindung **19** gemäß Schema 5 sein soll, das Reaktionsprodukt **4, 7, 9** oder **15** mit Thiophosgen oder Thiocarbonyldiimidazol und einem entsprechenden Amin in einem geeigneten, inerten Lösungsmittel, wie beispielsweise Dimethylformamid, Tetrahydrofuran, Toluol, Dichlormethan, Ethanol oder Acetonitril umgesetzt werden. Gegebenenfalls wird eine geeignete Base, vorzugsweise Pyridin, Natriumhydrogencarbonat, Kaliumcarbonat, Triethylamin oder Imidazol verwendet. Das Reaktionsgemisch läßt man mehrere Stunden, beispielsweise 1 bis 24 Stunden, in einem Temperaturbereich zwischen -10 und 80 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **20** gemäß Schema 5 sein soll, das Reaktionsprodukt **4, 7, 9** oder **15** mit Phosgen oder Carbonyldiimidazol und einem entsprechenden Hydroxyl-Amin in einem geeigneten, inerten Lösungsmittel, wie beispielsweise Dimethylformamid, Tetrahydrofuran, Dioxan, Dichlormethan oder Toluol umgesetzt werden. Gegebenenfalls wird eine geeignete Base, vorzugsweise Pyridin, Natriumcarbonat, Triethylamin oder Natriumacetat verwendet. Das Reaktionsgemisch läßt man einige Zeit, beispielsweise 1 Stunde bis 24 Stunden, in einem Temperaturbereich zwischen 0 und 100 ˚C reagieren. Alternativ kann das Reaktionsprodukt **4**, **7, 9** oder **15** mit einem entsprechenden Hydroxylamin-Phenyl-Carbamat-Reagenz und gegebenenfalls mit einer geeigneten Base, vorzugsweise Pyridin, Natriumcarbonat, Triethylamin oder Natriumacetat, in einem geeigneten, inerten Lösungsmittel, wie beispielsweise Tetrahydrofuran, Dioxan, Dichlormethan, Dimethylformamid oder Toluol, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 1 Stunde bis 18 Stunden, in einem Temperaturbereich zwischen Raumtemperatur und 100 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der

ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **21** gemäß Schema 5 sein soll, das Reaktionsprodukt **4, 7, 9** oder **15** mit Phosgen oder Carbonyldiimidazol und einem entsprechenden Hydrazin in einem geeigneten, inerten Lösungsmittel, wie beispielsweise Dioxan, Chloroform, Toluol oder Ethanol umgesetzt werden. Gegebenenfalls wird eine geeignete Base, vorzugsweise Pyridin, Natriumcarbonat, Diisopropylethylamin oder Natriumacetat verwendet. Das Reaktionsgemisch läßt man einige Zeit, beispielsweise 1 Stunde bis 24 Stunden, in einem Temperaturbereich zwischen 0 und 100 ˚C reagieren. Alternativ kann das Reaktionsprodukt **4, 7, 9** oder **15** mit einem entsprechenden Hydrazin-Phenyl-Carbamat-Reagenz und gegebenenfalls mit einer geeigneten Base, vorzugsweise Pyridin, Natriumcarbonat, Triethylamin oder Natriumacetat, in einem geeigneten, inerten Lösungsmittel, wie beispielsweise Tetrahydrofuran, Dioxan, Dichlormethan, Dimethylformamid oder Toluol, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 1 Stunde bis 15 Stunden, in einem Temperaturbereich zwischen 0 ˚C und 100 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

**Schema 6: 1. Stufe**

Im Anschluß an die Grundverfahren können in Folgereaktionen die nach dem Grundverfahren entstandenen Produkte in einer dem Fachmann bekannten Vorgehensweise zu erfindungsgemässen Folgeprodukten gemäß der Formel I umgesetzt werden.

So kann, wenn das Produkt ein Derivat der Verbindung **23** gemäß Schema 6 sein soll, das Reaktionsprodukt **22** mit entsprechenden Aryl/Heteroaryl-Boronsäure-Derivaten oder Aryl/Heteroaryl-Organozinn-Verbindungen und einem geeigneten Katalysator, wie z. B. $Pd(PPh_3)_4$, [1,1'-Bis(diphenylphosphino)ferrocen]-dichlor-palladium(II) oder $Pd_2(dba)_3$, und einer geeigneten Base, beispielsweise, Natriumcarbonat, Cäsiumcarbonat oder Triethylamin, in einem geeignetem Lösungsmittel, wie beispielsweise Dimethylformamid, Dimethylformamid/Wasser, Toluol, Acetonitril, Dimethoxyethan oder Dioxan, umgesetzt werden. Das Reaktionsgemisch lässt man einige Zeit, beispielsweise 6 Stunden bis mehrere Tage, in einem Temperaturbereich zwischen 60 ˚C und 120 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. werden evtl. vorhandene Katalysatorreste abfiltriert mit dem entsprechenden Lösungsmittel nachgewaschen und das Lösungsmittel im Vakuum entfernt, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **23a** gemäß Schema 6 sein soll, das Reaktionsprodukt **22** mit entsprechenden Alkyl-Zink-Halogeniden und einem geeigneten Katalysator, wie z. B. $Pd(PPh_3)_4$, [1,1'-Bis(diphenylphosphino)ferrocen]-dichlor-palladium(II) oder $PdCl_2(PPh_3)_2$ in einem geeignetem Lösungsmittel, wie beispielsweise Dimethylformamid, Tetrahydrofuran, Toluol, Dimethoxyethan oder Dioxan, umgesetzt werden. Das Reaktionsgemisch lässt man einige Zeit, beispielsweise 30 Minuten bis 48 Stunden, in einem Temperaturbereich zwischen Raumtemperatur und 120 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. werden evtl. vorhandene Katalysatorreste abfiltriert mit dem entsprechenden Lösungsmittel nachgewaschen und das Lösungsmittel im Vakuum entfernt, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **24** gemäß Schema 6 sein soll, das Reaktionsprodukt **22** mit entsprechenden Vinyl-Boronsäure-Derivaten, Vinyl-Organozinn-Verbindungen oder Alken-Derivaten und einem geeigneten Katalysator, wie z. B. $Pd(PPh_3)_4$, [1,1'-Bis(diphenylphosphino)ferrocen]-dichlor-palladium(II) oder $Pd(OAc)_2$, und einem geeigneten Liganden, wie z. B. Triphenylphosphin oder Tri-o-tolylphosphin, und einer geeigneten Base, beispielsweise Kaliumcarbonat, Natriumcarbonat, Triethylamin oder Natriumacetat, in einem geeignetem Lösungsmittel, wie beispielsweise Toluol, Dimethylformamid, Dimethylformamid/Wasser, Acetonitril, Dimethoxyethan oder Dimethylacetamid, umgesetzt werden. Das Reaktionsgemisch lässt man einige Zeit, beispielsweise 3 Stunden bis 24 Stunden, in einem Temperaturbereich zwischen 60 °C und 140 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. werden evtl. vorhandene Katalysatorreste abfiltriert mit dem entsprechenden Lösungsmittel nachgewaschen und das Lösungsmittel im Vakuum entfernt, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Toluol, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **25** gemäß Schema 6 sein soll, das Reaktionsprodukt **22** mit entsprechenden Alkin-Derivaten und einem geeigneten Katalysator, wie z. B. $Pd(PPh_3)_4$, $PdCl_2(PPh_3)_2$ oder $Pd_2(dba)_3$, und einem geeigneten Zusatzstoff, wie beispielsweise Kupfer(i)iodid, und einer geeigneten Base, beispielsweise Kaliumcarbonat, Triethylamin oder Kaliumacetat, in einem geeignetem Lösungsmittel, wie beispielsweise Dimethylformamid, Tetrahydrofuran, Tetrahydrofuran/Wasser, Toluol oder Dimethylacetamid, umgesetzt werden. Das Reaktionsgemisch lässt man einige Zeit, beispielsweise 1 Stunde bis mehrere Tage, in einem Temperaturbereich zwischen Raumtemperatur und 120 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. werden evtl. vorhandene Katalysatorreste abfiltriert mit dem entsprechenden Lösungsmittel nachgewaschen und das Lösungsmittel im Vakuum entfernt, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Toluol, oder durch Säulen- bzw. Flash-Chromatographie an Kieselgel oder Aluminiumoxid. Als Laufmittel dient beispielsweise ein Gemisch aus Methanol und Dichlormethan.

**Schema 7: 1. Stufe**

Im Anschluß an die Grundverfahren können in Folgereaktionen die nach dem Grundverfahren entstandenen Produkte in einer dem Fachmann bekannten Vorgehensweise zu erfindungsgemässen Folgeprodukten gemäß der Formel I umgesetzt werden.

Die Zwischenstufe **22** kann mit einem entsprechenden Amin und gegebenenfalls mit einer geeigneten Base, vorzugsweise Natriumhydrid, Pyridin, Triethylamin, Kaliumcarbonat oder Natriummethanolat in Methanol, in einem geeigneten, inerten Lösungsmittel, wie beispielsweise Dimethylformamid, Dimethylsulfoxid, Methanol, Toluol, oder in einer Base als Lösungsmittel, wie z. B. Pyridin oder Triethylamin, oder ohne Lösungsmittel, umgesetzt werden. Das Reaktionsgemisch lässt man einige Zeit, beispielsweise 1 Stunde bis mehrere Tage, in einem Temperaturbereich zwischen 20 °C und 140 °C reagieren. Alternativ kann die Zwischenstufe **22** mit einem entsprechenden Amin und einem geeigneten Katalysator, wie z. B. Palladiumacetat oder $Pd_2(dba)_3$, und einem geeigneten Liganden, wie z. B. BINAP, und einer geeigneten Base, beispielsweise, Kaliumcarbonat oder Natrium-tert.butanolat, in einem geeignetem Lösungsmittel, wie

beispielsweise Toluol oder Dioxan, umgesetzt werden. Das Reaktionsgemisch lässt man einige Zeit, beispielsweise 10 Stunden bis 30 Stunden, in einem Temperaturbereich zwischen 60 °C und 120 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. werden evtl. vorhandene Katalysatorreste abfiltriert mit dem entsprechenden Lösungsmittel nachgewaschen und das Lösungsmittel im Vakuum entfernt, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Dioxan, Ethylacetat oder Toluol, oder durch Säulen- bzw. Flash-Chromatographie an Kieselgel oder Aluminiumoxid. Als Laufmittel dient beispielsweise ein Gemisch aus Methanol und Dichlormethan.

**2. Stufe**

So kann, wenn das Produkt ein Derivat der Verbindung **27** gemäß Schema 7 sein soll, das Reaktionsprodukt **26** mit einem entsprechenden Carbonsäurechlorid und gegebenenfalls einer geeigneten Base, vorzugsweise Natriumhydrid, Kaliumhydroxid, Pyridin, Triethylamin oder Kaliumcarbonat, und gegebenenfalls einem Katalysator, wie z. B. Dimethylaminopyridin, in einem geeigneten, inerten Lösungsmittel, wie beispielsweise Tetrahydrofuran, Toluol, Acetonitril, Dichlormethan, Aceton oder Dioxan, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 30 Minuten bis 24 Stunden, in einem Temperaturbereich zwischen 0 und 110 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **28** gemäß Schema 7 sein soll, das Reaktionsprodukt **26** mit einem entsprechenden Sulfonsäurechlorid und gegebenenfalls einer geeigneten Base, vorzugsweise Natriumhydrid, Kaliumhydroxid, Pyridin, Triethylamin oder Kaliumcarbonat, und gegebenenfalls einem Katalysator, wie z. B. Dimethylaminopyridin, in einem geeigneten, inerten Lösungsmittel, wie beispielsweise Tetrahydrofuran, Toluol, Acetonitril, Dichlormethan, Aceton, Dimethylformamid oder Dioxan, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 30 Minuten bis 16 Stunden, in einem Temperaturbereich zwischen 0 und 120 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **29** gemäß Schema 7 sein soll, das Reaktionsprodukt **26** mit einem entsprechenden Chlorkohlensäureester und gegebenenfalls einer geeigneten Base, vorzugsweise Natriumhydrid, Natriumhydroxid, Pyridin, Triethylamin oder Kaliumcarbonat, und gegebenenfalls einem Katalysator, wie z. B. Dimethylaminopyridin, in einem geeigneten, inerten Lösungsmittel, wie beispielsweise Dioxan, Tetrahydrofuran, Dichlormethan, Aceton, Dimethylformamid oder Dichlorethan, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 1 Stunde bis 24 Stunden, in einem Temperaturbereich zwischen -10 °C und 100 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das

Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **30** gemäß Schema 7 sein soll, das Reaktionsprodukt 26 mit einem entsprechenden Isocyanat oder Carbaminsäurechlorid und gegebenenfalls einer geeigneten Base, vorzugsweise Natriumhydrid, Pyridin, Triethylamin, Piperidin oder Kaliumcarbonat, und gegebenenfalls einem Katalysator, wie z. B. Dimethylaminopyridin, in einem geeigneten, inerten Lösungsmittel, wie beispielsweise Dioxan, Tetrahydrofuran, Dimethylformamid, Toluol oder Acetonitril, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 2 Stunde bis 40 Stunden, in einem Temperaturbereich zwischen Raumtemperatur und 100 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

**Schema 8: 1. Stufe**

Im Anschluß an die Grundverfahren können in Folgereaktionen die nach dem Grundverfahren entstandenen Produkte in einer dem Fachmann bekannten Vorgehensweise zu erfindungsgemässen Folgeprodukten gemäß der Formel I umgesetzt werden.

So kann, wenn das Produkt ein Derivat der Verbindung **32** oder **35** gemäß Schema 8 sein soll, das Reaktionsprodukt **31** oder **34** mit einem entsprechenden Chlorkohlensäureester und gegebenenfalls einer geeigneten Base, vorzugsweise Natriumhydrid, Natriumhydroxid, Pyridin, Triethylamin oder Kaliumcarbonat, und gegebenenfalls einem Katalysator, wie z. B. Dimethylaminopyridin, in einem geeigneten, inerten Lösungsmittel, wie beispielsweise Dioxan, Tetrahydrofuran, Dichlormethan, Aceton, Dimethylformamid oder Dichlorethan, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 1 Stunde bis 24 Stunden, in einem Temperaturbereich zwischen -10 ˚C und 100 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung **33** oder **36** gemäß Schema 8 sein soll, das Reaktionsprodukt **31** oder **34** mit einem entsprechenden Isocyanat oder Carbaminsäurechlorid und gegebenenfalls einer geeigneten Base, vorzugsweise Natriumhydrid, Pyridin, Triethylamin, Piperidin oder Kaliumcarbonat, und gegebenenfalls einem Katalysator, wie z. B. Dimethylaminopyridin, in einem geeigneten, inerten Lösungsmittel, wie beispielsweise Dioxan, Tetrahydrofuran, Dimethylformamid, Toluol oder Acetonitril, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 2 Stunde bis 40 Stunden, in einem Temperaturbereich zwischen Raumtemperatur und 100 ˚C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische

Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

Unter einigen der genannten Reaktionsbedingungen können OH-, SH- und $NH_2$-Gruppen möglicherweise unerwünschte Nebenreaktionen eingehen. Es ist daher bevorzugt, diese mit Schutzgruppen zu versehen oder im Falle von $NH_2$ durch $NO_2$ zu ersetzen und nachfolgend die Schutzgruppe abzuspalten oder die $NO_2$-Gruppe zu reduzieren. So kann in Abwandlung der oben beschriebenen Verfahren in den Ausgangsverbindungen mindestens eine OH-Gruppe beispielsweise durch eine Benzyloxygruppe und/oder mindestens eine SH-Gruppe beispielsweise durch eine S-Benzylgruppe und/oder mindestens eine $NH_2$-Gruppe durch eine NH-Benzylgruppe oder durch eine $NO_2$-Gruppe ersetzt werden. Nachfolgend kann mindestens eine - vorzugsweise alle - Benzyloxygruppe/n oder NH-Benzylgruppe/n beispielsweise mit Wasserstoff und Palladium auf Kohle und/oder mindestens eine - vorzugsweise alle - S-Benzylgruppe/n beispielsweise mit Natrium in Ammoniak abgespalten und/oder mindestens eine - vorzugsweise alle - $NO_2$-Gruppe/n beispielsweise mit Wasserstoff und Raney-Nickel zu $NH_2$ reduziert werden.

Unter einigen der genannten Reaktionsbedingungen können OH-, $NH_2$- und COOH-Gruppen möglicherweise unerwünschte Nebenreaktionen eingehen. Es ist daher bevorzugt, Ausgangsverbindungen und Zwischenstufen, welche mindestens eine OH- und/oder mindestens eine $NH_2$- und/oder mindestens eine COOH-Gruppe enthalten, in entsprechende Carbonsäureester- und Carbonsäureamid-Derivate zu überführen. In Abwandlung der oben beschriebenen Verfahren können Ausgangsverbindungen und Zwischenstufen, welche mindestens eine OH-Gruppe besitzen, und/oder welche mindestens eine $NH_2$-Gruppe besitzen, durch Umsetzung mit einer aktivierten Carbonsäuregruppe, beispielsweise einer Carbonsäurechloridgruppe, in Carbonsäureester- bzw. Carbonsäureamid-Derivate überführt werden. In Abwandlung der oben beschriebenen Verfahren können Ausgangsverbindungen und Zwischenstufen, welche mindestens eine COOH-Gruppe besitzen, durch Umsetzung mit einem Aktivierungsmittel, wie beispielsweise Thionylchlorid oder Carbonyldiimidazol, und nachfolgender Umsetzung mit einem geeigneten Alkohol oder Amin in Carbonsäureester- bzw. Carbonsäureamid-Derivate überführt werden. Nachfolgend kann mindestens eine - vorzugsweise alle - Carbonsäureester- bzw. Carbonsäureamidgruppe/n in den Ausgangsverbindungen und Zwischenstufen beispielsweise mit verdünnten wässrigen Säuren bzw. Basen gespalten werden, um mindestens eine - vorzugsweise alle - OH-Gruppe/n und/oder $NH_2$-Gruppe/n und/oder COOH-Gruppe/n in Freiheit zu setzen.

Die Benennung der erfindungsgemäßen Verbindungen der allgemeinen Formel I samt bevorzugter Ausführungsbeispiele, und insbesondere der Verbindungen **1** bis **15**, erfolgte mit der AutoNom 2000 - Software (ISIS ™/ Draw 2.5; MDL).

Die Erfindung soll anhand der nachfolgenden Beispiele näher erläutert werden, ohne jedoch auf diese Beispiele beschränkt zu sein.

## Beispiele

Gemäß den allgemeinen Synthesevorschriften, denen die Syntheseschemata 1 - 8 zugrundeliegen, wurden folgende Verbindungen synthetisiert, die unter der Angabe der jeweiligen chemischen Bezeichnung aus der nachfolgenden Übersicht hervorgehen. Ferner sind ihre NMR-spektroskopischen Daten und massenspektrometrischen Daten bzw. Schmelzpunkte beigefügt. In der sich anschließenden Tabelle 1 sind aus der allgemeinen Formel II und den Substituenten R1, R2, R3, R4, sowie R5 und Y die Strukturen dieser Verbindungen zu ersehen.

Die eingesetzten Chemikalien und Lösungsmittel wurden kommerziell bei den herkömmlichen Anbietern erworben (Acros, Aldrich, Fluka, Lancaster, Maybridge, Merck, Sigma, TCI, etc.) oder synthetisiert.

Die Erfindung soll anhand der nachfolgenden Beispiele näher erläutert werden, ohne darauf beschränkt zu sein.

### Beispiel 1: 1-Ethyl-3-(3-phenylethynyl-pyrido[2,3-b]pyrazin-6-yl)-harnstoff

Herstellung von 1-(3-Chlor-pyrido[2,3-b]pyrazin-6-yl)-3-ethyl-harnstoff (Umsetzung gemäß Schema 5)

100 mg 3-Chlor-pyrido[2,3-b]pyrazin-6-yl-amin (0.55 mmol) werden in 5 ml Pyridin vorgelegt und 44 µL Ethylisocyanat (0.55 mmol) bei Raumtemperatur zugegeben. Man lässt das Gemisch 3h bei 75 °C rühren und fügt dann nochmal insgesamt 132 µL Ethylisocyanat (1.65 mmol) über 18 h in kleinen Portionen zum Reaktionsgemisch hinzu. Dann wird das Lösungsmittel im Vakuum entfernt. Der resultierende Feststoff wird über Säulenchromatographie an Kieselgel (Laufmittel Dichlormethan/Methanol) gereinigt. Man erhält einen hellgelben Feststoff.

Herstellung von 1-Ethyl-3-(3-phenylethynyl-pyrido[2,3-b]pyrazin-6-yl)-harnstoff (Umsetzung gemäß Schema 6)

98.1 mg 1-(3-Chlor-pyrido[2,3-b]pyrazin-6-yl)-3-ethyl-harnstoff (0.39 mmol), 10.1 mg Kupfer(I)iodid (0.05 mmol)

und 193 μl Triethylamin (1.38 mmol) werden in 2 ml wasserfreiem Dimethylformamid unter Stickstoff als Schutzgas vorgelegt. Anschließend werden nacheinander 29.1 mg Dichlor-bis(triphenylphosphin)-palladium(II) (0.04 mmol) und 54 μL Phenylacetylen (0.49 mmol) zugegeben und das Gemisch wird 16h bei Raumtemperatur gerührt. Zur Aufarbeitung wird das Gemisch mit Dichlormethan verdünnt und in verdünnte Salzsäure gegeben. Der ausgefallene Feststoff wird abgesaugt und die organische Phase mit verdünnter Salzsäure und destilliertem Wasser gewaschen. Nach Phasentrennung wird das organische Lösungsmittel im Vakuum entfernt. Die weitere Aufreinigung erfolgt durch Säulenchromatographie an Kieselgel (Laufmittelgemisch Dichlormethan/Methanol). Man erhält einen gelben Feststoff.

Schmelzpunkt: 236-238 ˚C (Zers.)

ESI-MS: gef. m/z = 318.0 (M + $H^+$); ber. 317 amu

$^1$H-NMR ($d_6$-DMSO): δ = 1.20 (t, 3H), 3.35 - 3.45 (m, 2H), 7.51 - 7.59 (m, 3H), 7.68 (d, 1H), 7.75 (d, 2H), 8.37 (d, 1 H), 9.01 (s, 1 H), 9.14 (s, 1 H), 10.23 (s, 1 H) ppm.

Folgende Beispiele wurden gemäß Beispiel 1 und der allgemeinen Synthesevorschriften synthetisiert :

Beispiel 2: 1-Ethyl-3-(3-thiophen-3-ylethynyl-pyrido[2,3-b]pyrazin-6-yl)-harnstoff

Smp.: 239-242 ˚C (Zers.)

ESI-MS: gef. m/z = 324.2 (M + $H^+$); ber. 323 amu

$^1$H-NMR ($d_6$-DMSO): δ = 1.20 (t, 3H), 3.25 - 3.35 (m, 2H), 7.43 (d, 1 H), 7.66 (d, 1H), 7.76 (dd, 1H), 8.21 (d, 1H), 8.36 (d, 1H), 8.97 (s, 1H), 9.14 (s, 1H), 10.23 (s, 1H) ppm.

Beispiel 3: 1-(3-Cyclopropylethynyl-pyrido[2,3-b]pyrazin-6-yl)-3-ethyl-harnstoff

Smp.: 227-228 ˚C (Zers.)

ESI-MS: gef. m/z = 282.3 (M + $H^+$); ber. 281 amu

$^1$H-NMR ($d_6$-DMSO): δ = 1.00 - 1.10 (m, 2H), 1.19 (t, 3H), 1.69 - 1.79 (m, 1 H), 3.25 - 3.35 (m, 2H), 7.63 (d, 1 H), 8.32 (d, 1 H), 8.77 (s, 1 H), 9.11 (s, 1 H), 10.18 (s, 1 H) ppm.

Beispiel 4: 1-[3-(3-Dimethylamino-prop-1-ynyl)-pyrido[2,3-b]pyrazin-6-yl]-3-ethyl-harnstoff

ESI-MS: gef. m/z = 299.2 (M + $H^+$); ber. 298 amu

$^1$H-NMR ($d_6$-DMSO): δ = 1.19 (t, 3H), 2.33 (s, 6H), 3.27 - 3.35 (m, 2H), 3.67 (s, 2H), 7.67 (d, 1 H), 8.34 (d, 1 H), 8.85 (s, 1 H), 9.05 (s, 1 H), 10.19 (s, 1 H) ppm.

Beispiel 5: 1-[3-((E)-2-Cyclohexyl-vinyl)-pyrido[2,3-b]pyrazin-6-yl]-3-ethyl-harnstoff (Umsetzung gemäß Schema 6)

99.6 mg 1-(3-Chlor-pyrido[2,3-b]pyrazin-6-yl)-3-ethyl-harnstoff (0.40 mmol), 73.6 mg Cyclohexylvinylboronsäure (0.48 mmol), 84.4 mg Natriumcarbonat (0.80 mmol) und 33.4 mg [1,1'-Bis(diphenylphosphino)ferrocen]dichlorpalladium(II) (0.04 mmol) werden in 6 ml Dimethylformamid/Wasser (1:1) unter Stickstoff vorgelegt. Dann wird die Mischung 6.5h auf 90 ˚C erhitzt. Zum Reaktionsgemisch wird dann destilliertes Wasser hinzugefügt und der entstandene Niederschlag abgesaugt. Die weitere Aufreinigung erfolgt durch Säulenchromatographie an Kieselgel (Laufmitteigemisch Ethylacetat/Heptan). Man erhält einen hellbraunen Feststoff.

Smp.: 202-204 ˚C (Zers.)

ESI-MS: gef. m/z = 326.0 (M + $H^+$); ber. 325 amu

$^1$H-NMR ($d_6$-DMSO): δ = 1.10 - 1.40 (m, 8H), 1.65 - 1.90 (m, 5H), 2.28 - 2.38 (m, 1 H), 3.25 - 3.35 (m, 2H), 6.69 (d, 1 H), 7.15 (dd, 1 H), 7.58 (d, 1 H), 8.28 (d, 1 H), 8.98 (s, 1 H), 9.15 (s, 1 H), 10.05 (s, 1 H) ppm.

Folgendes Beispiel wurde gemäß Beispiel 5 und der allgemeinen Synthesevorschriften synthetisiert:

Beispiel 6: 1-Ethyl-3-[3-((E)-3-methoxy-propenyl)-pyrido[2,3-b]pyrazin-6-yl]-harnstoff

ESI-MS: gef. m/z = 288.3 (M + $H^+$); ber. 287 amu

$^1$H-NMR ($d_6$-DMSO): δ = 1.20 (t, 3H), 3.25 - 3.35 (m, 2H), 3.39 (s, 3H), 4.25 (d, 2H), 6.92 (d, 1 H), 7.15 - 7.25 (m, 1 H), 7.62 (d, 1 H), 8.30 (d, 1 H), 9.02 (s, 1 H), 9.15 (s, 1H), 10.10 (s, 1 H) ppm.

Beispiel 7: 1-Methoxy-3-(3-phenyl-pyrido[2,3-b]pyrazin-6-yl)-harnstoff

Herstellung von 3-Phenyl-pyrido[2,3-b]pyrazin-6-yl-amin (Umsetzung gemäß Schema 6)

1.00 g 3-Chlor-pyrido[2,3-b]pyrazin-6-yl-amin (5.54 mmol), 743 mg Phenylboronsäure (6.09 mmol), 640 mg Tetrakis(triphenylphosphin)-palladium(0) (0.55 mmol) und 1.76 g Natriumcarbonat (16.6 mmol) werden in 100 ml Dimethylformamid/Wasser (1:1) unter Stickstoff als Schutzgas vorgelegt und 2h bei 80 °C gerührt. Nach beendeter Umsetzung wird das Gemisch abgesaugt und das Filtrat auf 800 ml destilliertes Wasser gegossen. Die wässrige Phase wird mehrmals mit Ethylacetat extrahiert. Die organische Phase wird im Vakuum von Lösungsmitteln befreit. Der resultierende Feststoff wird über Säulenchromatographie an Kieselgel aufgereinigt (Laufmittel Dichlormethan/Methanol). Man erhält einen gelben Feststoff.

Herstellung von 1-Methoxy-3-(3-phenyl-pyrido[2,3-b]pyrazin-6-yl)-harnstoff (Umsetzung gemäß Schema 5)

100 mg 3-Phenyl-pyrido[2,3-b]pyrazin-6-yl-amin (0.45 mmol) werden in Pyridin gelöst und mit 191 mg p-Nitrophenyl-N-methoxy-carbamat (0.90 mmol) versetzt. Das Gemisch wird 4h unter Rückfluß erhitzt. Zur Aufarbeitung wird das Pyridin im Vakuum entfernt und der Rückstand in Ethylacetat und destilliertem Wasser verteilt. Die Phasen werden getrennt und die wässrige Phase zweimal mit Ethylacetat extrahiert. Die vereinigten organischen Phasen werden über $MgSO_4$ getrocknet und das Lösungsmittel im Vakuum entfernt. Der Rückstand wird säulenchromatographisch an Kieselgel aufgereinigt (Laufmittel Dichlormethan/Methanol). Es wird ein hellbeiger Feststoff isoliert.

ESI-MS: gef. m/z = 296.2 (M + $H^+$); ber. 295 amu

$^1$H-NMR ($d_6$-DMSO): $\delta$ = 3.75 (s, 3H), 7.58 - 7.65 (m, 3H), 8.03 (s, 1H), 8.36 (d, 2H), 8.45 (d, 1 H), 9.52 (s, 1 H), 10.16 (s, 1 H), 11.01 (s, 1 H) ppm.

Folgendes Beispiel wurde gemäß Beispiel 7 und der allgemeinen Synthesevorschriften synthetisiert:

Beispiel 8: 1-Allyloxy-3-(3-phenyl-pyrido[2,3-b]pyrazin-6-yl)-harnstoff

ESI-MS: gef. m/z = 322.2 (M + $H^+$); ber. 321 amu

$^1$H-NMR ($d_6$-DMSO): $\delta$ = 4.44 (d, 2H), 5.33 (d, 1 H), 5.41 (d, 1H), 6.02 - 6.10 (m, 1 H), 7.58 - 7.65 (m, 3H), 8.00 (s, 1 H), 8.36 (d, 2H), 8.45 (d, 1 H), 9.52 (s, 1 H), 10.13 (s, 1 H), 11.07 (s, 1 H) ppm.

Beispiel 9: Kohlensäure-4-[6-(3-ethyl-harnstoff)-pyrido[2,3-b]pyrazin-3-yl]-phenylmethyl-ester

Herstellung von 1-Ethyl-3-[3-(4-hydroxy-phenyl)-pyrido[2,3-b]pyrazin-6-yl]-harnstoff (Umsetzung gemäß Schema 5)

0.50 g 4-(6-Amino-pyrido[2,3-b]pyrazin-3-yl)-phenol (2.10 mmol) werden in 5.0 ml Pyridin vorgelegt. 183 µL Ethylisocyanat (2.31 mmol) werden bei Raumtemperatur zugetropft und das Gemisch wird für 2h auf 70 - 80 °C erhitzt. Danach werden nochmals 183 µL Ethylisocyanat zugegeben und das Gemisch weitere 2h auf 70 -80 °C erhitzt. Nach beendeter Umsetzung wird das Reaktionsgemisch auf Eiswasser gegeben und mit 1 N Salzsäure neutralisiert. Das ausgefallene Produkt wird abgesaugt und getrocknet. Das abgesaugt Produkt wird in Ethanol angelöst und mit 52 mg Kaliumhydroxid (0.93 mmol), in Wasser gelöst, versetzt. Das Gemisch erwärmt man ca.1h auf 40 °C. Danach wird die Reaktionslösung mit 1N Salzsäure neutral gestellt und das Lösungsmittel im Vakuum entfernt. Der resultierende Feststoff wird säulenchromatographisch an Kieselgel gereinigt (Laufmittel Dichlormethan/Methanol). Man erhält einen gelben Feststoff.

Herstellung von Kohlensäure-4-[6-(3-ethyl-harnstoff)-pyrido[2,3-b]pyrazin-3-yl]-phenyl-methyl-ester (Umsetzung gemäß Schema 8)

100 mg 1-Ethyl-3-[3-(4-hydroxy-phenyl)-pyrido[2,3-b]pyrazin-6-yl]-harnstoff (0.32 mmol), 30 µL Chlorameisensäuremethylester (0.39 mmol) und 45 µL Triethylamin (0.32 mmol) werden in 10 ml wasserfreiem Dioxan vorgelegt und 1h auf 100 °C erhitzt. Nach dem Abkühlen wird der ausgefallene Feststoff abgesaugt und getrocknet. Nach Umkristallisieren aus Ethanol erhält man einen schwach gelben Feststoff.

Smp.: 243-245 °C

ESI-MS: gef. m/z = 368.2 (M + $H^+$); ber. 367 amu

$^1$H-NMR ($d_6$-DMSO): $\delta$ = 1.20 (t, 3H), 3.30 - 3.39 (m, 2H), 3.90 (s, 3H), 7.49 (d, 2H), 7.70 (d, 1 H), 8.38 (d, 1 H), 8.42 (d, 2H), 9.03 (s, 1 H), 9.47 (s, 1 H), 10.09 (s, 1 H) ppm.

Folgende Beispiele wurden gemäß Beispiel 9 und der allgemeinen Synthesevorschriften synthetisiert :

Beispiel 10: Kohlensäure-4-[6-(3-ethyl-harnstoff)-pyrido[2,3-b]pyrazin-3-yl]-phenyl-(2-methoxy-ethyl)-ester

Smp.: 230-232 °C

ESI-MS: gef. m/z = 412.2 (M + $H^+$); ber. 411 amu

$^1$H-NMR ($d_6$-DMSO): δ = 1.20 (t, 3H), 3.30 - 3.39 (m, 5H), 3.68 (t, 2H), 4.40 (t, 2H), 7.50 (d, 2H), 7.70 (d, 1 H), 8.38 (d, 1H), 8.41 (d, 2H), 9.10 (s, 1 H), 9.50 (s, 1 H), 10.15 (s, 1 H) ppm.

Beispiel 11: Kohlensäure-4-[6-(3-ethyl-harnstoff)-pyrido[2,3-b]pyrazin-3-yl]-phenyl-phenyl-ester

ESI-MS: gef. m/z = 430.2 (M + $H^+$); ber. 429 amu

$^1$H-NMR ($d_6$-DMSO): δ = 1.21 (t, 3H), 3.30 - 3.33 (m, 2H), 7.33 - 7.38 (m, 1 H), 7.43 (d, 2H), 7.51 (t, 2H), 7.65 (d, 2H), 8.39 (d, 1 H), 8.45 (d, 2H), 9.11 (s, 1 H), 9.50 (s, 1 H), 10.19 (s, 1 H) ppm.

Beispiel 12: Diethyl-carbaminsäure-4-[6-(3-ethyl-harnstoff)-pyrido[2,3-b]pyrazin-3-yl]-phenyl-ester

ESI-MS: gef. m/z = 409.4 (M + $H^+$); ber. 408 amu

$^1$H-NMR ($d_6$-DMSO): δ = 1.15 (t, 3H), 1.20 (t, 3H), 1.24 (t, 3H), 3.30 - 3.39 (m, 4H), 3.45 (q, 2H), 7.37 (d, 2H), 7.69 (d, 1 H), 8.34 - 8.39 (m, 3H), 9.11 (s, 1 H), 9.48 (s, 1H), 10.15 (s, 1 H) ppm.

Beispiel 13: (E)-3-Phenyl-acrylsäure-4-[6-(3-ethyl-harnstoff)-pyrido[2,3-b]pyrazin-3-yl]-phenyl-ester

Herstellung von (E)-3-Phenyl-acrylsäure-4-[6-(3-ethyl-harnstoff)-pyrido[2,3-b]pyrazin-3-yl]-phenyl-ester

38.9 mg 1-Ethyl-3-[3-(4-hydroxy-phenyl)-pyrido[2,3-b]pyrazin-6-yl]-harnstoff (0.13 mmol) werden in 3.0 ml getrocknetem Pyridin vorgelegt. 17.5 mg Dimethylaminopyridin (0.14 mmol) und 22.9 mg *trans*-Zimtsäurechlorid (0.13 mmol) werden bei Raumtemperatur zugegeben und das Reaktionsgemisch wird bei Raumtemperatur gerührt. Nach 3h werden nochmal 22.9 mg trans-Zimtsäurechlorid (0.13 mmol) zugegeben und das Gemisch weitere 3h bei Raumtemperatur gerührt. Zur Aufarbeitung gießt man das Gemisch auf Eiswasser und neutralisiert mit 1N Salzsäure. Der ausgefallene Niederschlag wird abgesaugt und im Vakuum getrocknet. Ohne weitere Aufreinigung erhält man als Produkt einen gelben Feststoff.

Smp.: 236-238 °C

ESI-MS: gef. m/z = 440.3 (M + $H^+$); ber. 439 amu

$^1$H-NMR ($d_6$-DMSO): δ = 1.20 (t, 3H), 3.25 - 3.40 (m, 2H), 6.95 (d, 1 H), 7.46 - 7.51 (m, 5H), 7.70 (d, 1 H), 7.83 - 7.87 (m, 2H), 7.94 (d, 1 H), 8.38 (d, 1 H), 8.43 (d, 2H), 9.12 (s, 1 H), 9.49 (s, 1H), 10.19 (s, 1 H) ppm.

Beispiel 14: Nonadecansäure-4-[6-(3-ethyl-harnstoff)-pyrido[2,3-b]pyrazin-3-yl]-phenyl-ester

Herstellung von Nonadecansäure-4-[6-(3-ethyl-harnstoffrpyrido[2,3-b]pyrazin-3-yl]-phenyl-ester

50.0 mg 1-Ethyl-3-[3-(4-hydroxy-phenyl)-pyrido[2,3-b]pyrazin-6-yl]-harnstoff (0.16 mmol) werden in 5.0 ml getrocknetem Pyridin vorgelegt. 19.7 mg Dimethylaminopyridin (0.16 mmol) und 51.2 mg Nonadecanoylchlorid (0.16 mmol) werden bei Raumtemperatur zugegeben und das Reaktionsgemisch wird 4 h bei Raumtemperatur gerührt. Zur Aufarbeitung gibt man das Reaktionsgemisch in ca. 200 ml destilliertes Wasser. Der ausgefallene Niederschlag wird abgesaugt und mit Wasser gewaschen. Nach säulenchromatographischer Reinigung des Rohproduktes an Kieselgel (Laufmittel Dichlormethan/Methanol) erhält man einen weißen Feststoff.

Smp.: 147.2 °C

$^1$H-NMR ($d_6$-DMSO): δ = 0.90 (t, 3H), 1.20 - 1.50 (m, 33H), 1.75 - 1.85 (m, 2H), 2.60 - 2.68 (m, 2H), 3.50 - 3.60 (m, 2H), 7.26 - 7.36 (m, 3H), 8.25 - 8.31 (m, 3H), 8.71 (s, 1 H), 9.22 (s, 1 H), 9.75 (s, 1 H) ppm.

Beispiel 15: 1-(3-Benzyl-pyrido[2,3-b]pyrazin-6-yl]-3-ethyl-harnstoff

Herstellung von 1-(3-Benzyl-pyrido[2,3-b]pyrazin-6-yl]-3-ethyl-harnstoff (Umsetzung gemäß Schema 6)

2.4 ml einer 0.5 M Lösung von Benzylzinkbromid (1.20 mmol) in THF und 24.7 mg Tetrakis(triphenylphosphin)-palladium(0) (0.02 mmol) werden unter Stickstoff in 1 ml Dioxan vorgelegt. Anschließend werden portionsweise 102.4 mg 1-(3-Chlor-pyrido[2,3-b]pyrazin-6-yl)-3-ethyl-harnstoff (0.41 mmol) und 1 ml Dioxan zugegeben. Danach wird

das Gemisch auf 100 ˚C erhitzt. Nach 7h lässt man das Reaktionsgemisch abkühlen und gibt dann gesättigte Ammoniumchlorid-Lösung hinzu. Die wässrige Phase wird mehrfach mit Dichlormethan extrahiert und die gesammelten organischen Phasen werden mit gesättigter Kochsalz-Lösung gewaschen, über Natriumsulfat getrocknet und im Vakuum vom Lösungsmittel befreit. Nach säulenchromatographischer Aufreinigung an Kieselgel (Laufmittel Dichlormethan/Methanol) erhält man einen gelben Feststoff.

ESI-MS: gef. m/z = 308.3 (M + $H^+$); ber. 307 amu

$^1H$-NMR ($d_6$-DMSO): $\delta$ = 1.19 (t, 3H), 3.25 - 3.35 (m, 2H), 4.35 (s, 2H), 7.25 (t, 1 H), 7.30 - 7.39 (m, 4H), 7.64 (d, 1 H), 8.32 (d, 1 H), 8.77 (s, 1 H), 9.05 (s, 1 H), 10.08 (s, 1 H) ppm.

Tabelle 1: Ausführungsbeispiele

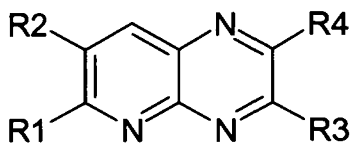

II

R1 / R2: Wasserstoff oder 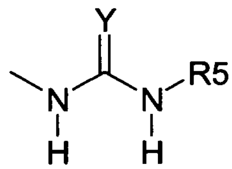

| Beispiel | R1 | R2 | R3 | R4 | R5 |
|---|---|---|---|---|---|
| 1 | Y = O | H | Phenyl-ethinyl- | H | Ethyl- |
| 2 | Y = O | H | Thiophen-3-yl-ethinyl- | H | Ethyl- |
| 3 | Y = O | H | Cyclopropyl-ethinyl- | H | Ethyl- |
| 4 | Y = O | H | Dimethylamino-prop-1-inyl- | H | Ethyl- |
| 5 | Y = O | H | (E)-2-Cyclohexyl-vinyl- | H | Ethyl- |
| 6 | Y = O | H | (E)-3-Methoxy-propenyl- | H | Ethyl- |
| 7 | Y = O | H | Phenyl- | H | Methoxy- |
| 8 | Y = O | H | Phenyl- | H | Allyloxy- |
| 9 | Y = O | H | 4-(MeO-C(O)O)-phenyl- | H | Ethyl- |
| 10 | Y = O | H | 4-($MeOCH_2CH_2O$-C(O)O)-phenyl- | H | Ethyl- |
| 11 | Y = O | H | 4-(PhO-C(O)O)-phenyl- | H | Ethyl- |
| 12 | Y = O | H | 4-($Et_2N$-C(O)O)-phenyl- | H | Ethyl- |
| 13 | Y = O | H | 4-((E)-PhCH=CHC(O)O)-phenyl- | H | Ethyl- |
| 14 | Y = O | H | $C_{18}H_{37}$-C(O)O-phenyl- | H | Ethyl- |
| 15 | Y = O | H | Benzyl- | H | Ethyl- |

## Biologische Wirkungen der erfindungsgemäßen Verbindungen

1. Zellfreie Kinaseassays (mittels ALPHA-Technologie)

Die inhibitorische Wirkung der erfindungsgemäßen Verbindungen wurde an diversen humanen Serin/ Threonin-, Tyrosin- und Lipidkinasen in enzymatischen Assays getestet. Dabei wurden rekombinante humane Kinasen wie z.B. Erk2, PI3Kalpha, - beta, -gamma, -delta, p38alpha, p38gamma, Jnk1, Jnk2 und andere eingesetzt, teils als Volllängenkinasen, teils als verkürzte Fragmente - mindestens aber bestehend aus der funktionellen Kinasedomäne. Die käuflichen Kinaseproteine (Proqinase, Upstate) wurden als rekombinante Fusionsproteine mit GST-(Glutathion-S-Transferase) oder His-Tag eingesetzt. Je nach Substrattyp wurden die verschiedenen Kinasereaktionen mittels geeigneter ALPHA™-beads (PerkinElmer) quantifiziert.

Testung

Nachfolgend wird die Substanztestung am Erk-Assay genauer beschrieben. Ausgewählte Testergebnisse der Erk2-, PI3Kalpha-Assays sind unten aufgeführt. Zur Bestimmung des $IC_{50}$-Wertes wurden die potenziellen Inhibitorsubstanzen bei 10 halblogaritmisch abgestuften Konzentration von 3.16nM-100μM untersucht.

a) Erk2-ALPHA: Die Testsubstanz, 0.625ng Erk2 (#14-173, Upstate), 10μM ATP und 15nM biotinyliertem MBP (myelin basic protein)-Substrat wurden auf einer 384er Optiplate (PerkinElmer) in einem Volumen von 15μl für 1h in 25mM Tris, 10mM $MgCl_2$, 0.1% Tween-20, 100μM $NaVO_4$, 2mM DTT bei pH 7.5 inkubiert. Anschließend wurde die Kinasereaktion durch Zugabe von 10μl des mit anti-phospho MBP-Antikörpers (320pM, #05-429/ Upstate) präinkubierten ALPHA-Beadmixes (10μg/ml, #6760617/ PerkinElmer) in 25mM Tris, 200mM NaCl, 100mM EDTA und 0.3% BSA abgestoppt und über Nacht stehen gelassen.

b) PI3K-ALPHAs (z.B. PI3Kalpha): Die Testsubstanz, 1ng PI3Kalpha (#14-602, Upstate), 100μM ATP und 20μM $PIP_2$-Substrat (#P4508, Echelon) auf einer 384er Optiplate (PerkinElmer) für 1h in 50mM Hepes, 50mM NaCl, 5mM $MgCl_2$, 0.05% Chaps, 5mM DTT bei pH 7.4 inkubiert. Anschließend wurde die Kinasereaktion durch Zugabe des mit 1nM GST:Grp1-Fusionsprotein (Upstate) und 15nM biotinyliertem PIP3 (#C-39B6/ Echelon) präinkubierten ALPHA-Beadmixes (10μg/ml, #6760603/ PerkinElmer) in 50mM Hepes, 50mM NaCl, 50mM EDTA und 0.1% BSA abgestoppt und über Nacht stehen gelassen.

Die Fluoreszenzdetektion erfolgte am nächsten Morgen in einem Fusion™alpha-Gerät (PerkinElmer).

Auswertung

Die Berechnung der %-Inhibitionswerte je Substanz-Konzentration geschah mittels folgender Formel aus den im Fusion™alpha ermittelten Rohdaten:

$$\%\ \text{Kinase Inhibition}_{(\text{Probe})} = 100 - \left(100x\,\frac{\text{Mittelwert}_{(\text{Probe})} - \text{Mittelwert}_{(0\%\ \text{Kontrolle})}}{\text{Mittelwert}_{(100\%\ \text{Kontrolle})} - \text{Mittelwert}_{(0\%\ \text{Kontrolle})}}\right)$$

Die Kontrollen wurden jeweils 8fach, die Substanzproben jeweils 2fach bestimmt. Die 0%-Kontrolle enthielt entweder kein ATP oder kein Substrat, die 100%-Kontrolle enthielt keine Testsubstanz. Die $IC_{50}$-Werte wurden mit GraphPadPrism ermittelt.

Die erfindungsgemäßen Verbindungen zeigten eine effektive Inhibition der Erk und der PI3K mit $IC_{50}$-Werten bis zu 88nM (siehe Ausführungsbeispiele in Tabelle 2 unten).

Tabelle 2:

| | $IC_{50}$ [μM] bei 10μM bzw. 100μM* ATP | |
|---|---|---|
| Ausführungsbeispiele | Erk2 | PI3Kalpha |
| 3 | 5.8 | 16.8 |
| 6 | 2.4 | 21.3 |
| 7 | 0.407 | 27.9 |
| 8 | 4.1 | 27.2 |
| 9 | 0.117 | 7.8 |
| 10 | 0.088 | 2.6 |
| 11 | 0.27 | >31.6 |
| 12 | 2.8 | 3.4 |
| 13 | 0.64 | >31.6 |

2. Zellulärer Assay: Testung auf anti-proliferative Wirkung (XTT-Assay)

Das Prinzip dieses Testes beruht auf der intrazellulären Reduktion des Tetrazolium-Farbstoffes XTT (Natrium 3'-[1-(phenylaminocarbonyl)-3,4-tetrazolium]-bis(4-methoxy-6-nitro)benzensulfonsäure, Sigma) zu einem Formazan-Farbstoff durch mitochondriale Dehydrogenasen. Der Farbstoff wird nur von stoffwechselaktiven Zellen gebildet, seine photometrisch messbare Intensität ist ein quantitativer Indikator für das Vorhandensein lebender Zellen. Die Reduzierung der Farbstoffbildung durch Inkubation der Zellen mit Substanzen dient als Parameter für die anti-proliferative Wirkung.

Testung

Die Tumorzelllinien (ATCC) wurden in 96er Mikrotiterplatten in definierter Zellzahl (5000 Zellen/ Well für BxPC3 und Hct116; 10000 Zellen/ Well für MDA MB468) eingesäat und anschließend über Nacht im Brutschrank bei 37˚C, 5% $CO_2$ und 95% Luftfeuchtigkeit inkubiert. Die Testsubstanzen wurden als Stammlösungen (10 mM) in DMSO angesetzt. Zur Bestimmung der $EC_{50}$-Werte wurden die potenziellen Inhibitorsubstanzen in viertellogarithmisch abgestuften Verdünnungen zu den Zellen gegeben, sodass finale Konzentrationen von 0.28µM-50µM resulierten. Die Zellplatten wurden dann für 45h im Brutschrank bei 37˚C, 5% $CO_2$ und 95% Luftfeuchtigkeit inkubiert.

Für die Detektionssreaktion wurde das Substrat XTT mit PMS (N-Methyl Dibenzopyrazine Methylsulfat, Sigma) versetzt und zu den Zellen gegeben, sodass eine finale Konzentration von 325µg XTT/ml und 2.5µg PMS/ml resultierte. Es wurde dann für 3h bei 37˚C, 95% Luftfeuchte inkubiert. Anschließend konnte das durch zelluläre Dehydrogenasen gebildete Formazan Salz bei einer Adsorption bei 490nm quantifiziert werden.

Auswertung

Die Auswertung der %-Inhibitions-Werte geschah mittels folgender Formel aus den Werten für die jeweils gemessenen optischen Dichten bei 490nm:

$$\text{\% Inhibition Zellproliferation}_{(\text{Probe})} = 100 - \left( 100x \frac{\text{Mittelwert}_{(\text{Probe})} - \text{Mittelwert}_{(0\%\ \text{Kontrolle})}}{\text{Mittelwert}_{(100\%\ \text{Kontrolle})} - \text{Mittelwert}_{(0\%\ \text{Kontrolle})}} \right)$$

Die Kontrollen wurden jeweils 8fach, die Substanzproben jeweils 2fach bestimmt. Die 0%-Kontrolle enthielt keine Zellen, die 100%-Kontrolle enthielt keine Testsubstanz. Die $EC_{50}$-Werte wurden mit GraphPadPrism ermittelt.

Die erfindungsgemäßen Verbindungen zeigten eine teils effektive Inhibition der Zellproliferation mit $EC_{50}$-Werten bis zu 5µM (siehe Ausführungsbeispiele in Tabelle 3 unten).

Tabelle 3:

| | $EC_{50}$ [µM] | | |
|---|---|---|---|
| Ausführungsbeispiele | BxPC3 | MDA-MB468 | Hct116 |
| 10 | 9 | 7 | 5 |
| 9 | 20 | 15 | 10 |

3. Zellulärer Assay: Testung auf Substrat-Inhibtion (Western Blotting)

Diese Methode ermöglicht eine Aussage darüber, ob der untersuchte Kinasemodulator auch in einem zellulären Kontext die gewünschte Wirkung erzielt d.h. hierbei wird ein der Targetkinase nachgeschaltetes Substrat-Protein auf seinen Phosphorylierungs-Status untersucht. Dazu werden die mit Substanz inkubierten Zellen lysiert und das Gesamtprotein auf einem reduzierenden Polyacrylamidgel aufgetrennt. Anschließend werden die Proteine mittels Western Blotting auf eine PVDF-Membran transferiert und die gesuchten Substratbanden mit spezifischen Antikörpern und einer geeigneten Detektionsmethode sichtbar gemacht. Die den Targetkinasen nachgeschalteten Substratproteine werden mit einem jeweils speziellen anti-Phospho-Antikörper und gleichzeitig einem total-Antikörper, welcher das Substrat-Totalprotein erkennt, gleichzeitig detektiert. Die Duplex-Technologie des ODYSSEY-Imagers (LiCOR) ermöglicht diese simultane Messung. Die Intensität der Totalsubstrat-Banden wird zur Normalisierung bzw. Quantifizierung der Phosphorylierungs-Inhibition oder Aktivierung heran gezogen.

Testung

Geeignete Tumorzelllinien (z.B. BxPC3, Hct116 oder MDA MB468) wurden in 6-Well Mikrotiterplatten in definierter Zellzahl (z.B. 350 000 Zellen/ Well für BxPC3 und Hct116) in den jeweiligen Standard-Vollmedien eingesäat und anschließend über Nacht im Brutschrank bei 37˚C, 5% $CO_2$ und 95% Luftfeuchtigkeit inkubiert. Die Zellen wurden anschließend für weitere 24h unter Serum-reduzierten Bedingungen d.h. im jeweiligen Medium, jedoch bei nur 0.25% Serum, weiter inkubiert. Die Testsubstanzen wurden als Stammlösungen (10 mM) in DMSO angesetzt und bei finalen Konzentrationen von 5, 15.8 und 50μM für 5h mit den Zellen inkubiert. Daraufhin erfolgte die Zelllyse in 25mM Tris, 150mM NaCl, 10mM Na-Pyrophosphat, 2mM EGTA, 25mM beta-Glycerophosphat, 25mM NaF, 10% Glycerin, 0.75% NP-40, 100μM $NaVO_4$-Puffer. Nach Proteinquantifizierung mittels BCA-(Bicinchonic acid protein assay kit, Sigma)Assay wurden Proteinmengen von etwa 20μg pro Laufspur auf einem Lämmli-Polyacrylamidgel aufgetrennt und danach mittels Semi-Dry Western-Blotting bei 0.8mA/$cm^2$ für 1h auf eine PVDF-Membran (Millipore) transferiert. Es folgte eine 1 stündige Prähyridisierung der Membran in I-Block Reagenz (Applied Biosystems) und die Über-Nacht-Inkubation mit den spezifischen Antikörpern. Zur Bestimmung der Erk- und PI3K-Inhibition wurden die jeweils abwärts folgenden Substrate Rsk1 mit dem Total-Antikörper (Rsk #sc-231g C-21, Santa Cruz) und dem Phospho-Antikörper (Phospho-p90RSK (S380) #9341, NEB Cell Signalling) und Akt mit dem Total-Antikörper (Akt1 #sc-1618 C-20, Santa Cruz) und dem Phospho-Antikörper (Phospho-Akt (Ser 473) #9271, NEB Cell Signaling) detektiert. Nach Waschen der Membran erfolgte die Sekundärantikörperinkubation mit anti-Kaninchen IR Dye 800 (#611-732-127, Rockland) für die Phospho-Antikörper und anti-Ziege Alexa Fluor 680 (#A-21081, Molecular Probes) für die Totalprotein-Antikörper. Nach Inkubation für 30min bei Raumtemperatur im Dunkeln wurde die Hybridisierung des Detektionsantikörpers an die Membran durch Scannen im ODYSSEY-Imager (LiCOR) detektiert.

Auswertung

Bei Konzentrationen von 5-50μM zeigten die erfindungsgemäßen Verbindungen eine duale Inhibition der Erk (MAPK1/2) und der PI3K (siehe Ausführungsbeispiele in Tabelle 4 unten), welche durch Hemmung der Bandenintensität beider korrespondierender phospho-Substratproteine Rsk1 und Akt angezeigt wird.

Tabelle 4:

| Ausführungsbeispiele | Inhibition Substratphosphorylierung (bei 50μM) | |
|---|---|---|
| | Erk →pRsk | PI3K →pAkt |
| 10 | 100% | 100% |
| 9 | 90% | 50% |

Abkürzungen

| | |
|---|---|
| Akt | von: murine Akt8 retrovirus oder protein kinase B (PKB) |
| Ask1 | apoptosis signal-regulating kinase |
| ATR | ataxia-telangiectasia and Rad3-related |
| ATM | Ataxia-telangiectasia mutated |
| Bag1 | Bcl-2 associated athanogene-1 |
| Bcl-2 | B-cell leukemia/lymhoma-2 gene |
| DNA-PK | DNA-dependent protein kinase |
| Erk | extracellular signal-regulated kinase |
| Flt-3 | fms like tyrosine kinase 3 |
| GSK-3 | Glycogen synthase kinase-3 |
| hSMG-1 | human ortholog of product of seven nematode gene-1 |
| JAK-3 | Janus kinase 3 |
| JNK | c-jun N-terminal kinase |
| MAPK | mitogen activated protein kinase |
| Mek | MAP or Erk kinase |
| mTOR | mammalian target of rapamycin |
| PDGFR | platelet derived growth factor receptor |
| PI3K | phosphoinositol 3-Kinase |

| | |
|---|---|
| PIKK | phosphoinositol 3-Kinase related kinase |
| $PIP_2$ | phosphatidylinositol-biphosphat |
| $PIP_3$ | phosphatidylinositol-triphosphat |
| PtdIns | phosphatidylinositol |
| Raf | rapid accelerated fibrosarcoma |
| Ras | rat sarcoma |
| RTK | receptor tyrosine kinase |
| SAPK | stress-activated protein kinase |
| Ser | Serin |
| Syk | spleen tyrosine kinase |
| Thr | Threonin |
| Tyr | Tyrosin |
| VEGFR | vascular endothelial growth factor receptor |

**Patentansprüche**

1. Neue Pyrido[2,3-b]pyrazin-Derivate gemäß der allgemeinen Formel I

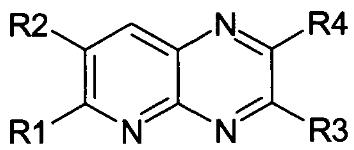

**I**

worin die Substituenten R1-R4 folgende Bedeutung haben:

R1 und R2 können unabhängig voneinander Wasserstoff oder NR5R6 bedeuten, unter der Voraussetzung, dass, wenn R1 = NR5R6 ist, R2 = H ist, und wenn R2 = NR5R6 ist, R1 = H ist,
wobei R5 Wasserstoff, Alkyl, R38, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum mit F, Cl, Br, I, CN, $CF_3$, $NH_2$, NH-Alkyl, NH-Aryl, $N(Alkyl)_2$, $NO_2$, SH, S-Alkyl, OH, $OCF_3$, $O(-Alkyl-O)_p$-Alkyl, O-Aryl, $OSO_3H$, $OP(O)(OH)_2$, $OP(O)(OAlkyl)_2$, $OP(O)(OAryl)_2$ ,CHO, C(O)OH, C(O)OR12, $C(O)NH_2$, C(O)NHR12, C(O)NR12R13, $SO_3H$, $SO_2Alkyl$, $SO_2Aryl$, $P(O)(OH)_2$, $P(O)(OAlkyl)_2$, $P(O)(OAryl)_2$, Alkyl, Cycloalkyl, Heterocyclyl, Aryl, Heteroaryl ein- oder mehrfach, gleich oder verschieden substituiert sein können, p den Wert 0, 1, 2, 3, 4 oder 5 annehmen kann und die Reste R12 und R13 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R12 und R13 zusammen einen Heterocyclyl-Ring bilden können
und R6:
-C(Y)NR7R8 bedeuten kann, wobei Y unabhängig voneinander O oder S sein kann und R7 und R8 unabhängig voneinander Wasserstoff,
unsubstituiertes oder substituiertes Alkyl, wobei der Alkylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, $O(-Alkyl-O)_p$-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, $C(O)-NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Cycloalkyl, Heterocyclyl, Aryl oder Heteroaryl, ein- oder mehrfach, gleich oder verschieden substituiert sein kann, wobei p den Wert 0, 1, 2, 3, 4 oder 5 annehmen kann,

unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC (O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, OH, $O(\text{-Alkyl-O})_p$-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O) NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, Alkyl, oder Aryl ein- oder mehrfach, gleich oder verschieden substituiert sein kann, wobei p den Wert 0, 1, 2, 3, 4 oder 5 annehmen kann,

unsubstituiertes oder substituiertes Heterocyclyl, wobei der Heterocyclylrest mit OH, O-Alkyl, O-Aryl, $NH_2$, NH-Alkyl, NH-Aryl, Alkyl, Alkyl-Aryl oder Aryl ein- oder mehrfach, gleich oder verschieden substituiert sein kann,

unsubstituiertes oder substituiertes Aryl, wobei der Arylrest mit F, Cl, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-R38, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, $O(\text{-Alkyl-O})_p$-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C (O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, S03H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, wobei p den Wert 0, 1, 2, 3, 4 oder 5 und n den Wert 1, 2 oder 3 annehmen kann,

unsubstituiertes oder substituiertes Heteroaryl, wobei der Heteroarylrest F, Cl, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-R38, NHC (O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, $O(\text{-Alkyl-O})_p$-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2NH_2$, $SO_2NH$-Alkyl, $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, wobei p den Wert 0, 1, 2, 3, 4 oder 5 annehmen kann,

-C(O)-R39, wobei R39 Alkyl, Aryl oder Heteroaryl sein kann, und die Alkyl-, Aryl- und Heteroarylsubstituenten ihrerseits wiederum substituiert sein können,
sein können, oder R7 und R8 zusammen einen Heterocyclyl-Ring bilden können,
R3 und R4 können unabhängig voneinander:
Wasserstoff, wobei R3 und R4 nicht gleichzeitig Wasserstoff sind,
substituiertes Alkyl, wobei der Alkylrest mit F, Cl, Br, I, CN, $CF_3$, $NH_2$, NH-Alkyl, NH-Aryl, $N(Alkyl)_2$, SH, S-Alkyl, OH, $OCF_3$, $O(-Alkyl-O)_p$-Alkyl, O-Aryl, $OSO_3H$, $OP(O)(OH)_2$, $OP(O)(OAlkyl)_2$, $OP(O)(OAryl)_2$, C(O)OH, C(O)OR14, $C(O)NH_2$, C(O)NHR14, C(O)NR14R15, $SO_3H$, $SO_2Alkyl$, $SO_2Aryl$, $P(O)(OH)_2$, $P(O)(OAlkyl)_2$, $P(O)(OAryl)_2$, Cycloalkyl, Heterocyclyl, Aryl oder Heteroaryl ein- oder mehrfach, gleich oder verschieden substituiert ist, wobei p den Wert 0, 1, 2, 3, 4 oder 5 annehmen kann und die Reste R14 und R15 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R14 und R15 zusammen einen Heterocyclyl-Ring bilden können,
substituiertes Aryl, wobei der Arylrest ein- oder mehrfach, gleich oder verschieden substituiert ist mit Substituenten ausgewählt aus der Gruppe NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, $N(Aryl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, S-Alkyl, S-Aryl, S-Heteroaryl, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, C(O)-Alkyl, C(O)-Aryl, C(O)-Heteroaryl, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH$-Alkyl, $SO_2NH$-Aryl, $SO_2NH$-Heteroaryl, $SO_2NH$-Alkyl-Aryl, $SO_2O$-Alkyl, $SO_2O$-Aryl, $SO_2O$-Alkyl-Aryl, Alkyl, Cycloalkyl, Heterocyclyl, Aryl, Heteroaryl und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum mit $O(-Alkyl-O)_p$-Alkyl, $OP(O)(OAlkyl)_2$, $OP(O)(OAryl)_2$, C(O)OR16, $C(O)NH_2$, C(O)NHR16, C(O)NR16R17, $SO_2Alkyl$, $SO_2Aryl$, $P(O)(OH)_2$, $P(O)(OAlkyl)_2$, $P(O)(OAryl)_2$, Cycloalkyl, Heterocyclyl, Aryl, Heteroaryl oder Alkylaryl substituiert sind, wobei p den Wert 1, 2, 3, 4 oder 5 annehmen kann und die Reste R16 und R17 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R16 und R17 zusammen einen Heterocyclyl-Ring bilden können, unter der Voraussetzung, dass, wenn R3 oder R4 Alkyl-Heterocyclyl-substituiertes Aryl bedeutet, entsprechend R4 bzw. R3 $\neq$ Aryl ist,
und wobei der Arylrest ein- oder mehrfach, gleich oder verschieden substituiert ist mit Substituenten ausgewählt aus der Gruppe NR20-Alkyl, NH-R38, NHC(O)-R38, NR19C(O)-Alkyl, NR19C(O)-Cycloalkyl, NR19C(O)-Heterocyclyl, NR19C(O)-Aryl, NR19C(O)-Heteroaryl, NR18C(O)-Alkyl-Cycloalkyl, NR18C(O)-Alkyl-Heterocyclyl, NR19C(O)-Alkyl-Aryl, NR19C(O)-Alkyl-Heteroaryl, NR18C(O)O-R19, NR18C(O)NR18R18, O-R38, OC(O)-R38, OC(O)-Alkyl-Cycloalkyl, OC(O)-Alkyl-Heterocyclyl, OC(O)O-R19, OC(O)NR18R18, $OP(O)(OAlkyl)_2$, $OP(O)(OAryl)_2$, C(O)O-R38, C(O)NH-R38, C(O)NR20-Alkyl, C(O)NR19-Alkyl-R21, C(O)NR18O-R18, C(O)NR18NR18R18 und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum mit F, Cl, Br, I, CN, $CF_3$, $NH_2$, NH-Alkyl, NH-Aryl, $N(Alkyl)_2$, $NO_2$, SH, S-Alkyl, OH, $OCF_3$, $O(-Alkyl-O)_p$-Alkyl, O-Aryl, $OSO_3H$, $OP(O)(OH)_2$, $OP(O)(OAlkyl)_2$, $OP(O)(OAryl)_2$, CHO, C(O)OH, C(O)OR22, $C(O)NH_2$, C(O)NHR22, C(O)NR22R23, $SO_3H$, $SO_2Alkyl$, $SO_2Aryl$, $P(O)(OH)_2$, $P(O)(OAlkyl)_2$, $P(O)(OAryl)_2$, Alkyl, Cycloalkyl, Heterocyclyl, Aryl, Heteroaryl oder Alkylaryl substituiert sein können, wobei p den Wert 0, 1, 2, 3, 4 oder 5 annehmen kann und die Reste R22 und R23 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R22 und R23 zusammen einen Heterocyclyl-Ring bilden können,
substituiertes Heteroaryl, wobei der Heteroarylrest ein- oder mehrfach, gleich oder verschieden substituiert ist mit Substituenten ausgewählt aus der Gruppe NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, $N(Aryl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, S-Alkyl, S-Aryl, S-Heteroaryl, O-Alkyl, O-Cycloalkyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Al-

kyl-Heteroaryl, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, C(O)-Alkyl, C(O)-Aryl, C(O)-Heteroaryl, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, C(O)N$(Cycloalkyl)_2$, C(O)N$(Aryl)_2$, C(O)N$(Heteroaryl)_2$, $SO_2$NH-Alkyl, $SO_2$NH-Aryl, $SO_2$NH-Heteroaryl, $SO_2$NH-Alkyl-Aryl, $SO_2$O-Alkyl, $SO_2$O-Aryl, $SO_2$O-Alkyl-Aryl, Alkyl, Cycloalkyl, Heterocyclyl, Aryl, Heteroaryl und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum mit O(-Alkyl-O)$_p$-Alkyl, $OP(O)(OAlkyl)_2$, $OP(O)(OAryl)_2$, C(O)OR16, $C(O)NH_2$, C(O)NHR16, C(O)NR16R17, $SO_2$Alkyl, $SO_2$Aryl, $P(O)(OH)_2$, $P(O)(OAlkyl)_2$, $P(O)(OAryl)_2$, Cycloalkyl, Heterocyclyl, Aryl, Heteroaryl oder Alkylaryl substituiert sind, wobei p den Wert 1, 2, 3, 4 oder 5 annehmen kann und die Reste R16 und R17 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R16 und R17 zusammen einen Heterocyclyl-Ring bilden können,

und wobei der Heteroarylrest ein- oder mehrfach, gleich oder verschieden substituiert ist mit Substituenten ausgewählt aus der Gruppe NR20-Alkyl, NH-R38, NHC(O)-R38, NR19C(O)-Alkyl, NR19C(O)-Cycloalkyl, NR19C(O)-Heterocyclyl, NR19C(O)-Aryl, NR19C(O)-Heteroaryl, NR18C(O)-Alkyl-Cycloalkyl, NR18C(O)-Alkyl-Heterocyclyl, NR19C(O)-Alkyl-Aryl, NR19C(O)-Alkyl-Heteroaryl, NR18C(O)O-R19, NR18C(O)NR18R18, $NHSO_2$-Alkyl-Heterocyclyl, O-R38, O-Heterocyclyl, OC(O)-R38, OC(O)-Alkyl-Cycloalkyl, OC(O)-Alkyl-Heterocyclyl, OC(O)O-R19, OC(O)NR18R18, $OP(O)(OAlkyl)_2$, $OP(O)(OAryl)_2$, C(O)O-R38, C(O)NH-R38, C(O)NR20-Alkyl, C(O)NR19-Alkyl-R21, C(O)NR18O-R18, C(O)NR18NR18R18 und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum mit F, Cl, Br, I, CN, $CF_3$, $NH_2$, NH-Alkyl, NH-Aryl, $N(Alkyl)_2$, $NO_2$, SH, S-Alkyl, OH, $OCF_3$, O(-Alkyl-O)$_p$-Alkyl, O-Aryl, $OSO_3H$, $OP(O)(OH)_2$, $OP(O)(OAlkyl)_2$, $OP(O)(OAryl)_2$, CHO, C(O)OH, C(O)OR22, $C(O)NH_2$, C(O)NHR22, C(O)NR22R23, $SO_3H$, $SO_2$Alkyl, $SO_2$Aryl, $P(O)(OH)_2$, $P(O)(OAlkyl)_2$, $P(O)(OAryl)_2$, Alkyl, Cycloalkyl, Heterocyclyl, Aryl, Heteroaryl oder Alkylaryl substituiert sein können, wobei p den Wert 0, 1, 2, 3, 4 oder 5 annehmen kann und die Reste R22 und R23 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R22 und R23 zusammen einen Heterocyclyl-Ring bilden können,

NR24R25, wobei R24 -C(O)-R26, -$SO_2$R26, -C(O)OR26 oder -C(O)-NR27R28 sein kann und wobei R25 Wasserstoff, Alkyl, Cycloalkyl, Aryl oder Heteroaryl sein kann und wobei R26 Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl sein kann, und R27 und R28 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl sein können, oder R27 und R28 zusammen einen Heterocyclyl-Ring bilden können und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können,

und R18 Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R19 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R20 Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R21 Cycloalkyl, Heterocyclyl, Aryl, Heteroaryl sein kann,

und R38 Alkyl sein kann, wobei der Alkylrest gesättigt oder ungesättigt, geradkettig oder verzweigt sein kann, mit 9 bis 30 C-Atomen, und die $C_9$-$_{30}$-Alkenyle mindestens eine C-C-Doppelbindung und $C_9$-$_{30}$-Alkinyle mindestens eine C-C-Dreifachbindung aufweisen, wobei die Alkenyle sowohl in (E)- als auch in (Z)-Konformation vorliegen können,

bedeuten;

sowie physiologisch verträgliche Salze, Derivate oder Analoga der Verbindungen nach Formel I, sowie deren Solvate, Hydrate, polymorphe Formen und Prodrugs,

wobei die Verbindungen der allgemeinen Formel I sowie deren Salze, Derivate oder Analoga, deren Solvate, Hydrate, polymorphe Formen und Prodrugs in Form ihrer Racemate, in Form der reinen Enantiomeren und/oder Diastereomeren oder in Form von Mischungen dieser Enantiomeren und/oder Diasteromeren oder in Form der Tautomeren vorliegen können.

**2.** Neue Pyrido[2,3-b]pyrazin-Derivate der allgemeinen Formel I worin die Substituenten R1-R4 folgende Bedeutung haben:

R1 und R2 können unabhängig voneinander Wasserstoff oder NR5R6 bedeuten, unter der Voraussetzung, dass, wenn R1 = NR5R6 ist, R2 = H ist, und wenn R2 = NR5R6 ist, R1 = H ist,
wobei R5 Wasserstoff, Alkyl, R38, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum mit F, Cl, Br, I, CN, $CF_3$, $NH_2$, NH-Alkyl, NH-Aryl, $N(Alkyl)_2$, $NO_2$, SH, S-Alkyl, OH, $OCF_3$, $O(-Alkyl-O)_p$-Alkyl, O-Aryl, $OSO_3H$, $OP(O)(OH)_2$, $OP(O)(OAlkyl)_2$, $OP(O)(OAryl)_2$, CHO, C(O)OH, $C(O)NH_2$, C(O)OR12, C(O)NHR12, C(O) NR12R13, $SO_3H$, $SO_2$Alkyl, $SO_2$Aryl, $P(O)(OH)_2$, $P(O)(OAlkyl)_2$, $P(O)(OAryl)_2$, Alkyl, Cycloalkyl, Heterocyclyl, Aryl, Heteroaryl ein- oder mehrfach, gleich oder verschieden substituiert sein können, p den Wert 0, 1, 2, 3, 4 oder 5 annehmen kann und die Reste R12 und R13 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R12 und R13 zusammen einen Heterocyclyl-Ring bilden können
und R6:
-C(O)NR9-Y-R10 bedeuten kann, wobei Y unabhängig voneinander O oder NR11 sein kann
und R9 Wasserstoff, Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl sein kann und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits substituiert sein können,
und R10 und R11 unabhängig voneinander
Wasserstoff,
unsubstituiertes oder substituiertes Alkyl,
unsubstituiertes oder substituiertes Cycloalkyl,
unsubstituiertes oder substituiertes Heterocyclyl, wobei der Heterocyclylrest mit OH, O-Alkyl, O-Aryl, $NH_2$, NH-Alkyl, NH-Aryl, Alkyl, Alkyl-Aryl oder Aryl ein- oder mehrfach, gleich oder verschieden substituiert sein kann,
unsubstituiertes oder substituiertes Aryl, wobei der Arylrest mit F, Cl, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-R38, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, $O(-Alkyl-O)_p$-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, $O-(CH_2)_n-O$, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C (O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, SO3H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, wobei p den Wert 0, 1, 2, 3, 4 oder 5 und n den Wert 1, 2 oder 3 annehmen kann,
unsubstituiertes oder substituiertes Heteroaryl, wobei der Heteroarylrest F, Cl, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-R38, NHC (O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, $O(-Alkyl-O)_p$-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2NH_2$, $SO_2NH$-Alkyl, $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Alkyl, Cycloalkyl, Heterocyclyl, Aryl oder Heteroaryl,

ein- oder mehrfach, gleich oder verschieden substituiert sein kann, wobei p den Wert 0, 1, 2, 3, 4 oder 5 annehmen kann,

sein können, oder R10 und R11 zusammen einen Heterocyclyl-Ring bilden können,

R3 und R4 können unabhängig voneinander:

Wasserstoff

Hydroxyl

Halogen, wie Fluor, Chlor, Brom, Iod

unsubstituiertes oder substituiertes Alkyl, wobei der Alkylrest mit F, Cl, Br, I, CN, $CF_3$, $NH_2$, NH-Alkyl, NH-Aryl, $N(Alkyl)_2$, SH, S-Alkyl, OH, $OCF_3$, $O(-Alkyl-O)_p$-Alkyl, O-Aryl, $OSO_3H$, $OP(O)(OH)_2$, $OP(O)(OAlkyl)_2$, $OP(O)(OAryl)_2$, C(O)OH, C(O)OR14, $C(O)NH_2$, C(O)NHR14, C(O)NR14R15, $SO_3H$, $SO_2$Alkyl, $SO_2$Aryl, $P(O)(OH)_2$, $P(O)(OAlkyl)_2$, $P(O)(OAryl)_2$, Cycloalkyl, Heterocyclyl, Aryl oder Heteroaryl ein- oder mehrfach, gleich oder verschieden substituiert sein kann, wobei p den Wert 0, 1, 2, 3, 4 oder 5 annehmen kann und die Reste R14 und R15 unabhängig voneinander Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können oder R14 und R15 zusammen einen Heterocyclyl-Ring bilden können,

unsubstituiertes oder substituiertes Aryl, wobei der Arylrest mit F, Cl, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, $N(Aryl)_2$, NR20-Alkyl, NHC(O)-Alkyl, NHC(O)-R38, NR19(O)-Alkyl, NHC(O)-Cycloalkyl, NR19C(O)-Cycloalkyl, NHC(O)-Heterocyclyl, NR19C(O)-Heterocyclyl, NHC(O)-Aryl, NR19C(O)-Aryl, NHC(O)-Heteroaryl, NR19C(O)-Heteroaryl, NR18C(O)-Alkyl-Cycloalkyl, NR18C(O)-Alkyl-Heterocyclyl, NHC(O)-Alkyl-Aryl, NR19C(O)-Alkyl-Aryl, NHC(O)-Alkyl-Heteroaryl, NR19C(O)-Alkyl-Heteroaryl, NR18C(O)O-R19, NR18C(O)NR18R18, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Heterocyclyl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, $O-(CH_2)_n-O$, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Cycloalkyl, OC(O)-Alkyl-Heterocyclyl OC(O)-Alkyl-Aryl, OC(O)-Alkyl-Heteroaryl, OC(O)O-R19, OC(O)NR18R18, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, $OP(O)(OAlkyl)_2$, $OP(O)(OAryl)_2$, C(O)-Alkyl, C(O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, $C(O)-NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, C(O)NR20-Alkyl, C(O)NR19-Alkyl-R21, -C(O)NR18O-R18, -C(O)NR18NR18R18, SO-Alkyl, SO-Aryl, $SO_2$-Alkyl, $SO_2$-Aryl, $SO_2NH_2$ $SO_2NH$-Alkyl $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Alkyl, Alkyl-Cycloalkyl, Alkyl-Heterocyclyl, Alkyl-Aryl, Alkyl-Heteroaryl, Cycloalkyl, Heterocyclyl, Aryl oder Heteroaryl, ein- oder mehrfach, gleich oder verschieden substituiert sein kann, n den Wert 1,2 oder 3 annehmen kann, und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum substituiert sein können,

unsubstituiertes oder substituiertes Heteroaryl, wobei der Heteroarylrest mit F, Cl, Br, I, $CF_3$, CN, $NH_2$, NH-Alkyl, NH-R38, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, $N(Aryl)_2$, NR20-Alkyl, NHC(O)-Alkyl, NHC(O)-R38, NR19C(O)-Alkyl, NHC(O)-Cycloalkyl, NR19C(O)-Cycloalkyl, NHC(O)-Heterocyclyl, NR19C(O)-Heterocyclyl, NHC(O)-Aryl, NR19C(O)-Aryl, NHC(O)-Heteroaryl, NR19C(O)-Heteroaryl, NR18C(O)-Alkyl-Cycloalkyl, NR18C(O)-Alkyl-Heterocyclyl, NHC(O)-Alkyl-Aryl, NR19C(O)-Alkyl-Aryl, NHC(O)-Alkyl-Heteroaryl, NR19C(O)-Alkyl-Heteroaryl, NR18C(O)O-R19, NR18C(O)NR18R18, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Heterocyclyl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-R38, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-R38, OC(O)-Cycloalkyl, OC(O)-Heterocyclyl, OC(O)-Aryl, OC(O)-Heteroaryl, OC(O)-Alkyl-Cycloalkyl, OC(O)-Alkyl-Heterocyclyl OC(O)-Alkyl-Aryl, OC(O)-Alkyl-Heteroaryl, OC(O)O-R19, OC(O)NR18R18, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, $OP(O)(OAlkyl)_2$, $OP(O)(OAryl)_2$, C(O)-Alkyl, C(O)-Aryl, C(O)-Heteroaryl, $CO_2H$, $CO_2$-Alkyl, $CO_2$-R38, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, $C(O)-NH_2$, C(O)NH-Alkyl, C(O)NH-R38, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, C(O)N(He-

teroaryl)$_2$, C(O)NR20-Alkyl, C(O)NR19-Alkyl-R21, -C(O)NR18O-R18, -C(O)NR18NR18R18, $SO_2NH_2$, $SO_2NH$-Alkyl, $SO_2NH$-Aryl, $SO_2NH$-Heteroaryl, $SO_2NH$-Alkyl-Aryl, S03H, $SO_2O$-Alkyl, $SO_2O$-Aryl, $SO_2O$-Alkyl-Aryl, Alkyl, Alkyl-Cycloalkyl, Alkyl-Heterocyclyl, Alkyl-Aryl, Alkyl-Heteroaryl, Cycloalkyl, Heterocyclyl, Aryl oder Heteroaryl ein- oder mehrfach, gleich oder verschieden substituiert sein kann, und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum substituiert sein können,

OR29, wobei R29 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl-, Heteroaryl-, Alkyl-Cycloalkyl, Alkyl-Heterocyclyl, Alkyl-Aryl oder Alkyl-Heteroarylsubstituenten ihrerseits wiederum substituiert sein können,

SR30, wobei R30 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl-, Alkyl-Heterocyclyl-, Alkyl-Aryl- oder Alkyl-Heteroarylsubstituenten ihrerseits wiederum substituiert sein können,

NR31 R32, wobei R31 und R32 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl, -C(O)-R33, $-SO_2$R33, -C(O)OR33 und -C(O)-NR34R35 sein können, wobei R33 Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl sein kann, und R34 und R35 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl, Alkyl-Heteroaryl sein können oder R34 und R35 zusammen einen Heterocyclyl-Ring bilden können,

oder R31 und R32 zusammen einen Heterocyclyl-Ring bilden können,

und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können,

und R18 Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R19 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R20 Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann,

und R21 Cycloalkyl, Heterocyclyl, Aryl, Heteroaryl sein kann,

und R38 Alkyl sein kann, wobei der Alkylrest gesättigt oder ungesättigt, geradkettig oder verzweigt sein kann, mit 9 bis 30 C-Atomen, und die $C_9$-$_{30}$-Alkenyle mindestens eine C-C-Doppelbindung und $C_9$-$_{30}$-Alkinyle mindestens eine C-C-Dreifachbindung aufweisen, wobei die Alkenyle sowohl in (E)- als auch in (Z)-Konformation vorliegen können,

sowie physiologisch verträgliche Salze, Derivate oder Analoga der Verbindungen nach Formel I, sowie deren Solvate, Hydrate, polymorphe Formen und Prodrugs,

wobei die Verbindungen der allgemeinen Formel I sowie deren Salze, Derivate oder Analoga, deren Solvate, Hydrate, polymorphe Formen und Prodrugs in Form ihrer Racemate, in Form der reinen Enantiomeren und/ oder Diastereomeren oder in Form von Mischungen dieser Enantiomeren und/oder Diasteromeren oder in Form der Tautomeren vorliegen können.

**3.** Pyrido[2,3-b]pyrazin-Derivate der allgemeinen Formel I nach einem der Ansprüche 1 bis 2, wobei R2 = H ist.

**4.** Pyrido[2,3-b]pyrazin-Derivate der allgemeinen Formel I nach einem der Ansprüche 1 bis 2, wobei R2 und R4 = H sind.

**5.** Pyrido[2,3-b]pyrazin-Derivate der allgemeinen Formel I nach Anspruch 1, wobei R2 und R4 = H sind und R6 = -C (Y)NR7R8 bedeutet, wobei Y unabhängig voneinander O oder S sein kann und R7 und R8 die im Anspruch 1 genannnte Bedeutung haben.

**6.** Pyrido[2,3-b]pyrazin-Derivate der allgemeinen Formel I nach den Ansprüchen 1 bis 5, insbesondere eine der folgenden Verbindungen:

1-Ethyl-3-(3-phenylethynyl-pyrido[2,3-b]pyrazin-6-yl)-harnstoff (Beispiel 1)
1-Ethyl-3-(3-thiophen-3-ylethynyl-pyrido[2,3-b]pyrazin-6-yl)-harnstoff (Bsp. 2)
1-(3-Cyclopropylethynyl-pyrido[2,3-b]pyrazin-6-yl)-3-ethyl-harnstoff (Bsp. 3)
1-[3-(3-Dimethylamino-prop-1-ynyl)-pyrido[2,3-b]pyrazin-6-yl]-3-ethyl-harnstoff (Bsp. 4)
1-[3-((E)-2-Cyclohexyl-vinyl)-pyrido[2,3-b]pyrazin-6-yl]-3-ethyl-harnstoff (Bsp. 5)
1-Ethyl-3-[3-((E)-3-methoxy-propenyl)-pyrido[2,3-b]pyrazin-6-yl]-harnstoff (Bsp. 6)

1-Methoxy-3-(3-phenyl-pyrido[2,3-b]pyrazin-6-yl)-harnstoff (Bsp. 7)
1-Allyloxy-3-(3-phenyl-pyrido[2,3-b]pyrazin-6-yl)-harnstoff (Bsp. 8)
Kohlensäure-4-[6-(3-ethyl-harnstoff)-pyrido[2,3-b]pyrazin-3-yl]-phenyl-methyl-ester (Bsp. 9)
Kohlensäure-4-[6-(3-ethyl-harnstoff)-pyrido[2,3-b]pyrazin-3-yl]-phenyl-(2-methoxyethyl)-ester (Bsp. 10)
Kohlensäure-4-[6-(3-ethyl-harnstoff)-pyrido[2,3-b]pyrazin-3-yl]-phenyl-phenyl-ester (Bsp. 11)
Diethyl-carbaminsäure-4-[6-(3-ethyl-harnstoff)-pyrido[2,3-b]pyrazin-3-yl]-phenylester (Bsp. 12)
(E)-3-Phenyl-acrylsäure-4-[6-(3-ethyl-harnstoff)-pyrido[2,3-b]pyrazin-3-yl]-phenylester (Bsp. 13)
Nonadecansäure 4-[6-(3-ethyl-harnstoff)-pyrido[2,3-b]pyrazin-3-yl]-phenyl-ester (Bsp. 14)
1-(3-Benzy)-pyrido[2,3-b]pyrazin-6-yl]-3-ethyl-harnstoff (Bsp. 15)

**7.** Pyrido[2,3-b]pyrazin-Derivate der allgemeinen Formel I nach einem der Ansprüche 1 bis 2, **dadurch gekennzeichnet, dass** der Alkylrest Methyl, Ethyl, n-Propyl, 2-Propyl, *n*-Butyl, *sec*.-Butyl, *tert*.-Butyl, *n*-Pentyl, *iso*-Pentyl, *neo*-Pentyl, *n*-Hexyl, 2-Hexyl, *n*-Octyl, Ethylenyl (Vinyl), Ethinyl, Propenyl ($-CH_2CH=CH_2$; $-CH=CH-CH_3$, $-C(=CH_2)-CH_3$), Propinyl ($-CH_2-C\equiv CH$, $-C\equiv C-CH_3$), Butenyl, Butinyl, Pentenyl, Pentinyl, Hexenyl, Hexinyl, Heptenyl, Heptinyl, Octenyl und Octinyl sein kann.

**8.** Pyrido[2,3-b]pyrazin-Derivate der allgemeinen Formel I nach einem der Ansprüche 1 bis 2, **dadurch gekennzeichnet, dass** der Heterocyclyl-Rest Tetrahydrofuryl, Pyrrolidinyl, Imidazolidinyl, Thiazolidinyl, Tetrahydropyranyl, Piperidinyl, Piperazinyl und Morpholinyl sein kann.

**9.** Pyrido[2,3-b]pyrazin-Derivate der allgemeinen Formel I nach einem der Ansprüche 1 bis 2, **dadurch gekennzeichnet, dass** der Heteroaryl-Rest Pyrrolyl, Furyl, Thienyl, Thiazolyl, Oxazolyl, Oxadiazolyl, Isoxazolyl, Pyrazolyl, Imidazolyl, Triazol, Tetrazol, Pyridinyl, Pyrimidinyl, Pyridazinyl, Pyrazinyl, Triazin, Phthalazinyl, Indolyl, Indazolyl, Indolizinyl, Chinolinyl, Isochinolinyl, Chinoxalinyl, Chinazolinyl, Pteridinyl, Carbazolyl, Phenazinyl, Phenoxazinyl, Phenothiazinyl, Acridinyl sein kann.

**10.** Arzneimittel, enthaltend mindestens eines der Pyrido[2,3-b]pyrazin-Derivate der gemäß einem der Ansprüche 1 bis 9.

**11.** Arzneimittel gemäß Anspruch 10, enthaltend das Pyrido[2,3-b]pyrazin-Derivat in Kombination mit mindestens einem weiteren pharmazeutischen Wirkstoff und/oder pharmazeutischen Trägerstoffen und/oder Verdünnungsmitteln beziehungsweise sonstigen Hilfsmitteln.

**12.** Verfahren zur Herstellung eines Arzneimittels nach einem der Ansprüche 10 bis 11, **dadurch gekennzeichnet, daß** ein oder mehrere Pyrido[2,3-b]pyrazin-Derivate der allgemeinen Formel I nach einem der Ansprüche 1 bis 9 mit gebräuchlichen pharmazeutischen Trägerstoffen und/oder Verdünnungsmitteln beziehungsweise sonstigen Hilfsstoffen zu pharmazeutischen Zubereitungen verarbeitet, beziehungsweise in eine therapeutisch anwendbare Form gebracht werden.

**13.** Verbindung gemäß einem der Ansprüche 1 bis 9 zur Verwendung als pharmazeutisches Mittel.

**14.** Verwendung einer Verbindung gemäß einem der Ansprüche 1 bis 9 zur Modulation von fehlgeleiteten zellulären Signaltransduktionsprozessen, insbesondere zur Beeinflussung der Funktion von aktiven und inaktiven Rezeptor-Tyrosin-kinasen, sowie cytoplasmatischen Tyrosin-, Serin/Threonin- und Lipidkinasen, wie c-Raf, B-Raf, Mek, MAPKs, PDGFRbeta, Flt-3, IGF1R, PI3K, PKB/Akt1, c-Kit, c-Abl, FGFR1 und KDR.

**15.** Verwendung einer Verbindung gemäß einem der Ansprüche 1 bis 9 zur Herstellung eines Arzneimittels zur Behandlung oder Prophylaxe von durch Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" vermittelten physiologischen und/oder pathophysiologischen Zuständen in Säugetieren.

**16.** Verwendung einer Verbindung gemäß einem der Ansprüche 1 bis 9, 15, wobei die Behandlung oder Prophylaxe durch Modulation des oder der Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" bewirkt wird.

**17.** Verwendung gemäß einem der Ansprüche 1 bis 9, 15, wobei die physiologischen und/oder pathophysiologischen Zustände durch Enzyme ausgewählt aus der Gruppe bestehend aus: "ATM, ATR, mTOR, DNA-PK, hSMG-1" vermittelt werden.

**18.** Verwendung gemäß einem der Ansprüche 1 bis 9, 15 bis 17, wobei die Behandlung oder Prophylaxe durch Modulation eines oder mehrerer Enzyme ausgewählt aus der Gruppe bestehend aus: "ATM, ATR, mTOR, DNA-PK, hSMG-1" bewirkt wird.

**19.** Verwendung gemäß einem der Ansprüche 1 bis 9, 15, 16, wobei die vermittelnden und/oder modulierten Signaltransduktionswege der ras-Raf-Mek-Erk-Signaltransduktionsweg und der PI3K-Akt-Signaltransduktionsweg sind.

**20.** Verwendung gemäß einem der Ansprüche 1 bis 9, 15, 16, wobei der vermittelnde und/oder modulierte Signaltransduktionsweg der ras-Raf-Mek-Erk-Signaltransduktionsweg ist.

**21.** Verwendung gemäß einem der Ansprüche 1 bis 9, 15, 16, wobei der vermittelnde und/oder modulierte Signaltransduktionsweg der PI3K-Akt-Signaltransduktionsweg ist.

**22.** Verwendung gemäß einem der Ansprüche 1 bis 9, 15, 16, wobei die vermittelnden und/oder modulierten Signaltransduktionswege der SAPK-Signaltransduktionsweg und der PI3K-Akt-Signaltransduktionsweg sind.

**23.** Verwendung gemäß einem der Ansprüche 1 bis 9, 15, 16, wobei der vermittelnde und/oder modulierte Signaltransduktionsweg der SAPK-Signaltransduktionsweg ist.

**24.** Verwendung gemäß einem der Ansprüche 1 bis 9, 15, 16, 19, 20, wobei die Modulation des ras-Raf-Mek-Erk-Signaltransduktionsweges bewirkt wird durch Modulation eines oder mehrerer Enzyme ausgewählt aus der Gruppe bestehend aus: "Tyrosinkinase, Serin/Threoninkinase, Rezeptor-Tyrosinkinase, cytoplasmatische Tyrosinkinase, cytoplasmatische Serin/Threoninkinase".

**25.** Verwendung gemäß Anspruch 24, wobei das Enzym ausgewählt ist aus der Gruppe bestehend aus: "Erk, Erk1, Erk2".

**26.** Verwendung gemäß einem der Ansprüche 1 bis 9, 15, 16, 19, 21, 22, wobei die Modulation des PI3K-Akt-Signaltransduktionsweges bewirkt wird durch Modulation eines oder mehrerer Enzyme ausgewählt aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

**27.** Verwendung gemäß einem der Ansprüche 1 bis 9, 15, 16, 22, 23, wobei die Modulation des SAPK-Signaltransduktionsweges bewirkt wird durch Modulation eines oder mehrerer Enzyme ausgewählt aus der Gruppe bestehend aus: "Tyrosinkinase, Serin/Threoninkinase, Rezeptor-Tyrosinkinase, cytoplasmatische Tyrosinkinase, cytoplasmatische Serin/Threoninkinase"

**28.** Verwendung gemäß Anspruch 27, wobei das Enzym ausgewählt ist aus der Gruppe bestehend aus: "Jnk, Jnk1, Jnk2, Jnk3, p38, p38alpha, p38beta, p38gamma, p38delta".

**29.** Verwendung gemäß einem der Ansprüche 1 bis 9, 14 bis 28, wobei zwei oder mehrere Enzyme moduliert werden.

**30.** Verwendung gemäß Anspruch 29, wobei mindestens ein Enzym ausgewählt ist aus der Gruppe bestehend aus: "Erk, Erk1, Erk2" und mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

**31.** Verwendung gemäß Anspruch 29, wobei mindestens ein Enzym ausgewählt ist aus der Gruppe bestehend aus: "Jnk, Jnk1, Jnk2, Jnk3, p38, p38alpha, p38beta, p38gamma, p38delta" und mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

**32.** Verwendung gemäß Anspruch 29, wobei mindestens ein Enzym ausgewählt ist aus der Gruppe bestehend aus: "Erk, Erk1, Erk2" und mindestens ein Enzym ausgewählt ist aus der Gruppe bestehend aus: "ATM, ATR, mTOR, DNA-PK, hSMG-1".

**33.** Verwendung gemäß Anspruch 29, wobei mindestens ein Enzym ausgewählt ist aus der Gruppe bestehend aus: "Jnk, Jnk1, Jnk2, Jnk3, p38, p38alpha, p38beta, p38gamma, p38delta" und mindestens ein Enzym ausgewählt ist aus der Gruppe bestehend aus: "ATM, ATR, mTOR, DNA-PK, hSMG-1".

**34.** Verwendung gemäß Anspruch 29, wobei mindestens ein Enzym ausgewählt ist aus der Gruppe bestehend aus:

"PI3K, PI3Kalpha, PI3Kbeta, PI3Kgamma, PI3Kdelta, PI3K-C2alpha, PI3K-C2beta, PI3K-Vps34p" und mindestens ein Enzym ausgewählt ist aus der Gruppe bestehend aus: "ATM, ATR, mTOR, DNA-PK, hSMG-1".

**35.** Verwendung gemäß einem der Ansprüche 1 bis 9, 14 bis 34, wobei die Modulation eine Inhibition ist.

**36.** Verwendung gemäß einem der Ansprüche 1 bis 9, 15 bis 35, wobei das Säugetier ausgewählt ist aus der Gruppe bestehend aus: "Mensch, Nutztier, Vieh, Haustier, Rind, Kuh, Schaf, Schwein, Ziege, Pferd, Pony, Esel, Maulesel, Maultier, Hase, Kaninchen, Katze, Hund, Meerschweinchen, Hamster, Ratte, Maus" und bevorzugt ein Mensch ist.

**37.** Verwendung gemäß einem der Ansprüche 1 bis 9, 15 bis 36, wobei die physiologischen und/oder pathophysiologischen Zustände ausgewählt sind aus der Gruppe bestehend aus: "maligne Tumore, benigne Tumore, entzündliche Erkrankungen, Entzündungen, Schmerzen, rheumatische Erkrankungen, arthritische Erkrankungen, HIV Infektionen, neurologische oder neurodegenerative Erkrankungen, Rheuma, Arthritis, AIDS, ARC (AIDS related complex), Kaposi-Sarkom, Tumore ausgehend vom Hirn und/oder Nervensystem und/oder Himhäuten, Demenz, Alzheimer, hyperproliferative Erkrankungen, Psoriasis, Endometriose, Narbenbildung, benigne Prostatahyperpläsie (BPH), Erkrankungen des Immunsystems, Autoimmunerkrankungen, Immundefizienzerkrankungen, Kolontumor, Magentumor, Darmtumor, Lungentumor, Pankreastumor, Ovarialtumor, Prostatatumor, Leukämie, Melanom, Lebertumor, Nierentumor, Kopftumor, Halstumor, Gliom, Brust-Tumor, Gebärmutterkrebs, Endometriumkrebs, Gebärmutterhalskrebs, Himtumor, Adenokanthom, Blasenkrebs, Magentumor, Kolorectalrumor, Speiseröhrenkrebs, gynokologischer Tumor, Ovartumor, Schilddrüsenkrebs, Lymphom, chronische Leukämie, akute Leukämie, Restenose, Diabetis, diabetische Nephropathie, fibrotische Erkrankungen, cystische Fibrose, maligne Nephrosklerose, thrombotische Microangiopathie Syndrom, Organtransplantat-Abstoßung, Glomerulpathien, Erkrankungen des Stoffwechsels, solide/feste Tumore, rheumatische Arthritis, diabetische Retinopathie, Asthma, Allergien, allergische Erkrankungen, chronische obstruktive pulmonare Erkrankungen, inflammatorische Bowel-Erkrankung, Fibrose, Atheriosklerose, Herzerkrankungen, cardiovaskuläre Erkrankungen, Erkrankungen des Herzmuskels, vaskuläre Erkrankungen, angiogenetische Erkrankungen, Nierenerkrankungen, Rhinitis, Grave Erkrankung, fokale Ischemie, Herzversagen, Ischemie, kardische Hypertrophie, Nierenversagen, kardische Myozyten Fehlfunktion, Bluthochdruck, Vasoconstriktion, Schlaganfall, anaphylaktischer Schock, Blutplättchen-Verklumpung, Skelettmuskelatrophie, Fettleibigkeit, Übergewicht, Glukose Homeostase, kongestive Herzinsuffizienz, Angina, Herzanfall, Herzinfarkt, Hyperglykämie, Hypoglykämie, Hypertension".

**38.** Verwendung gemäß einem der Ansprüche 1 bis 9, 10, 11, 13, 15 bis 37, wobei das Arzneimittel mindestens eine weitere pharmakologisch aktive Substanz umfasst.

**39.** Verwendung gemäß einem der Ansprüche 1 bis 9, 10, 11, 13, 15 bis 37, wobei das Arzneimittel vor und/oder während und/oder nach der Behandlung mit mindestens einer weiteren pharmakologisch aktiven Substanz verabreicht wird.

**40.** Verwendung gemäß einem der Ansprüche 1 bis 9, 10, 11, 13, 15 bis 37, wobei das Arzneimittel vor und/oder während und/oder nach der Behandlung mit Strahlentherapie und/oder Chirurgie verabreicht wird.

**41.** Verwendung gemäß einem der Ansprüche 38 bis 39, wobei die weitere pharmakologisch aktive Substanz ausgewählt ist aus der Gruppe bestehend aus: "DNA Topoisomerase I und/oder II Inhibitoren, DNA Interkalatoren, alkylierende Agentien, Microtubuli Destabilisatoren, Hormon- und/oder Wachstumsfaktor-Rezeptor-Agonisten und/oder -Antagonisten, Antikörper gegen Wachstumsfaktoren und deren Rezeptoren, Kinase Inhibitoren, Antimetabolite".

**42.** Verwendung gemäß einem der Ansprüche 38, 39, 41, wobei die weitere pharmakologisch aktive Substanz ausgewählt ist aus der Gruppe bestehend aus: "Asparaginase, Bleomycin, Carboplatin, Carmustin, Chlorambucil, Cisplatin, Colaspase, Cyclophosphamid, Cytarabin, Dacarbazin, Dactinomycin, Daunorubicin, Doxorubicin(Adriamycin), Epirubicin, Etoposide, 5-Fluorouracil, Hexamethylmelamin, Hydroxharnstoff, Ifosfamid, Irinotecan, Leucovorin, Lomustin, Mechlorethamin,6-Mercaptopurin, Mesna, Methotrexat, Mitomycin C, Mitoxantrone, Prednisolon, Prednison, Procarbazin, Raloxifen, Streptozocin, Tamoxifen, Thioguanin, Topotecan, Vinblastin, Vincristin, Vindesin, Aminoglutethimid, L-Asparaginase, Azathioprin, 5-Azacytidin cladribin, Busulfan, Diethylstilbestrol, 2', 2'-Difluorodeoxycytidin, Docetaxel, Erythrohydroxynonyladenin, Ethinylestradiol, 5-Fluorodeoxyuridin, 5-Fluorodeoxyuridin Monophosphat, Fludarabin Phosphate, Fluoxymesteron, Flutamid, Hydroxyprogesteron Caproat, Idarubicin, Interferon, Medroxyprogesteron Acetat, Megestrol Acetat, Melphalan, Mitotan, Paclitaxel, Oxaliplatin, Pentostatin, N-Phosphonoacetyl-L-aspartat (PALA), Plicamycin, Semustin, Teniposide, Testosteron Propionat, Thiotepa, Trimethylmelamin, Uridine, Vinorelbin, Epothilon, Gemcitabin, Taxotere, BCNU, CCNU, DTIC, 5-Fluorouarcil, Herceptin, Avastin, Erbitux, Sorafenib, Gleevec, Iressa, Tarceva, Rapamycin, Actinomycin D".

**43.** Pharmazeutische Zusammensetzung, die eine pharmakologisch aktive Menge mindestens einer Verbindung gemäß einem der Ansprüche 1 bis 9 umfasst.

**44.** Pharmazeutische Zusammensetzung gemäß Anspruch 43, wobei der Wirkstoff in einer Einheitsdosis von 0,001 mg bis 100 mg pro kg Körpergewicht eines Patienten vorliegt.

**45.** Pharmazeutische Zusammensetzung gemäß einem der Ansprüche 43 bis 44, wobei die Zusammensetzung weiterhin mindestens einen pharmazeutisch verträglichen Trägerstoff und/oder Hilfsstoff enthält.

**46.** Pharmazeutische Zusammensetzung gemäß einem der Ansprüche 43 bis 45, wobei die Zusammensetzung mindestens einen weiteren pharmakologisch aktiven Wirkstoff enthält.

**47.** Pharmazeutische Zusammensetzung gemäß Anspruch 46, wobei die weitere pharmakologisch aktive Substanz ausgewählt ist aus der Gruppe bestehend aus: "DNA Topoisomerase I und/oder II Inhibitoren, DNA Interkalatoren, alkylierende Agentien, Microtubuli Destabilisatoren, Hormon- und/oder Wachstumsfaktor-Rezeptor-Agonisten und/oder -Antagonisten, Antikörper gegen Wachstumsfaktoren und deren Rezeptoren, Kinase Inhibitoren, Antimetabolite".

**48.** Pharmazeutische Zusammensetzung gemäß einem der Ansprüche 46 bis 47, wobei der weitere pharmakologisch aktive Wirkstoff ausgewählt ist aus der Gruppe bestehend aus: "Asparaginase, Bleomycin, Carboplatin, Carmustin, Chlorambucil, Cisplatin, Colaspase, Cyclophosphamid, Cytarabin, Dacarbazin, Dactinomycin, Daunorubicin, Doxorubicin(Adriamycin), Epirubicin, Etoposide, 5-Fluorouracil, Hexamethylmelamin, Hydroxharnstoff, Ifosfamid, Irinotecan, Leucovorin, Lomustin, Mechlorethamin,6-Mercaptopurin, Mesna, Methotrexat, Mitomycin C, Mitoxantrone, Prednisolon, Prednison, Procarbazin, Raloxifen, Streptozocin, Tamoxifen, Thioguanin, Topotecan, Vinblastin, Vincristin, Vindesin, Aminoglutethimid, L-Asparaginase, Azathioprin, 5-Azacytidin cladribin, Busulfan, Diethylstilbestrol, 2', 2'-Difluorodeoxycytidin, Docetaxel, Erythrohydroxynonyladenin, Ethinylestradiol, 5-Fluorodeoxyuridin, 5- Fluorodeoxyuridin Monophosphat, Fludarabin Phosphate, Fluoxymesteron, Flutamid, Hydroxyprogesteron Caproat, Idarubicin, Interferon, Medroxyprogesteron Acetat, Megestrol Acetat, Melphalan, Mitotan, Paclitaxel, Oxaliplatin, Pentostatin, N-Phosphonoacetyl-L-aspartat (PALA), Plicamycin, Semustin, Teniposide, Testosteron Propionat, Thiotepa, Trimethylmelamin, Uridine, Vinorelbin, Epothilon, Gemcitabin, Taxotere, BCNU, CCNU, DTIC, 5-Fluorouarcil, Herceptin, Avastin, Erbitux, Sorafenib, Gleevec, Iressa, Tarceva, Rapamycin, Actinomycin D".

**49.** Kit umfassend eine pharmakologisch aktive Menge mindestens einer Verbindung gemäß einem der Ansprüche 1 bis 9 und eine pharmakologisch aktive Menge mindestens eines weiteren pharmakologisch aktiven Wirkstoffs gemäß einem der Ansprüche 46 bis 48.

Europäisches Patentamt

# EUROPÄISCHER TEILRECHERCHENBERICHT

der nach Regel 45 des Europäischen Patent-übereinkommens für das weitere Verfahren als europäischer Recherchenbericht gilt

Nummer der Anmeldung
EP 05 02 4693

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (IPC) |
|---|---|---|---|
| D,X | WO 2004/104002 A (ZENTARIS GMBH) 2. Dezember 2004 (2004-12-02) * Seite 34; Tabelle 1 * * Ansprüche 9-21 * ----- | 1-49 | INV. C07D471/04 A61K31/4985 A61P35/00 |
| D,X | WO 2004/104003 A (ZENTARIS GMBH) 2. Dezember 2004 (2004-12-02) * Seite 52; Tabelle 1 * * Ansprüche 6-14 * ----- | 1-49 | |
| D,X | WO 2005/007099 A (IMCLONE SYSTEMS INCORPORATED; KAWAKAMI, JOEL; DUNCTON, MATTHEW; SHERMA) 27. Januar 2005 (2005-01-27) * Seite 1 * * Ansprüche 1,6,17 * * Beispiel 23; Tabelle 1 * * Beispiel 21; Tabelle 2 * ----- | 1-49 | |
| | | | RECHERCHIERTE SACHGEBIETE (IPC) |
| | | | C07D A61K A61P |

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

2

| Recherchenort | Abschlußdatum der Recherche | Prüfer |
|---|---|---|
| Den Haag | 24. April 2006 | Seitner, I |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C09)

Europäisches
Patentamt

**UNVOLLSTÄNDIGE RECHERCHE
ERGÄNZUNGSBLATT C**

Nummer der Anmeldung
EP 05 02 4693

Obwohl der Anspruch 14 sich auf ein Verfahren zur Behandlung des menschlichen/tierischen Körpers beziehen (Artikel 52(4) EPÜ), wurde die Recherche durchgeführt und gründete sich auf die angeführten Wirkungen der Verbindung/Zusammensetzung.

-----

**ANHANG ZUM EUROPÄISCHEN RECHERCHENBERICHT ÜBER DIE EUROPÄISCHE PATENTANMELDUNG NR.** EP 05 02 4693

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-04-2006

| Im Recherchenbericht angeführtes Patentdokument | Datum der Veröffentlichung | Mitglied(er) der Patentfamilie | Datum der Veröffentlichung |
|---|---|---|---|
| WO 2004104002 A | 02-12-2004 | AU 2004240746 A1 | 02-12-2004 |
| | | CA 2524948 A1 | 02-12-2004 |
| | | DE 10323345 A1 | 16-12-2004 |
| | | EP 1628976 A1 | 01-03-2006 |
| WO 2004104003 A | 02-12-2004 | AU 2004240747 A1 | 02-12-2004 |
| | | CA 2524525 A1 | 02-12-2004 |
| | | EP 1636228 A1 | 22-03-2006 |
| WO 2005007099 A | 27-01-2005 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9932111 A
- WO 03068223 A
- WO 0206213 A
- WO 0035435 A
- WO 0037141 A
- WO 0181346 A
- WO 04017950 A
- WO 04104002 A
- WO 04104003 A
- WO 9917759 A
- WO 05007099 A, Kawakami
- WO 05056547 A, Bemis
- WO 04005472 A, White
- WO 03084473 A, Barnett
- WO 03086394 A, Bilodeau
- WO 03086403 A, Lindsley
- WO 03024448 A, Delorme
- WO 2004104002 A
- WO 2004104003 A
- GB 1184848 A, Degussa
- EP 735025 A, S. Seko


### In der Beschreibung aufgeführte Nicht-Patentliteratur


- **C. TEMPLE, JR.** *J. Med. Chem.,* 1990, 3044-3050
- *J. Org. Chem,* 1968, 2393-2397
- **C.TEMPLE, JR.** *J. Med. Chem,* 1968, 1216-1218
- **HOUBEN-WEYL.** Methoden der Organischen Chemie. vol. 4/1a, 343-350
- **HOUBEN-WEYL.** Methoden der Organischen Chemie. vol. E 7b, 579
- **D. CATARZI et al.** *J. Med. Chem,* 1996, 1330-1336
- **J. K. SEYDEL et al.** *J. Med. Chem,* 1994, 3016-3022
- **HOUBEN-WEYL.** Methods of Organic Chemistry. vol. E 9c, 231-235
- **HOUBEN-WEYL.** Science of Synthesis. vol. 16, 1269
- **C. L. LEESE ; H. N. RYDON.** *J. Chem. Soc.,* 1955, 303-309
- **T. S. OSDENE ; G. M. TIMMIS.** *J. Chem. Soc.,* 1955, 2033-2035
- **W. HE et al.** *Bioorg. Med. Chem. Lett.,* 2003, vol. 13, 3097-3100
- **M. S. A. EL-GABY et al.** *Indian J. Chem. Sect. B,* 2001, vol. 40, 195-200
- **M. R. MYERS et al.** *Bioorg. Med. Chem. Lett.,* 2003, vol. 13, 3091-3096
- **A. R. RENSLO et al.** *J. Amer. Chem. Soc.,* 1999, vol. 121, 7459-7460
- **C. O. OKAFOR et al.** *J. Heterocyclic Chem.,* 1983, vol. 20, 199-203
- **C. R. HOPKINS et al.** *Tet. Lett.,* 2004, vol. 45, 8631-8633
- **J. YIN et al.** *Org. Lett.,* 2002, vol. 4, 3481-3484
- **O. A. EL-SAYED et al.** *Arch. Pharm.,* 2002, vol. 335, 403-410
- **C. TEMPLE et al.** *J. Med. Chem,* 1992, vol. 35, 988-993
- **A. M. THOMPSON et al.** *J. Med. Chem.,* 2000, vol. 43, 4200-4211
- **N. A. DALES et al.** *Org. Lett.,* 2001, 2313-2316
- **G. DANNHARDT et al.** *Arch. Pharm.,* 2000, 267-274
- **G. S. POINDEXTER et al.** *Bioorg. Med. Chem.,* 2004, vol. 12, 507-521
- **J.-M. RECEVEUR et al.** *Bioorg. Med. Chem. Lett.,* 2004, vol. 14, 5075-5080
- **G. HEINISCH et al.** *Arch. Pharm.,* 1997, 207-210
- **K. MATSUNO et al.** *J. Med. Chem.,* 2002, vol. 45, 4513-4523
- **A. M. PAPINI et al.** *J. Med. Chem.,* 2004, vol. 47, 5224-5229
- **J. MINDL et al.** *Collect. Czech. Chem. Commun,* 1983, vol. 48, 900-905
- **S. SASAKI et al.** *J. Med. Chem.,* 2003, vol. 46, 113-124
- **B.-B. ZENG et al.** *Bioorg. Med. Chem. Lett.,* 2004, vol. 14, 5565-5568
- **Q. WANG et al.** *Synthetic Commun.,* 2004, vol. 34, 255-264
- **W. MEDERSKI et al.** *Bioorg. Med. Chem. Lett.,* 2003, vol. 13, 13715-3718
- **R. J. BROWN et al.** *Tetrahedron,* 2004, vol. 60, 4361-4375
- **L. MAO et al.** *Synthesis,* 2004, vol. 15, 2535-2539

- **M. DARABANTU et al.** *Tetrahedron,* 2005, vol. 61, 2897-2905
- **E. FORD et al.** *Tet. Lett.,* 2000, vol. 41, 3197-3198
- **T. SHIOTA et al.** *J. Org. Chem.,* 1999, vol. 64, 453-457
- **E. C. TAYLOR et al.** *Synthetic Commun.,* 1987, vol. 17, 1865-1868
- **G. A. MOLANDER et al.** *J. Org. Chem.,* 2002, vol. 67, 8424-8429
- **G. HUGHES et al.** *Org. & Biomolecular Chem.,* 2004, vol. 2, 3363-3367
- **R. P. TANGALLAPALLY et al.** *J. Med. Chem.,* 2004, vol. 47, 5276-5283
- **R. H. BRADBURRY et al.** *J. Med. Chem.,* 1997, vol. 40, 996-1004
- **X. HE et al.** *Bioorg. Med. Chem.,* 2004, vol. 12, 4003-4008
- **A. GOPALSAMY et al.** *Bioorg. Med. Chem. Lett.,* 2005, vol. 15, 1591-1594
- **J.-F. CHENG et al.** *Bioorg. Med. Chem. Lett.,* 2004, vol. 14, 2411-2416
- **E. R. PARMEE et al.** *Bioorg. Med. Chem. Lett.,* 2004, vol. 14, 43-46